# FASCICULE DE BREVET EUROPEEN

(11) **EP 2 331 546 B1**
(45) Date de publication et mention de la délivrance du brevet: **01.02.2012**
(21) Numéro de dépôt: 09737011.8
(22) Date de dépôt: 12.08.2009
(51) Int. Cl.: C07D 487/04, A61K 31/5025, A61P 25/00, A61P 35/00

(54) **DERIVES DE 2-ALKYL-6-CYCLOAMINO-3-(PYRIDIN-4-YL)IMIDAZO[1,2-IB]-PYRIDAZINE, LEUR PREPARATION ET LEUR APPLICATION EN THERAPEUTIQUE**
2-ALKYL-6-CYCLOAMINO-3-(PYRIDIN-4-YL)IMIDAZO [1,2-IB]-PYRIDAZINDERIVATE, DEREN HERSTELLUNG UND DEREN THERAPEUTISCHE ANWENDUNG
2-ALKYL-6-CYCLOAMINO-3-(PYRIDIN-4-YL)IMIDAZO[1,2-IB]-PYRIDAZINE DERIVATIVES, PREPARATION THEREOF, AND THERAPEUTIC APPLICATION THEREOF

(30) Priorité: 12.08.2008 FR 0804573; 12.08.2008 US 88126 P
(43) Date de publication de la demande: 15.06.2011
(73) Titulaire: SANOFI, 75013 Paris (FR)
(72) Inventeur: ALMARIO GARCIA, Antonio, F-75013 Paris (FR); BURNIER, Philippe, F-75013 Paris (FR); COTE-DES COMBES, Sylvain, F-75013 Paris (FR); GILBERT, Jean-François, F-75013 Paris (FR); PACAUD, Christophe, F-75013 Paris (FR); PUECH, Frédéric, F-75013 Paris (FR); CHIANG, Yulin, Bridgewater, NJ 08807 (US); DAVIS, Larry, Bridgewater, NJ 08807 (US); GAO, Zhongli, Bridgewater, NJ 08807 (US); ZHAO, Qiuxia, Bridgewater, NJ 08807 (US)
(74) Mandataire: Le Coupanec, Pascale A.M.P.
(86) Numéro de dépôt international: PCT/FR2009/001001
(87) Numéro de publication internationale: WO 2010/018327

(56) Documents cités:
- WO-A-2009/016286
- US-A1- 2007 093 490
- BEHREND L ET AL: "IC261, a specific inhibitor of the protein kinases casein kinase 1-delta and -epsilon, triggers the mitotic checkpoint and induces p53-dependent postmitotic effects" ONCOGENE, NATURE PUBLISHING GROUP, GB BASINGSTOKE, HANTS, vol. 19, 1 janvier 2000 (2000-01-01), pages 5303-5313, XP002975773 ISSN: 0950-9232

## Description

La présente invention se rapporte à des dérivés de 2-alkyl-6-cycloamino-3-(pyridin-4-yl)imidazo[1,2-b]pyridazine, à leur préparation et à leur application en thérapeutique, dans le traitement ou la prévention de maladies impliquant la caséine kinase 1 epsilon et/ou la caséine kinase 1 delta.

La présente invention a pour objet les composés répondant à la formule générale (I) 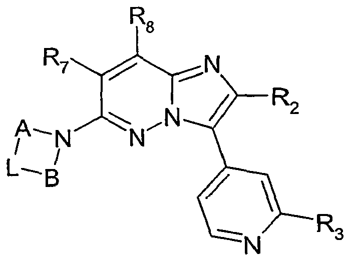 Dans laquelle
- R₂ représente un groupe C₁₋₆-alkyle, C₃₋₇-cycloalkyle, C₃₋₇-cycloalkyl-C₁₋₄-alkyle, C₁₋₄-alkyloxy-C₁₋₄-alkyle, C₃₋₇-cycloalkyloxy-C₁₋₄-alkyle, C₃₋₇-cycloalkyl-C₁₋₄-alkyloxy-C₁₋₄-alkyle, hydroxy-C₁₋₆-alkyle, C₁₋₄-fluoroalkyle ;
- R₃ représente un atome d'hydrogène ou un substituant choisi parmi les atomes d'halogène et les groupes C₁₋₃ alkyle, -NR₄R₅, hydroxyle ou C₁₋₄ alkyloxy ;
- A représente un groupe C₁₋₇-alkylène éventuellement substitué par un ou deux groupes Rₐ ;
- B représente un groupe C₁₋₇-alkylène éventuellement substitué par un groupe R_{b} ;
- L représente soit un atome d'azote éventuellement substitué par un groupe R_{c} ou R_{d}, soit un atome de carbone substitué par un groupe Rₑ₁ et un groupe R_{d} ou deux groupes Rₑ₂ ;
   les atomes de carbone de A et de B étant éventuellement substitués par un ou plusieurs groupes R_{f} identiques ou différents l'un de l'autre ;
   Rₐ, R_{b} et R_{c} sont définis tels que :
   deux groupes Rₐ peuvent former ensemble un groupe C₁₋₆-alkylène ;
   Rₐ et R_{b} peuvent former ensemble une liaison ou un groupe C₁₋₆-alkylène;
   Rₐ et R_{c} peuvent former ensemble une liaison ou un groupe C₁₋₆-alkylène
   R_{b} et R_{c} peuvent former ensemble une liaison ou un groupe C₁₋₆-alkylène ;
   R_{d} représente un groupe choisi parmi l'atome d'hydrogène et les groupes C₁₋₆-alkyle, C₃₋₇-cycloalkyle, C₃₋₇-cycloalkyl-C₁₋₆-alkyle, C₁₋₆-alkylthio-C-₁₋₆-alkyle, C₁₋₆-alkyloxy-C₁₋₆-alkyle, C₁₋₆-fluoroalkyle, hydroxy-C₁₋₆-alkyle ;
   Rₑ₁ représente un groupe -NR₄R₅ ou une monoamine cyclique comportant éventuellement un atome d'oxygène, la monoamine cyclique étant éventuellement substituée par un ou plusieurs substituants choisis parmi l'atome de fluor et les groupes C₁₋₆-alkyle, C₁₋₆-alkyloxy, hydroxyle ;
   Deux Rₑ₂ forment avec l'atome de carbone qui les porte une monoamine cyclique comportant éventuellement un atome d'oxygène, cette monoamine cyclique étant éventuellement substituée par un ou plusieurs groupes R_{f} identiques ou différents l'un de l'autre ;
   R_{f} représente un groupe C₁₋₆-alkyle, C₃₋₇-cycloalkyle, C₃₋₇-cycloalkyle-C₁₋₆-alkyle, C₁₋₆-alkyloxy-C₁₋₆-alkyle, C₃₋₇-cycloalkyloxy-C₁₋₄-alkyle, C₃₋₇-cycloalkyl-C₁₋₄-alkyloxy-C₁₋₄-alkyle, hydroxy-C₁₋₆-alkyle, C₁₋₆-fluoroalkyle;
   R₄ et R₅ représentent, indépendamment l'un de l'autre, un atome d'hydrogène ou un groupe C₁₋₄ alkyle, C₃₋₇-cycloalkyle, C₃₋₇-cycloalkyle-C₁₋₆-alkyle ;
- R₇ et R₈ représentent, indépendamment l'un de l'autre, un atome d'hydrogène ou un groupe un groupe C₁₋₆-alkyle.

Les composés de formule (I) peuvent comporter un ou plusieurs atomes de carbone asymétriques. Ils peuvent donc exister sous forme d'énantiomères ou de diastéréoisomères. Ces énantiomères, diastéréoisomères, ainsi que leurs mélanges, y compris les mélanges racémiques, font partie de l'invention.

Les composés de formule (I) peuvent exister à l'état de bases ou de sels d'addition à des acides. De tels sels d'addition font partie de l'invention. Ces sels sont avantageusement préparés avec des acides pharmaceutiquement acceptables, mais les sels d'autres acides utiles, par exemple, pour la purification ou l'isolement des composés de formule (I) font également partie de l'invention.

Les composés de formule (I) peuvent également exister sous forme d'hydrates ou de solvates, à savoir sous forme d'associations ou de combinaisons avec une ou plusieurs molécules d'eau ou avec un solvant. De tels hydrates et solvates font également partie de l'invention.

Dans le cadre de l'invention, on entend par :
- C_{t-z} où t et z peuvent prendre les valeurs de 1 à 7, une chaîne carbonée pouvant avoir de t à z atomes de carbone, par exemple 6₁₋₇ une chaîne carbonée qui peut avoir de 1 à 7 atomes de carbone ;
- alkyle, un groupe aliphatique saturé, linéaire ou ramifié; par exemple un groupe C₁₋₆-alkyle représente une chaîne carbonée de 1 à 6 atomes de carbone, linéaire ou ramifiée, par exemple un méthyle, éthyle, propyle, isopropyle, butyle, isobutyle, terbutyle, pentyle, hexyle ;
- alkylène, un groupe alkyle divalent saturé, linéaire ou ramifié, par exemple un groupe C₁₋₆-alkylène représente une chaîne carbonée divalente de 1 à 6 atomes de carbone, linéaire ou ramifiée, par exemple un méthylène, éthylène, 1-méthyléthylène, propylène ;
- cycloalkyle, un groupe alkyle cyclique, par exemple un groupe C₃₋₇-cycloalkyle représente un groupe carboné cyclique de 3 à 7 atomes de carbone, par exemple un cyclopropyle, cyclobutyle, cyclopentyle, cyclohexyle, cycloheptyle ;
- acyle, un groupe alkyle-C(O)- ;
- hydroxyle, un groupe -OH ;
- monoamine cyclique, une chaîne carbonée cyclique saturée comportant 1 atome d'azote ;
- hydroxyalkyle, un groupe alkyle dont un atome d'hydrogène a été substitué par un groupe hydroxyle ;
- alkyloxy, un groupe -O-alkyle ;
- alkylthio, un groupe -S-alkyle ;
- fluoroalkyle, un groupe alkyle dont un ou plusieurs atomes d'hydrogène ont été substitués par un atome de fluor ;
- fluoroalkyloxy, un groupe alkyloxy dont un ou plusieurs atomes d'hydrogène ont été substitués par un atome de fluor ;
- un atome d'halogène, un atome de fluor, de chlore, de brome ou d'iode ;
- aryle, un groupe aromatique mono- ou bicyclique comprenant entre 6 et 10 atomes de carbones. A titre d'exemple de groupe aryle, on peut citer les groupes phényle ou naphtyle.

A titre d'exemples non limitatifs d'amines ou diamines cycliques formées par N, A, L et B, on peut notamment citer :
- l'aziridine, l'azétidine, la pyrrolidine, la pipéridine, l'azépine, la morpholine, la thiomorpholine, l'homopipéridine, l'azabicyclo-heptane, l'azabicyclo-octane, l'azabicyclo-nonane, l'aza-oxo-bicyclo-heptane, l'aza-thia-bicyclo-heptane, l'aza-oxo-bicyclo-octane, l'aza-thia-bicyclo-octane ;
- la pipérazine, l'homopipérazine, la diaza-cyclo-octane, le diaza-cyclo-nonane, le diaza-cyclo-décane, le diaza-cyclo-undécane,
- l'hexahydro-pyrrolo-pyrazine, l'octahydro-pyrrolo-diazépine, l'hexahydro-pyrrolo-pyrrole, l'octahydro-pyrrolo-pyridine, le décahydro-naphthyridine,
- le diaza-bicyclo-heptane, le diaza-bicyclo-octane, le diaza-bicyclo-nonane,
- diaza-spiro-heptane, diaza-spiro-octane, le diaza-spiro-nonane, le diaza-spiro-décane, le diaza-spiro-undécane, le oxa-diaza-spiro-undécane.

Parmi les composés de formule générale (I) objets de l'invention, un premier groupe de composés est constitué par les composés pour lesquels R₂ représente un groupe C₁₋₄-alkyle, C₃₋₄-cycloalkyle-C₁₋₄-alkyle, C₁₋₄-alkyloxy-C₁₋₄-alkyle, C₁₋₄-fluoroalkyle ;
A, L, B, R₃, R₇ et R₈ étant tels que définis ci-dessus.

Parmi les composés de formule générale (I) objets de l'invention, un second groupe de composés est constitué par les composés pour lesquels R₂ représente un groupe méthyle, éthyle, isopropyle, isobutyle, cyclopropyle, cyclobutyle, cyclopropyl-méthyle, méthoxy-méthyle, trifluorométhyle ;
A, L, B, R₃, R₇ et R₈ étant tels que définis ci-dessus.

Parmi les composés de formule générale (I) objets de l'invention, un troisième groupe de composés est constitué par les composés pour lesquels R₃ représente atome d'hydrogène, de fluor ou de chlore ou un groupe méthyle, méthylamino, -NH₂, méthoxy ;
A, L, B, R₂, R₇ et R₈ étant tels que définis ci-dessus.

Parmi les composés de formule générale (I) objets de l'invention, un quatrième groupe de composés est constitué par les composés pour lesquels R₇ et R₈ représentent, indépendamment l'un de l'autre, un atome d'hydrogène ou un groupe méthyle ;
A, L, B, R₂ et R₃ étant tels que définis ci-dessus.

Parmi les composés de formule générale (I) objets de l'invention, un cinquième groupe de composés est constitué par les composés pour lesquels :
- A représente un groupe C₁₋₇-alkylène éventuellement substitué par un ou deux groupes Rₐ ;
- B représente un groupe C₁₋₇-alkylène éventuellement substitué par un groupe R_{b} ;
- L représente un atome d'azote éventuellement substitué par un groupe R_{c} ou R_{d},
   les atomes de carbone de A et de B étant éventuellement substitués par un ou plusieurs groupes R_{f} identiques ou différents l'un de l'autre ;
- deux groupes Rₐ peuvent former ensemble un groupe C₁₋₆-alkylène ;
- Rₐ et R_{b} peuvent former ensemble une liaison ou un groupe C₁₋₆-alkylène;
- Rₐ et R_{c} peuvent former ensemble une liaison ou un groupe C₁₋₆-alkylène ;
- R_{b} et Rₑ peuvent former ensemble une liaison ou un groupe C₁₋₆-alkylène ;
- R_{d} représente un groupe choisi parmi l'atome d'hydrogène et les groupes C₁₋₆-alkyle, C₃₋₇-cycloalkyle, C₃₋₇-cycloalkyl-C₁₋₆-alkyle, C₁₋₆-alkylthio-C-₁₋₆-alkyle, C₁₋₆-alkyloxy-C₁₋₆-alkyle, C₁₋₆-fluoroalkyle, hydroxy-C₁₋₆-alkyle ;
- R_{f} représente un groupe C₁₋₆-alkyle, C₃₋₇-cycloalkyle, C₁₋₆-alkyloxy-C₁₋₆-alkyle, C₃₋₇-cycloalkyloxy-C₁₋₄-alkyle, C₃₋₇-cycloalkyl-C₁₋₄-alkyloxy-C₁₋₄-alkyle, hydroxy-C₁₋₆-alkyle ;
- R₂, R₃, R₇ et R₈ étant tels que définis ci-dessus.

Parmi les composés de formule générale (I) objets de l'invention, un sixième groupe de composés est constitué par les composés pour lesquels :
L'amine cyclique formée par -N-A-L-B- représente un groupe pipérazinyle, hexahydropyrrolopyrazinyle, diazabicycloheptyle, diazabicyclononyle, hexahydropyrrolopyrrole, octahydropyrrolopyridine éventuellement substitué par un ou plusieurs groupes méthyle, éthyle, isopropyle, cyclobutyle, hydroxyméthyle ;

- R₂, R₃, R₇ et R₈ étant tels que définis ci-dessus.

Parmi les composés de formule générale (I) objets de l'invention, un septième groupe de composés est constitué par les composés pour lesquels :
L'amine cyclique formée par -N-A-L-B- représente un groupe pipérazin-1-yle, (*R*,*S*)-3-méthylpipérazin-1-yle, (*R*)-3-méthylpipérazin-1-yle, (*S*)-3-méthylpipérazin-1-yle, 4-méthylpipérazin-1-yle, 4-éthyl-pipérazin-1-yle, 4-(isopropyl)pipérazin-1-yle, 4-(cyclobutyl)pipérazin-1-yle, (*R*,*S*)-3-(hydroxyméthyl)-pipérazin-1-yle, 3,3-diméthylpipérazin-1-yle, *cis*-3,5-diméthylpipérazin-1-yle, (*S*)-hexahydropyrrolo[1,2-a]pyrazin-2-yle, (1*S*,4*S*)-2,5-diazabicyclo[2.2.1]hept-2-yle, (*R*,*S*)-2,5-diazabicyclo[2.2.1]hept-2-yle, (*R*,*S*)-1,4-diazabicyclonon-4-yle, (*R*,*S*)-hexahydropyrrolo[3,4-*b*]pyrrol-5(1*H*)-yle, hexahydropyrrolo[3,4-*c*]pyrrol-2(1*H*)-yle, 5-méthyl-hexahydropyrrolo[3,4-*c*]pyrrol-2(1*H*)-yle, 5-isopropyl-hexahydropyrrolo[3,4-*c*]pyrrol-2(1*H*)-yle, (*R*,*S*)-octahydropyrrolo[3,4-b]pyridin-6-yle;

- R₂, R₃, R₇ et R₈ étant tels que définis ci-dessus.

Parmi les composés de formule générale (I) objets de l'invention, un huitième groupe de composés est constitué par les composés pour lesquels :
- A représente un groupe C₁₋₇-alkylène éventuellement substitué par un ou deux groupes Rₐ ;
- B représente un groupe C₁₋₇-alkylène éventuellement substitué par un groupe R_{b};
- L représente un atome de carbone substitué par deux groupes Rₑ₂ ;
   les atomes de carbone de A et de B étant éventuellement substitués par un ou plusieurs groupes R_{f} identiques ou différents l'un de l'autre ;
- Deux Rₑ₂ forment avec l'atome de carbone qui les porte une monoamine cyclique comportant éventuellement un atome d'oxygène, cette monoamine cyclique étant éventuellement substituée par un ou plusieurs groupes R_{f} identiques ou différents l'un de l'autre ;
- R_{f} représente un groupe C₁₋₆-alkyle,
- R₂, R₃, R₇ et R₈ étant tels que définis ci-dessus.

Parmi les composés de formule générale (I) objets de l'invention, un neuvième groupe de composés est constitué par les composés pour lesquels :

L'amine cyclique formée par -N-A-L-B- représente un groupe diaza-spiro-nonyle, diaza-spiro-décyle, diaza-spiro-undécyle ou oxa-diaza-spiro-undécyle ;
- R₂, R₃, R₇ et R₈ étant tels que définis ci-dessus.

Parmi les composés de formule générale (I) objets de l'invention, un dixième groupe de composés est constitué par les composés pour lesquels :
L'amine cyclique formée par -N-A-L-B- représente une 2,7-diaza-spiro[3.5]non-7-yle, (*R*,*S*)-diaza-spiro[4.5]déc-2-yle, 2,9-diaza-spiro[5.5]undéc-9-yle ou 1-oxa-4,9-diaza-spiro-undéc-9-yle ;

- R₂, R₃, R₇ et R₈ étant tels que définis ci-dessus.

Parmi les composés de formule générale (I) objets de l'invention, un onzième groupe de composés est constitué par les composés pour lesquels :
- A représente un groupe C₁₋₇-alkylène;
- B représente un groupe C₁₋₇-alkylène ;
- L représente un atome de carbone substitué par un groupe Rₑ₁ et un groupe R_{d} ;
- R_{d} représente un atome d'hydrogène ;
- Rₑ₁ représente un groupe -NR₄R₅ où R₄ et R₅ représentent, indépendamment l'un de l'autre, un atome d'hydrogène ou un groupe C₁₋₄ alkyle, ou bien Rₑ₁ représente une monoamine cyclique comportant éventuellement un atome d'oxygène, la monoamine cyclique étant éventuellement substituée par un ou plusieurs substituants choisis parmi les groupes C₁₋₆-alkyle, hydroxyle ;
- R₂, R₃, R₇ et R₈ étant tels que définis ci-dessus.

Parmi les composés de formule générale (I) objets de l'invention, un douzième groupe de composés est constitué par les composés pour lesquels :
- A représente un groupe -C₂H₄- ou un groupe -CH₂- ;
- B représente un groupe -C₂H₄- ;
- L représente un atome de carbone substitué par un groupe Rₑ₁ et un groupe R_{d} ;
- R_{d} représente un atome d'hydrogène ;
- Rₑ₁ représente un groupement pyrrolidinyle ;
- R₂, R₃, R₇ et R₈ étant tels que définis ci-dessus.

Parmi les composés de formule générale (I) objets de l'invention, un treizième groupe de composés est constitué par les composés pour lesquels :
- l'amine cyclique formée par -N-A-L-B- représente une 4-(pyrrolidin-1-yl)-pipéridin-1-yle, (*R*,*S*)-[1,3']bipyrrolidinyl-1'-yle ;
- R₂, R₃, R₇ et R₈ étant tels que définis ci-dessus.

Parmi les composés de formule générale (I) objets de l'invention, un quatorzième groupe de composés est constitué par les composés pour lesquels :
- R₂ représente un groupe méthyle, éthyle, isopropyle, isobutyle, cyclopropyle, cyclobutyle, cyclopropyl-méthyle, méthoxy-méthyle, trifluorométhyle ;
- R₃ représente atome d'hydrogène, de fluor ou de chlore ou un groupe méthyle, méthylamino, -NH₂, méthoxy ;
- l'amine cyclique formée par -N-A-L-B- représente un groupe pipérazin-1-yle, (*R*,*S*)-3-méthylpipérazin-1-yle, (*R*)-3-méthylpipérazin-1-yle, (*S*)-3-méthylpipérazin-1-yle, 4-méthylpipérazin-1-yle, 4-éthyl-pipérazin-1-yle, 4-(isopropyl)pipérazin-1-yle, 4-(cyclobutyl)pipérazin-1-yle, (*R*,*S*)-3-(hydroxyméthyl)-pipérazin-1-yle, 3,3-diméthylpipérazin-1-yle, *cis*-3,5-diméthylpipérazin-1-yle, (*S*)-hexahydropyrrolo[1,2-a]pyrazin-2-ylè, (1*S*,4*S*)-2,5-diazabicyclo[2.2.1]hept-2-yle, (*R*,*S*)-2,5-diazabicyclo[2.2.1]hept-2-yle, (*R*,*S*)-1,4-diazabicyclonon-4-yle, (*R*,*S*)-hexahydropyrrolo[3,4-*b*]pyrrol-5(1*H*)-yle, hexahydropyrrolo[3,4-*c*]pyrrol-2(1*H*)-yle, 5-méthyl-hexahydropyrrolo[3,4-*c*]pyrrol-2(1*H*)-yle, 5-isopropyl-hexahydropyrrolo[3,4-*c*]pyrrol-2(1*H*)-yle, (*R*,*S*)-octahydropyrrolo[3,4-*b*]pyridin-6-yle ;
- R₇ et R₈ représentent, indépendamment l'un de l'autre, un atome d'hydrogène ou un groupe méthyle.

Parmi les composés de formule générale (I) objets de l'invention, un quinzième groupe de composés est constitué par les composés pour lesquels :
- R₂ représente un groupe méthyle ;
- R₃ représente atome d'hydrogène ;
- l'amine cyclique formée par -N-A-L-B- représente une 2,7-diaza-spiro[3.5]non-7-yle, (*R*,*S*)-diaza-spiro[4.5]déc-2-yle, 2,9-diaza-spiro[5.5]undéc-9-yle ou 1-oxa-4,9-diaza-spiro-undéc-9-yle ;
- R₇ et R₈ représentent, indépendamment l'un de l'autre, un atome d'hydrogène.

Parmi les composés de formule générale (I) objets de l'invention, un seizième groupe de composés est constitué par les composés pour lesquels :
- R₂ représente un groupe méthyle ;
- R₃ représente atome d'hydrogène ou un groupe méthyle ;
- l'amine cyclique formée par -N-A-L-B- représente une 4-(pyrrolidin-1-yl)-pipéridin-1-yle ou une (*R*,*S*)-[1,3']bipyrrolidinyl-1'-yle ;
- R₇ et R₈ représentent, indépendamment l'un de l'autre, un atome d'hydrogène.

Parmi les composés de formule générale (I) objets de l'invention, on peut notamment citer les composés suivants :
- 2-Méthyl-6-pipérazin-1-yl-3-pyridin-4-yl-imidazo[1,2- *b*]pyridazine et son chlorhydrate (3:1) ;
- 3-(2-Fluoro-pyridin-4-yl)-2-méthyl-6-pipérazin-1-yl-imidazo[1,2- *b*]pyridazine ;
- 2,7,8-Triméthyl-6-pipérazin-1-yl-3-pyridin-4-yl-imidazo[1,2- *b*]pyridazine et son chlorhydrate (3:1);
- 2-Méthoxyméthyl-6-pipérazin-1-yl-3-pyridin-4-yl-imidazo[1,2-*b*]pyridazine ;
- 2-Ethyl-6-pipérazin-1-yl-3-pyridin-4-yl-imidazo[1,2-*b*]pyridazine et son chlorhydrate (3:1) ;
- 2-Ethyl-6-pipérazin-1-yl-3-(2-méthyl-pyridin-4-yl)-imidazo[1,2-*b*]pyridazine ;
- 2-Isopropyl-6-pipérazin-1-yl-3-pyridin-4-yl-imidazo[1,2-*b*]pyridazine ;
- 2-Isopropyl-3-(2-méthyl-pyridin-4-yl)-6-pipérazin-1-yl-imidazo[1,2-*b*]pyridazine ;
- 2-Cyclopropyl-6-pipérazin-1-yl-3-pyridin-4-yl-imidazo[1,2-*b*]pyridazine ;
- 2-Cyclopropyl-3-(2-méthyl-pyridin-4-yl)-6-pipérazin-1-yl-imidazo[1,2-*b*]pyridazine ;
- 4-(2-Cyclopropyl-6-pipérazin-1-yl-imidazo[1,2-*b*]pyridazin-3-yl)-pyridin-2-ylamine ;
- 2-Cyclopropyl-3-(2-méthoxy-pyridin-4-yl)-6-pipérazin-1-yl-imidazo[1,2-*b*]pyridazine ;
- 3-(2-Chloro-pyridin-4-yl)-2-cyclopropyl-6-pipérazin-1-yl-imidazo[1,2-*b*]pyridazine ;
- 2-Isobutyl-6-pipérazin-1-yl-3-pyridin-4-yl-imidazo[1,2-*b*]pyridazine et son chlorhydrate (3:1) ;
- 2-Cyclopropylméthyl-6-pipérazin-1-yl-3-pyridin-4-yl-imidazo[1,2-*b*]pyridazine ;
- 2-Cyclobutyl-6-pipérazin-1-yl-3-pyridin-4-yl-imidazo[1,2-*b*]pyridazine ;
- (*R*,*S*)-2-Méthyl-6-(3-méthyl-pipérazin-1-yl)-3-pyridin-4-yl-imidazo[1,2-*b*]pyridazine ;
- (*R*,*S*)-2-Méthyl-6-(3-méthyl-pipérazin-1-yl)-3-(2-méthyl-pyridin-4-yl)-imidazo[1,2-*b*]pyridazine ;
- (*R*,*S*)-2-Cyclopropyl-6-(3-méthyl-pipérazin-1-yl)-3-(2-méthyl-pyridin-4-yl)-imidazo[1,2-*b*]pyridazine ;
- (*R*,*S*)-4-[2-Cyclopropyl-6-(3-méthyl-pipérazin-1-yl)-imidazo[1,2-*b*]pyridazin-3-yl]-pyridin-2-ylamine ;
- (*R*,*S*)-2-Cyclopropyl-3-(2-méthoxy-pyridin-4-yl)-6-(3-méthyl-pipérazin-1-yl)-imidazo[1,2-*b*]pyridazine ;
- (*R*,*S*)-3-(2-Chloro-pyridin-4-yl)-2-cyclopropyl-6-(3-méthyl-pipérazin-1-yl)-imidazo[1,2-*b*]pyridazine ;
- 2-Méthyl-6-((*R*)-3-méthyl-pipérazin-1-yl)-3-pyridin-4-yl-imidazo[1,2-*b*]pyridazine ;
- 2-Méthyl-6-((*R*)-3-méthyl-pipérazin-1-yl)-3-(2-méthyl-pyridin-4-yl)-imidazo[1,2-*b*]pyridazine ;
- 4-[2-Méthyl-6-((*R*)3-méthyl-pipérazin-1-yl)-imidazo[1,2-*b*]pyridazin-3-yl]-pyridin-2-ylamine ;
- Méthyl-{4-[2-méthyl-6-((*R*)-3-méthyl-pipérazin-1-yl)-imidazo[1,2-*b*]pyridazin-3-yl]-pyridin-2-yl}-amine ;
- 3-(2-Méthoxy-pyridin-4-yl)-2-méthyl-6-((*R*)-3-méthyl-pipérazin-1-yl)-imidazo[1,2-*b*]pyridazine ;
- 2-Ethyl-6-((*R*)-3-méthyl-pipérazin-1-yl)-3-pyridin-4-yl-imidazo[1,2-*b*]pyridazine ;
- 2-Ethyl-3-(2-fluoro-pyridin-4-yl)-6-((*R*)-3-méthyl-pipérazin-1-yl)-imidazo[1,2-*b*]pyridazine ;
- 2-Cyclopropylméthyl-6-((*R*)-3-méthyl-pipérazin-1-yl)-3-pyridin-4-yl-imidazo[1,2-*b*]pyridazine ;
- 2-Cyclopropylméthyl-6-((*R*)-3-méthyl-pipérazin-1-yl)-3-(2-méthyl-pyridin-4-yl)-imidazo[1,2-*b*]pyridazine ;
- 2-Méthyl-6-((*S*)-3-méthyl-pipérazin-1-yl)-3-pyridin-4-yl-imidazo[1,2-*b*]pyridazine ;
- 3-(2-Methoxy-pyridin-4-yl)-2-méthyl-6-((*S*)-3-méthyl-pipérazin-1-yl)-imidazo[1,2-*b*]pyridazine ;
- 2-Cyclopropyl-6-((*S*)-3-méthyl-pipérazin-1-yl)-3-pyridin-4-yl-imidazo[1,2-*b*]pyridazine ;
- 4-[2-Méthyl-6-((*S*)-3-méthyl-pipérazin-1-yl)-imidazo[1,2-*b*]pyridazin-3-yl]-pyridin-2-ylamine ;
- 2-Méthyl-6-(4-méthyl-pipérazin-1-yl)-3-pyridin-4-yl-imidazo[1,2-*b*]pyridazine ;
- 6-(4-Méthyl-pipérazin-1-yl)-3-pyridin-4-yl-2-trifluorométhyl-imidazo[1,2-*b*]pyridazine ;
- [4-(2-Méthyl-3-pyridin-4-yl-imidazo[1,2-*b*]pyridazin-6-yl)-pipérazin-2-yl]-méthanol ;
- 6-(4-Ethyl-pipérazin-1-yl)-2-méthyl-3-pyridin-4-yl-imidazo[1,2-*b*]pyridazine et son chlorhydrate (3:1) ;
- 6-(4-Ethyl-pipérazin-1-yl)-2-méthyl-3-(2-méthyl-pyridin-4-yl)-imidazo[1,2-*b*]pyridazine ;
- 6-(4-Isopropyl-pipérazin-1-yl)-2-méthyl-3-pyridin-4-yl-imidazo[1,2-*b*]pyridazine et son chlorhydrate (3:1) ;
- 6-(4-Isopropyl-pipérazin-1-yl)-2-méthyl-3-(2-méthyl-pyridin-4-yl)-imidazo[1,2-*b*]pyridazine ;
- 4-[6-(4-Isopropyl-pipérazin-1-yl)-2-méthyl-imidazo[1,2-*b*]pyridazin-3-yl]-pyridin-2-yl}-amine et son chlorhydrate (3:1) ;
- 6-(4-Cyclobutyl-pipérazin-1-yl)-2-méthyl-3-pyridin-4-yl-imidazo[1,2-*b*]pyridazine ;
- 4-[6-(4-Cyclobutyl-pipérazin-1-yl)-2-méthyl-imidazo[1,2-*b*]pyridazin-3-yl]-pyridin-2-ylamine ;
- 6-(3,3-Diméthyl-pipérazin-1-yl)-2-méthyl-3-(2-méthyl-pyridin-4-yl)-imidazo[1,2-b]pyridazine ;
- {4-[6-(3,3-Diméthyl-pipérazin-1-yl)-2-méthyl-imidazo[1,2-b]pyridazin-3-yl]-pyridin-2-yl}-méthyl-amine ;
- 2-Cyclopropyl-6-(3,3-diméthyl-pipérazin-1-yl)-3-(2-méthyl-pyridin-4-yl)-imidazo[1,2-b]pyridazine ;
- 4-[2-Cyclopropyl-6-(3,3-diméthyl-pipérazin-1-yl)-imidazo[1,2-b]pyridazin-3-yl]-pyridin-2-ylamine ;
- 2-Cyclopropyl-6-(3,3-diméthyl-pipérazin-1-yl)-3-(2-fluoro-pyridin-4-yl)-imidazo[1,2-b]pyridazine ;
- 6-(*cis*-3,5-Diméthyl-pipérazin-1-yl)-2-méthyl-3-pyridin-4-yl-imidazo[1,2-*b*]pyridazine et son chlorhydrate (3:1) ;
- 6-(*cis*-3,5-Diméthyl-pipérazin-1-yl)-2-méthyl-3-(2-méthyl-pyridin-4-yl)-imidazo[1,2-*b*]pyridazine ;
- 4-[6-(cis-3,5-Diméthyl-pipérazin-1-yl)-2-méthyl-imidazo[1,2-*b*]pyridazin-3-yl]-pyridin-2-ylamine ;
- {4-[6-(*cis*-3,5-Diméthyl-pipérazin-1-yl)-2-méthyl-imidazo[1,2-*b*]pyridazin-3-yl]-pyridin-2-yl}-méthyl-amine ;
- 2-Cyclopropyl-6-(*cis*-3,5-diméthyl-pipérazin-1-yl)-3-pyridin-4-yl-imidazo[1,2-*b*]pyridazine ;
- 4-[2-Cyclopropyl-6-(*cis*-3,5-diméthyl-pipérazin-1-yl)-imidazo[1,2-*b*]pyridazin-3-yl]-pyridin-2-ylamine ;
- 6-(*S*)-Hexahydro-pyrrolo[1,2-a]pyrazin-2-yl-2-méthyl-3-pyridin-4-yl-imidazo[1,2-*b*]pyridazine ;
- 6-(*S*)-Hexahydro-pyrrolo[1,2-a]pyrazin-2-yl-2-méthyl-3-(2-méthyl-pyridin-4-yl)-imidazo[1,2-*b*]pyridazine ;
- 6-(1*S*,4*S*)-2,5-Diaza-bicyclo[2.2.1]hept-2-yl-2-méthyl-3-pyridin-4-yl-imidazo[1,2-*b*]pyridazine ;
- (*R*,*S*)-6-(2,5-Diaza-bicyclo[2.2.1]hept-2-yl)-2-méthyl-3-(2-méthyl-pyridin-4-yl)-imidazo[1,2-*b*]pyridazine et son bromhydrate (1:1) ;
- 4-[2-Cyclopropyl-6-(1,4-diaza-bicyclo[3.2.2]non-4-yl)-3-(2-méthyl-pyridin-4-yl)-imidazo[1,2-b]pyridazine ;
- (*R*,*S*)-6-(Hexahydro-pyrrolo[3,4-*b*]pyrrol-5(1*H*)-yl)-2-méthyl-3-(2-méthyl-pyridin-4-yl)-imidazo[1,2-b]pyridazine ;
- 6-Hexahydro-pyrrolo[3,4-*c*]pyrrol-2(1*H*)-yl-2-méthyl-3-pyridin-4-yl-imidazo[1,2-*b*]pyridazine ;
- 6-Hexahydro-pyrrolo[3,4-*c*]pyrrol-2(1*H*)-yl-2-méthyl-3-(2-méthyl-pyridin-4-yl)-imidazo[1,2-*b*]pyridazine et son chlorhydrate (3:1) ;
- 4-(6-Hexahydro-pyrrolo[3,4-*c*]pyrrol-2(1*H*)-yl-2-méthyl-imidazo[1,2-*b*]pyridazin-3-yl)-pyridin-2-ylamine ;
- [4-(6-Hexahydro-pyrrolo[3,4-*c*]pyrrol-2(1*H*)-yl-2-méthyl-imidazo[1,2-*b*]pyridazin-3-yl)-pyridin-2-yl]-méthyl-amine ;
- 2-Méthyl-6-(5-méthyl-hexahydro-pyrrolo[3,4-*c*]pyrrol-2(1*H*)-yl)-3-pyridin-4-yl-imidazo[1,2-*b*]pyridazine ;
- 2,7-Diméthyl-6-(5-méthyl-hexahydro-pyrrolo[3,4-*c*]pyrrol-2(1*H*)-yl)-3-pyridin-4-yl-imidazo[1,2-*b*]pyridazine ;
- 6-(-5-Isopropyl-hexahydro-pyrrolo[3,4-*c*]pyrrol-2(1*H*)-yl)-2-méthyl-3-pyridin-4-yl-imidazo[1,2-*b*]pyridazine ;
- 4-[6-(-5-Isopropyl-hexahydro-pyrrolo[3,4-*c*]pyrrol-2(1*H*)-yl)-2-méthyl-imidazo[1,2-*b*]pyridazin-3-yl]-pyridin-2-ylamine ;
- 6-Hexahydro-pyrrolo[3,4-*c*]pyrrol-2(1*H*)-yl-3-(2-méthoxy-pyridin-4-yl)-2-méthyl-imidazo[1,2-*b*]pyridazine ;
- (*R*,*S*)-2-Méthyl-6-(octahydro-pyrrolo[3,4-*b*]pyridin-6-yl)-3-pyridin-4-yl-imidazo[1,2-*b*]pyridazine ;
- 6-(2,7-Diaza-spiro[3.5]non-7-yl)-2-méthyl-3-(2-méthyl-pyridin-4-yl)-imidazo[1,2-*b*]pyridazine ;
- (*R*,*S*)-6-(2,7-Diaza-spiro[4.5]déc-2-yl)-2-méthyl-3-pyridin-4-yl-imidazo[1,2-*b*]pyridazine ;
- 9-(2-Méthyl-3-pyridin-4-yl-imidazo[1,2-*b*]pyridazin-6-yl)-2,9-diaza-spiro[5.5]undécane et son chlorhydrate (3:1) ;
- 9-[2-Méthyl-3-(2-méthyl-pyridin-4-yl)-imidazo[1,2-*b*]pyridazin-6-yl]-2,9-diaza-spiro[5.5]undécane et son chlorhydrate (3:1) ;
- 9-(2-Méthyl-3-pyridin-4-yl-imidazo[1,2-*b*]pyridazin-6-yl)-1-oxa-4,9-diaza-spiro[5.5]undécane et son chlorhydrate (3:1) ;
- 4-[2-Méthyl-6-(1-oxa-4,9-diaza-spiro[5.5]undéc-9-yl)-imidazo[1,2-*b*]pyridazin-3-yl]-pyridin-2-ylamine et son chlorhydrate (3:1) ;
- 2-Méthyl-3-pyridin-4-yl-6-(4-pyrrolidin-1-yl-pipéridin-1-yl)-imidazo[1,2-*b*]pyridazine ;
- 2-Méthyl-3-(2-méthyl-pyridin-4-yl)-6-(4-pyrrolidin-1-yl-pipéridin-1-yl)-imidazo[1,2-*b*]pyridazine ;
- 4-[2-Méthyl-6-(4-pyrrolidin-1-yl-pipéridin-1-yl)-imidazo[1,2-b]pyridazin-3-yl]-pyridin-2-ylamine ;
- (*R*,*S*)-6-[1,3']Bipyrrolidinyl-1'-yl-2-méthyl-3-pyridin-4-yl-imidazo[1,2-b]pyridazine.

L'invention a également pour objet un procédé de préparation des composés de l'invention de formule (I).

Conformément à l'invention, on peut préparer les composés de formule générale (I) selon le procédé général décrit dans le schéma 1 ci-après.

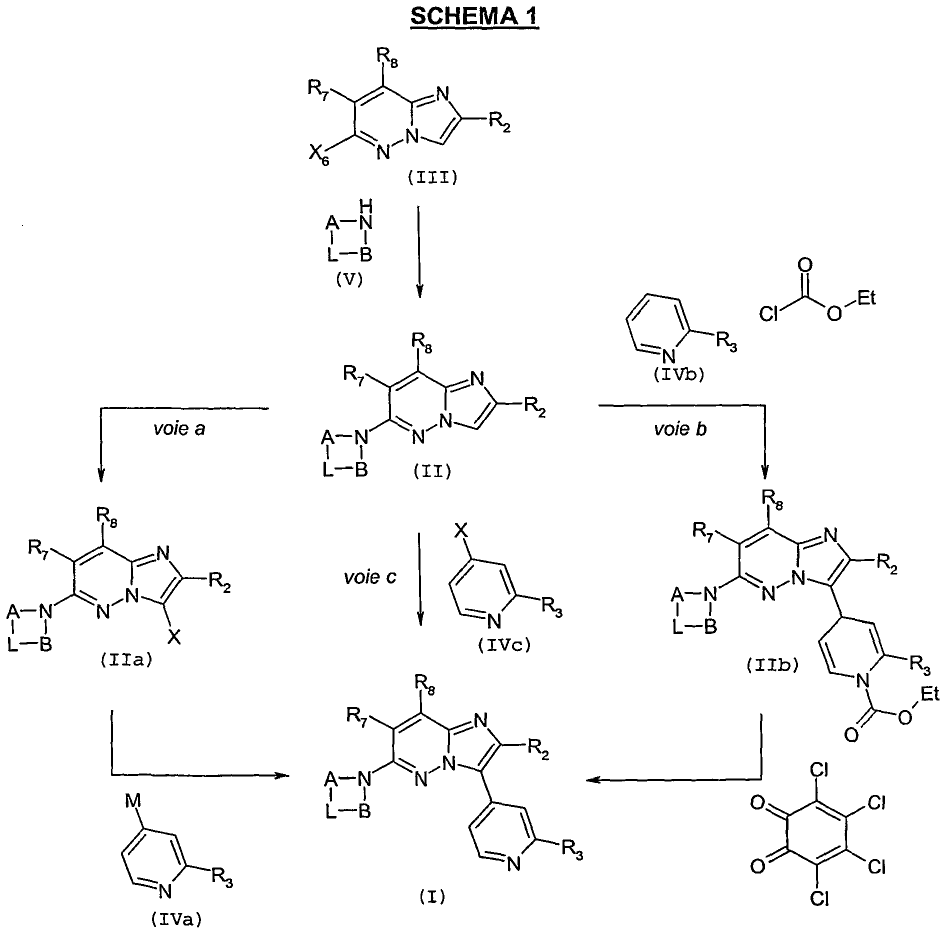

Dans ce qui suit, on entend par groupe partant un groupe pouvant être facilement clivé d'une molécule par rupture d'une liaison hétérolytique, avec départ d'une paire électronique. Ce groupe peut ainsi être remplacé facilement par un autre groupe lors d'une réaction de substitution, par exemple. De tels groupes partants sont, par exemple, les halogènes ou un groupe hydroxy activé tel qu'un mésyle, tosyle, triflate, acétyle, etc. Des exemples de groupes partants ainsi que des références pour leurs préparations sont données dans « Advances in Organic Chemistry », J. March, 3rd Edition, Wiley Interscience, p. 310-316.

Dans ce qui suit, on entend par groupe protecteur un groupe qui permet, de masquer une fonction réactive telle qu'un hydroxy ou une amine pendant une synthèse et de restaurer la fonction réactive intacte en fin de synthèse après une étape dite de déprotection. Des exemples de groupes protecteurs ainsi que des méthodes de protection et de déprotection sont données dans « Protective Groups in Organic Synthesis », Green et al., 2nd Edition (John Wiley & Sons, Inc., New York), 1991.

### Schéma 1 : introduction préliminaire de l'amine

### Voie a : Introduction de la pyridine - couplage de type « Stille » ou « Suzuki »

De manière générale et comme illustré dans le schéma 1, les dérivés de 2-alkyl-6-cycloamino-3-pyridin-4-ylimidazo[1,2-*b*]pyridazine de formule générale (I) dans laquelle R₂, R₃, A, L, B, R₇ et R₈ sont tels que définis ci-dessus peuvent être préparés en deux étapes à partir d'un dérivé de 2-alkyl-6-cycloamino-imidazo[1,2-*b*]pyridazine de formule générale (II) dans laquelle R₂, A, L, B, R₇ et R₈ sont tels que définis ci-dessus.

Le dérivé (II) est bromé ou iodé sélectivement en position 3 par traitement au moyen de *N-*bromo- ou iodosuccinimide ou de monochlorure d'iode dans un solvant polaire tel que l'acétonitrile, le tétrahydrofurane, le méthanol ou le chloroforme pour fournir le dérivé 6-amino-3-iodo- ou 3-bromo-imidazo[1,2-b]pyridazine de formule générale (IIa), dans laquelle R₂, A, L, B, R₇ et R₈ sont tels que définis ci-dessus et X représente un atome de brome ou d'iode. Celui-ci est transformé en composé de l'invention de formule générale (I) par couplage selon les conditions de Stille ou de Suzuki avec un stannane ou un boronate de pyridine de formule générale (IVa) dans laquelle R₃ est tel que défini ci-dessus et M représente un groupe trialkylstannyle, le plus fréquemment un groupement tributylstannyle ou un groupe dihydroxyboryle ou dialkyloxyboryle, le plus fréquemment un groupe 4,4,5,5-tétraméthyl-1,3,3,2-dioxaborolan-2-yle.

Les couplages selon la méthode de Stille sont par exemple réalisés par chauffage en présence d'un catalyseur tel que le tétrakis(triphénylphosphine)palladium, d'iodure de cuivre dans un solvant tel que le N,N-diméthylacétamide.

Les couplages selon la méthode de Suzuki sont par exemple réalisés par chauffage en présence d'un catalyseur tel que le 1,1'-bis (diphénylphosphino)ferrocènedichloropalladium, d'une base minérale telle que le carbonate de césium dans un mélange de solvant tels que le tétrahydrofurane et l'eau.

### Voie b : Introduction de la pyridine - addition nucléophile sur pyridinium

Alternativement, les dérivés de 2-alkyl-6-cycloamino-3-pyridin-4-ylimidazo[1,2-*b*]pyridazine de formule générale (I) dans laquelle R₂, R₃, A, L, B, R₇ et R₈ sont tels que définis ci-dessus peuvent encore être préparés en deux étapes à partir d'un dérivé de 2-alkyl-6-cycloamino-imidazo[1,2-*b*]pyridazine de formule générale (II) dans laquelle R₂, A, L, B, R₇ et R₈ sont tels que définis ci-dessus.

La réaction d'un dérivé de 2-alkyl-6-cycloamino-imidazo[1,2-*b*]pyridazine de formule générale (II) sur un mélange d'un dérivé de pyridine de formule générale (IVb) dans laquelle R₃ représente un atome d'hydrogène ou un groupe C₁₋₃-alkyle, et de chloroformiate d'alkyle, par exemple le chloroformiate d'éthyle, conduit au dérivé de formule générale (IIb) dans laquelle R₂, A, L, B, R₇ et R₈ sont tels que définis ci-dessus et dans laquelle R₃ représente un atome d'hydrogène ou un groupe C₁₋₃-alkyle. Le dérivé de formule générale (IIb) est ensuite oxydé au moyen d'ortho-chloranile dans un solvant tel que le toluène pour conduire aux dérivés de l'invention de formule générale (I) dans laquelle R₂, A, L, B, R₇ et R₈ sont tels que définis ci-dessus et dans laquelle R₃ représente un atome d'hydrogène ou un groupe C₁₋₃-alkyle.

### Voie c : Introduction de la pyridine - C-H arylation métallocatalysée

Enfin, les dérivés de 2-alkyl-6-cycloamino-3-pyridin-4-ylimidazo[1,2-b]pyridazine de formule générale (I) dans laquelle R₂, R₃, R₇, R₈, A, L, et B sont tels que définis ci-dessus peuvent être directement préparés à partir d'un dérivé de 2-alkyl-6-cycloamino-imidazo[1,2-b]pyridazine de formule générale (II), dans laquelle R₂, R₇, R₈, A, L, et B sont tels que définis ci-dessus par couplage métallo-catalysé avec un dérivé de pyridine de formule générale (IVc) dans laquelle R₃ est tels que défini précédemment et X représente un atome d'halogène, plus particulière l'iode. Ce couplage peut être réalisé en présence d'un catalyseur tel que l'acétate de Palladium, d'une base minérale telle que le carbonate de potassium et dans un solvant polaire aprotique tel que le diméthylformamide.

Les dérivés de 2-alkyl-6-cycloamino-imidazo[1,2-*b*]pyridazine de formule générale (II) dans laquelle R₂, A, L, B, R₇ et R₈ sont tels que définis ci-dessus peuvent être préparés à partir d'un dérivé de 2-alkyl-6-cycloamino-imidazo[1,2-b]pyridazine de formule générale (III), dans laquelle R₂, R₇ et R₈ sont tels que définis ci-dessus et X₆ représente un groupe partant tel qu'un halogène par traitement au moyen d'une amine de formule générale (V) dans laquelle A, L et B sont tels que définis précédemment. Cette réaction peut être effectuée par chauffage des réactifs dans un solvant polaire tel que le diméthylsulfoxyde ou les alcools aliphatiques, par exemple le pentanol.

### Schéma 2 : fonctionnalisation préliminaire de la position 3 de l'imidazo[1,2-b]pyridazine

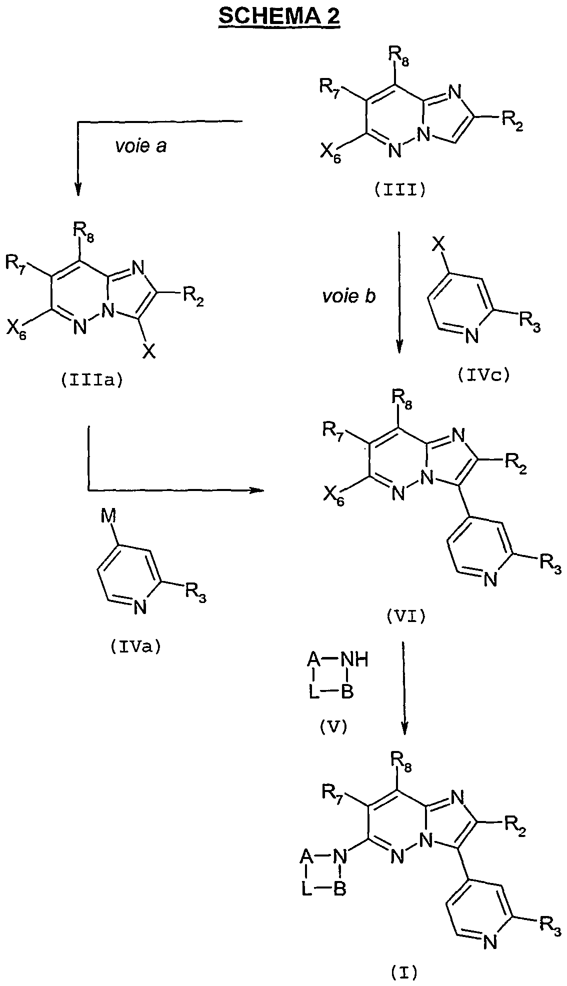

De manière générale et comme illustré dans le schéma 2, les dérivés de 2-alkyl-6-cycloamino-3-pyridin-4-yl-imidazo[1,2-*b*]pyridazine de formule générale (I) dans laquelle R₂, R₃, A, L, B, R₇ et R₈ sont tels que définis ci-dessus peuvent être préparés à partir d'un dérivé de 2-alkyl-3-pyridin-4-ylimidazo[1,2-*b*]pyridazine de formule générale (VI), dans laquelle R₂, R₃, R₇ et R₈ sont tels que définis ci-dessus et X₆ représente un groupe partant tel qu'un halogène par traitement au moyen d'une amine de formule générale (V) dans laquelle A, L et B sont tels que définis précédemment. Cette réaction peut être effectuée par chauffage des réactifs dans un solvant polaire tel que le diméthylsulfoxyde ou les alcools aliphatiques, par exemple le pentanol.

### Voie a : Introduction de la pyridine - couplage de type « Stille » ou « Suzuki »

Les dérivés de 2-alkyl-3-pyridin-4-ylimidazo[1,2-*b*]pyridazine de formule générale (VI) tels que définis ci-dessus peuvent être préparés en deux étapes à partir d'un dérivé de 2-alkyl-imidazo[1,2-b]pyridazine de formule générale (III) tel que définis précédemment :
Le dérivé (III) est bromé ou iodé sélectivement en position 3 par traitement au moyen de *N-*bromo- ou iodosuccinimide ou de monochlorure d'iode dans un solvant polaire tel que l'acétonitrile, le tétrahydrofurane, le méthanol ou le chloroforme pour fournir le dérivé de 2-alkyl-3-iodo- ou 3-bromo-imidazo[1,2-*b*]pyridazine de formule générale (IIIa), dans laquelle R₂, R₇ et R₈ sont tels que définis ci-dessus et X₆ représente un groupe partant tel qu'un halogène. Celui-ci est transformé en dérivé (VI) par couplage tel que défini précédemment selon les conditions de Stille ou de Suzuki avec un stannane ou un boronate de pyridine de formule générale (IVa) dans laquelle R₃ est tel que défini ci-dessus et M représente un groupe trialkylstannyle, le plus fréquemment un groupement tributylstannyle ou un groupe dihydroxyboryle ou dialkyloxyboryle, le plus fréquemment un groupe 4,4,5,5-tétraméthyl-1,3,3,2-dioxaborolan-2-yle.

Les couplages selon la méthode de Stille sont par exemple réalisés par chauffage en présence d'un catalyseur tel que le tétrakis(triphénylphosphine)palladium, d'iodure de cuivre dans un solvant tel que le N,N-diméthylacétamide.

Les couplages selon la méthode de Suzuki sont par exemple réalisés par chauffage en présence d'un catalyseur tel que le 1,1'-bis (diphénylphosphino)ferrocènedichloropalladium, d'une base minérale telle que le carbonate de césium dans un mélange de solvant tels que le tétrahydrofurane et l'eau.

### voie b : Introduction de la pyridine - C-H arylation métallocatalysée

Alternativement, les dérivés de 2-alkyl-3-pyridin-4-ylimidazo[1,2-*b*]pyridazine de formule générale (VI) tels que définis ci-dessus peuvent être directement préparés en une étape à partir d'un dérivé de 2-alkyl-imidazo[1,2-*b*]pyridazine de formule générale (III) tel que définis précédemment par couplage métallo-catalysé avec un dérivé de pyridine de formule générale (IVc) dans laquelle R₃ est tels que défini précédemment et X représente un atome d'halogène, plus particulière l'iode. Ce couplage peut être réalisé en présence d'un catalyseur tel que l'acétate de Palladium, d'une base minérale telle que le carbonate de potassium et dans un solvant polaire aprotique tel que le diméthylformamide.

Dans le cas particulier des composés de structure générale (I) ou (VI) pour lequels R₃ représente un groupe -NR₄R₅, et le groupe R₅ représente un groupe C₁₋₄ alkyle, C₃₋₇-cycloalkyle, C₃₋₇-cycloalkyle-C₁₋₆-alkyle, ces composés peuvent être préparés par alkylation du précurseur correspondant ou le groupe R₅ représentent un hydrogène au moyen d'un halogénure d'alkyle de formule R₅-X pour lequel X représente un groupe partant tel que défini ci-dessus. Cette réaction peut être réalisée par chauffage en présence d'une base comme l'hydrure de sodium et dans un solvant tel que le diméthylformamide ou le tétrahydrofurane. Dans le cas très particulier ou les groupes R₄ et R₅ représentent chacun un hydrogène, une monoalkylation peut être réalisée en utilisant un groupe protecteur tel qu'un tertio-butyloxycarbonyle pour masquer temporairement l'un des deux hydrogènes.

### Synthèse des précurseurs

Les dérivés de 2-alkyl-imidazo[1,2-b]pyridazine de formule générale (III) dans laquelle R₂, R₇ et R₈ sont tels que définis ci-dessus et X₆ représente un groupe partant sont connus ou peuvent être préparés par analogie avec des méthodes décrites dans la littérature (Abignente, Enrico ; Caprariis, Paolo de ; Patscot, Rosaria ; Sacchi, Antonella ; J. Heterocycl. Chem.; 23; 1986; 1031-1034 ; Barlin, Gordon B.; Davies, Les P.; Ireland, Stephen J.; Ngu, Maria M. L.; Zhang, Jiankuo ; Aust. J. Chem.; EN; 45; 4; 1992; 731-749 ; Mourad, Alaa E.; Wise, Dean S.; Townsend, Leroy B.; J. Heterocycl. Chem.; 30; 5; 1993; 1365-1372 ; Pollak et al.; Tetrahedron; 24; 1968; 2623 ; Hervet, Maud ; Galtier, Christophe ; Enguehard, Cécile; Gueiffier, Alain ; Debouzy, Jean-Claude ; Journal of Heterocyclic Chemistry (2002), 39(4), 737-742).

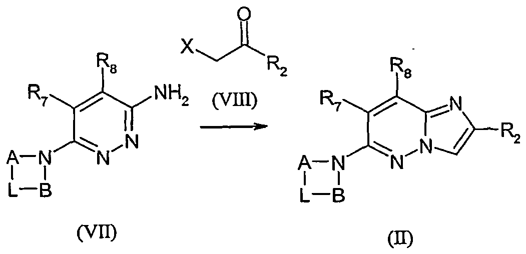

Les dérivés de 2-alkyl-6-cycloamino-3-pyridin-4-ylimidazo[1,2-b]pyridazine de formule générale (II) dans laquelle R₂, A, L, B, R₇ et R₈ sont tels que définis ci-dessus peuvent être préparés par analogie avec des méthodes décrites dans la littérature (par exemple, Watanabe et al.; Synthesis; 1977; 761 ; Jurgee et al.; J. Heterocycl. Chem.; 12; 1975; 253,255. ; WerbeI,L.M.; Zamora,M.L.; J. Heterocycl. Chem.; 2; 1965; 287-290; Yoneda et al.; Chem. Pharm. Bull.; 12; 1964; 1351,1353,1354 ; Tomoyasu; lizawa, Yuji; Okonogi, Kenji; Miyake, Akio; J. Antibiot.; 53; 10; 2000; 1053 -1070).

Ils sont usuellement préparés par condensation entre un dérivé de pyridazin-3-ylamine de formule générale (VII) dans laquelle A, L, B, R₇ et R₈ sont tels que définis ci-dessus et un dérivé de 2-bromo, chloro- ou iodoéthan-1-one de formule générale (VIII) dans laquelle R₂ est tel que défini ci-dessus.

La réaction peut être effectuée par chauffage des réactifs dans un solvant polaire tel que l'éthanol ou le butanol.

Dans les schémas de synthèse qui précédent, les composés de départ et les réactifs, quand leur mode de préparation n'est pas décrit, sont disponibles dans le commerce ou décrits dans la littérature, ou bien peuvent être préparés selon des méthodes qui y sont décrites ou qui sont connues de l'Homme du métier.

### Groupes protecteurs

Pour les composés de formule générale (I), (II), (IIa) ou (IIc) telles que définies ci-dessus et dans le cas où le groupement N-A-L-B comporte une fonction amine primaire ou secondaire, cette fonction peut éventuellement être protégée lors de la synthèse par des groupements protecteurs connus de l'homme de l'art, par exemple un benzyle ou un *tertio-*butyloxycarbonyle.

Pour les composés de formule générale (I), (VI) telles que définies ci-dessus et dans le cas où le groupement R₃ comporte une fonction amine primaire ou secondaire, cette fonction peut éventuellement être protégée par des groupements protecteurs connus de l'homme de l'art, par exemple un benzyle ou un t-butyloxycarbonyle.

Les produits de structure générale (I) tels que définis ci-dessus sont obtenus selon les procédés décrits après une étape finale supplémentaire de déprotection du groupement protecteur selon les conditions usuelles connues de l'Homme de l'art.

Les exemples suivants décrivent la préparation de certains composés conformes à l'invention. Ces exemples ne sont pas limitatifs et ne font qu'illustrer la présente invention. Les numéros des composés exemplifiés renvoient à ceux donnés dans le tableau ci-après, qui illustre les structures chimiques et les propriétés physiques de quelques composés selon l'invention.

### Exemple 1 (composé n°39) : 2-méthyl-6-(4-méthyl-pipérazin-1-yl)-3-pyridin-4-yl-imidazo[1,2-b]pyridazine

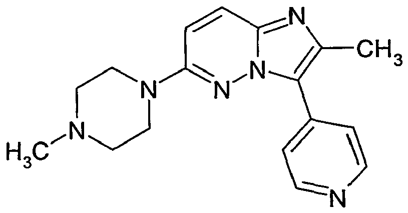

### Etape 1.1. 2-Méthyl-6-(4-méthyl-pipérazin-1-yl)-imidazo[1,2-b]pyridazine

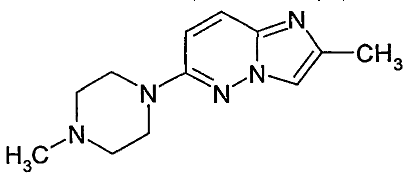

A une solution de 4,40 g (26,3 mmoles) de 6-chloro-2-méthyl-imidazo[1,2-*b*]pyridazine (CAS 14793-00-1 ; Mourad, Alaa E.; Wise, Dean S.; Townsend, Leroy B.; J. Heterocycl. Chem.; 30; 5; 1993; 1365-1372) dans 58 ml de 1-méthylpipérazine est chauffé à reflux pendant 24 heures. Le mélange est ensuite versé dans de l'eau et le produit est extrait avec du dichlorométhane. Les phases organiques sont rassemblées, séchées sur sulfate de sodium et concentrées sous pression réduite. L'huile marron obtenue est purifiée par chromatographie sur gel de silice en éluant avec un mélange de dichlorométhane, de méthanol et d'ammoniaque (95/5/0,5) pour conduire à 4,79 g de poudre beige après recristallisation dans l'éther di-isopropylique et séchage.
PF : 108-110°C
RMN ¹H (CDCl₃) δ : 7,65 (d, 1H), 7,50 (s, 1H), 6,80 (d, 1H), 3,55 (m, 4H), 2,60 (m, 4H), 2,45 (s, 3H), 2,40 (s, 3H) ppm.

### Etape 1.2. 3-Iodo-2-méthyl-6-(4-méthyl-pipérazin-1-yl)-imidazo[1,2-b]pyridazine

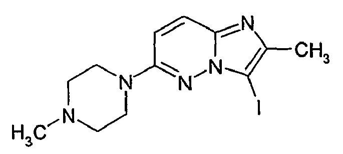

A une solution de 5,50 g (23,8 mmoles) de 2-méthyl-6-(4-méthyl-pipérazin-1-yl)-imidazo[1,2-*b*]pyridazine dans 100 ml de dichlorométhane, on additionne 5,89 g (26,2 mmoles) de N-iodosuccinimide. Le mélange est agité pendant une heure et demie à température ambiante puis le solvant est évaporé sous pression réduite. Le résidu solide obtenu est repris avec une solution aqueuse à 5% de thiosulfate de sodium et le produit est extrait avec du dichlorométhane. Les phases organiques sont rassemblées, séchées sur sulfate de sodium et concentrées sous pression réduite pour conduire à 6,37g de poudre beige après recristallisation dans l'acétonitrile, rinçage avec de l'éther diéthylique et séchage.
PF: 136-138°C
RMN ¹H (CDCl₃) δ : 7,55 (d, 1H), 6,80 (d, 1H), 3,60 (m, 4H), 2,60 (m, 4H), 2,50 (s, 3H), 2,40 (s, 3H) ppm.

### Etape 1.3. 2-Méthyl-6-(4-méthyl-pipérazin-1-yl)-3-pyridin-4-yl-imidazo[1,2-b]pyridazine

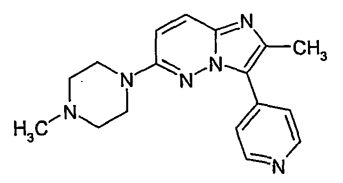

A un mélange de 0,50 g (1,40 mmole) de 3-iodo-2-méthyl-6-(4-méthyl-pipérazin-1-yl)-imidazo[1,2-*b*]pyridazine, de 0,207 g (1,68 mmole) d'acide (pyridin-4-yl)boronique, de 0,30 g (2,8 mmoles) de carbonate de sodium et de 46 mg (0,06 mmole) de complexe de 1,1'-bis(diphénylphosphino))ferrocènedichloropalladium (II) et de dichlorométhane (PdCl₂(dppf).CH₂Cl₂) dans 10 ml d'un mélange de diméthoxyéthane et d'eau (7/3), on additionne 3,0 g (9,3 mmoles) de carbonate de césium et on porte au reflux sous argon pendant 18 heures. On additionne alors de 1,72 g (1,40 mmole) d'acide (pyridin-4-yl)boronique et de 46 mg (0,06 mmole) de complexe de 1,1'-bis(diphénylphosphino)ferrocènedichloropalladium (II) et de dichlorométhane. La réaction est poursuivie pendant 3 heures supplémentaires à reflux puis le mélange est alors versé dans 200 ml d'eau. Le produit est alors extrait avec du dichlorométhane, la phase organique séchée sur sulfate de sodium, filtrée et le solvant évaporé sous pression réduite pour donner un solide marron. Le produit est purifié par chromatographie sur gel de silice en éluant avec un mélange de dichlorométhane, de méthanol et d'ammoniaque (95/5/0,5) pour donner 0,308 g de poudre beige après recristallisation dans l'acétonitrile, rinçage avec de l'éther di-isopropylique et séchage.
PF: 150-152°C
RMN ¹H (CDCl₃) δ : 8,70 (d, 2H), 7,7 (m, 3H), 6,90 (d, 1H), 3,55 (m, 4H), 2,65 (s, 3H), 2,60 (m, 4H), 2,40 (s, 3H) ppm.

### Etape 1.4. Stratégie alternative : 2-Méthyl-6-(4-méthyl-pipérazin-1-yl)-3-pyridin-4-yl-imidazo[1,2-b]pyridazine

Un mélange de 1,00 g (4,32 mmoles) de 2-méthyl-6-(4-méthyl-pipérazin-1-yl)-imidazo[1,2-b]pyridazine, de 1,06 g (5,19 mmoles) de 4-iodopyridine, de 0,717 g (5,19 mmoles) de carbonate de sodium et de 49 mg d'acétate de Palladium dans 10 ml de diméthylformamide est porté à 140 °C pendant 18 heures. Après refroidissement, le mélange est versé dans de l'eau. On ajoute de l'acétate d'éthyle et le mélange est filtré sur Buchner. La phase organique est séparée, lavée avec de l'eau, séchée sur sulfate de sodium. Le solvant est éliminé sous pression réduite et le résidu est purifié par chromatographie sur colonne de gel de silice en éluant avec un mélange de dichlorométhane de méthanol et d'ammoniaque (98/2/0,2) pour donner 0,7 g de produit après séchage.
PF: 154-156°C

### Exemple 2 (composé n° 19) : 2-Cyclobutyl-6-pipérazin-1-yl-3-pyridin-4-yl-imidazo[1,2-b]pyridazine

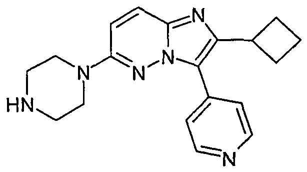

### Etape 2.1. 6-(4-Benzyl-pipérazin-1-yl)-pyridazin-3-ylamine

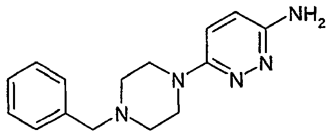

48,9 g (278 mmoles) de 1-benzylpipérazine et 12,0 g (92,6 mmoles) de 3-amino-6-chloropyridazine sont chauffés à 160°C pendant 1 heure. L'huile marron obtenue est versée dans 500 ml d'une solution de bicarbonate de sodium aqueuse et le produit est extrait avec du dichlorométhane. La phase organique est séchée puis concentrée sous pression réduite. L'huile obtenue est triturée dans l'éther diéthylique et 20,5 g de solide sont isolés après filtration et séchage.
Rendement : 82%
RMN ¹H (CDCl₃) δ : 7,45-7,65 (m, 6H), 7,20 (s, 2H), 5,5 (massif large, 2H) 3,80 (s, 2H), 3,60-3,75 (m, 4H), 2,80-2,85 (m, 4H) ppm.

### Etape 2.2. 6-(4-Benzyl-pipérazin-1-yl)-2-cyclobutyl-imidazo[1,2-b]pyridazine

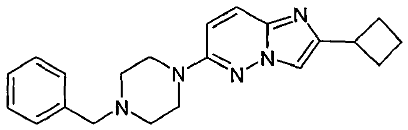

Une solution de 2,0 g (7,4 moles) 6-(4-benzyl-pipérazin-1-yl)-pyridazin-3-ylamine obtenu à l'étape 2.1. et de 1,6 g (8,9 mmoles) de 2-bromo-1-cyclobutyl-éthanone (CAS : 128312-69-6) dans 15 ml de n-butanol est chauffée à 90°C pendant 1 heure 40. Le milieu est ensuite versé dans 250 ml d'une solution aqueuse d'hydrogénocarbonate de sodium et le produit est extrait avec de l'acétate d'éthyle. La phase organique est séchée sur sulfate de sodium, filtrée et le solvant évaporé sous pression réduite. L'huile marron obtenue est purifiée par chromatographie sur 90 g de gel de silice en éluant avec un mélange de dichlorométhane de méthanol et d'ammoniaque (97/3/0,3) pour conduire à 1,6 g d'huile jaune.
Rendement : 62%
RMN ¹H (CDCl₃) δ : 7,65 (d, 1H), 7,50 (s, 1H), 7,20-7,40 (m, 5H), 6,75 (d, 1H), 3,75 (m, 1H), 3,60 (s, 2H), 3,45-3,55 (m, 4H), 2,50-2,65 (m, 4H), 1,8-2,5 (m, 6H) ppm.

### Etape 2.3. 4-[6-(4-Benzyl-pipérazin-1-yl)-2-cyclobutyl-imidazo[1,2-b]pyridazin-3-yl]-4H-pyridine-1-carboxylate d'éthyle

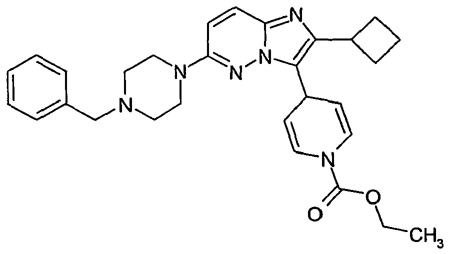

A une solution de 1,6 g (4,6 mmoles) de 6-(4-benzyl-pipérazin-1-yl)-2-cyclobutyl-imidazo[1,2-b]pyridazine obtenu à l'étape 2.2. dans 15 ml de pyridine refroidie à 0°C, on ajoute goutte à goutte 8,8 ml (92 mmoles) de chloroformiate d'éthyle en maintenant la température à 0°C. On laisse alors le mélange revenir à température ambiante tandis que le précipité formé disparaît.

Le mélange est à nouveau refroidi à 0°C et on additionne au goutte à goutte 8,8 ml (92 mmoles) de chloroformiate d'éthyle en maintenant la température à 0°C. On laisse à nouveau le mélange revenir à température ambiante tandis que le précipité formé disparaît. Le mélange est ensuite versé dans 300 ml d'eau et le produit est extrait avec de l'acétate d'éthyle. La phase organique est lavée avec de l'eau, séchée sur sulfate de sodium puis concentrée sous pression réduite. Le résidu est ensuite co-évaporé plusieurs fois avec du toluène puis le produit est cristallisé et enfin recristallisé dans l'acétonitrile.

On isole 1,0 g de poudre blanche après filtration et séchage.
PF : 140-412°C
Rendement : 45%
RMN ¹H (CDCl₃) δ : 7,60 (d, 1H), 7,10-7,30 (m, 5H), 6,80-7,00 (m, 2H), 6,65 (d, 1H), 4,60-4,9 (m, 3H), 4,00-4,25 (m, 2H), 3,7 (m, 1H), 3,50 (s, 2H), 3,25-3,40 (m, 4H), 2,05-2,50 (m, 8H), 1,75-2,05 (m, 2H), 1,20 (t, 3H) ppm.

### Etape 2.4. 6-(4-Benzyl-pipérazin-1-yl)-2-cyclobutyl-3-pyridin-4-yl-imidazo[1,2-b]pyridazine

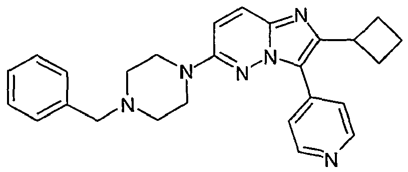

A une solution de 1,0 g (2,1 mmoles) 4-[6-(4-benzyl-pipérazin-1-yl)-2-cyclobutyl-imidazo[1,2-*b*]pyridazin-3-yl]-4*H*-pyridine-1-carboxylate d'éthyle obtenu à l'étape 2.3. dans 8 ml de toluène, on additionne goutte à goutte 0,55 g (2,25 mmoles) d'ortho-chloranile en solution dans 4 ml de toluène (solution rouge). Après la fin de l'addition, on laisse le milieu sous agitation à température ambiante pendant 30 minutes puis on le verse sur une solution aqueuse de soude 2N. Le produit est extrait avec de l'acétate d'éthyle, la phase organique est lavée avec de l'eau, séchée sur sulfate de sodium puis concentrée sous pression réduite. L'huile noire obtenue est purifiée par chromatographie sur 50 g de gel de silice en éluant avec un mélange de dichlorométhane de méthanol et d'ammoniaque (95/5/0,5) pour conduire à 0,87 g d'huile jaune.
Rendement: 100%
RMN ¹H (CDCl₃) δ : 8,70 (m, 2H), 7,85 (d, 1H), 7,70 (m, 2H), 7,25-7,45 (m, 5H), 6,90 (d, 1H), 3,90 (q, 1H), 3,60 (s, 2H), 3,55 (m, 4H), 2,50-2,75 (m, 6H), 2,30-2,50 (m, 2H), 1,95-2,20 (m, 2H) ppm.

### Etape 2.5. 2-Cyclobutyl-6-pipérazin-1-yl-3-pyridin-4-yl-imidazo[1,2-b]pyridazine

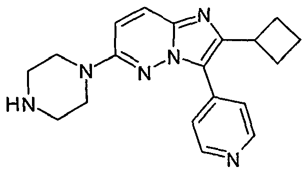

A une solution de 0,86 g (2,0 mmoles) de 6-(4-benzyl-pipérazin-1-yl)-2-cyclobutyl-3-pyridin-4-yl-imidazo[1,2-*b*]pyridazine obtenu à l'étape 2.4. dans 20 ml de méthanol, on additionne 1,9 g de formiate d'ammonium et 0,7 g de palladium sur charbon (10%) contenant 50% d'humidité. Le mélange est porté à reflux pendant 1 heure puis le catalyseur est éliminé par filtration sur Büchner et rinçage avec du méthanol. Le solvant est éliminé par évaporation, le résidu obtenu repris avec du dichlorométhane, lavé avec de l'eau. La phase organique est lavée avec de l'eau, séchée sur sulfate de sodium puis concentrée sous pression réduite pour conduire à un solide légèrement marron-jaune.

On isole 0,33 g de poudre blanche après recristallisation dans l'acétonitrile, filtration et séchage.
PF : 189-191°C
RMN ¹H (CDCl₃) δ : 8,70 (pseudo dd, 2H), 7,80 (d, 1H), 7,65 (pseudo dd, 2H), 6,85 (d, 1H), 3,85 (m, 1H), 3,50 (m, 4H), 3,05 (m, 4H), 2,45-2,75 (m, 2H), 2,25-2,45 (m, 2H), 1,85-2,25 (m, 2H) ppm.

### Exemple 3 (composé n° 49) : 6-(3,3-diméthyl-pipérazin-1-yl)-2-méthyl-3-(2-méthyl-pyridin-4-yl)-imidazo[1,2-b]pyridazine

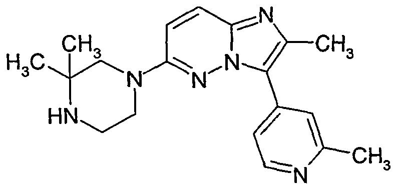

### Etape 3.1. 6-Chloro-3-iodo-2-méthyl-imidazo[1,2-b]pyridazine

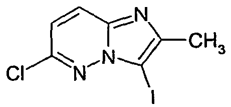

A une solution de 7,00 g (41,8 mmoles) de 6-chloro-2-méthyl-imidazo[1,2-*b*]pyridazine (CAS 14793-00-1) dans 300 ml de chloroforme refroidie à 0°C, on ajoute 10,2 g (62,7 mmoles) de monochlorure d'iode en solution dans 20 ml de méthanol. La réaction est ensuite laissée à température ambiante pendant 16 heures puis versée sur un mélange d'une solution aqueuse de thiosulfate de sodium 5% et d'hydrogénocarbonate de sodium. Le produit est extrait avec du dichlorométhane, la phase organique est séchée sur sulfate de sodium et le solvant évaporé sous pression réduite.
Le résidu solide est trituré avec de l'acétonitrile puis isolé par filtration pour donner 8,5 g d'un solide jaune après séchage.
RMN ¹H (CDCl₃) δ : 7,80 (d, 1H), 7,10 (d, 1H), 2,55 (s, 3H) ppm.

### Etape 3.2 : 6-Chloro-2-méthyl-3-(2-méthyl-pyridin-4-yl)-imidazo[1,2-b]pyridazine

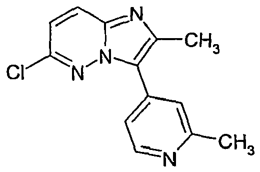

7,70 g (26,2 mmoles) de 6-chloro-3-iodo-2-méthyl-imidazo[1,2-b]pyridazine, 4,31 g (31,5 mmoles) d'acide 2-méthyl-pyridin-4-ylboronique obtenu à l'étape 3.2., 25,6 g (78,7 mmoles) de carbonate de césium et 1,93 g ( 2,36 mmoles) de complexe de 1,1'-bis(diphénylphosphino)ferrocènedichloro palladium (II) et de dichlorométhane (PdCl2(dppf).CH₂Cl₂) dans 480 ml d'un mélange de tétrahydrofurane et d'eau (90/10) sont chauffés 18 heures à reflux. Le mélange est versé dans de l'eau et le produit extrait avec de l'acétate d'éthyle. La phase organique est séchée sur sulfate de sodium et le solvant évaporé sous pression réduite. Le résidu est purifié sur 220 g de gel de silice en éluant avec un mélange de dichlorométhane, de méthanol et d'ammoniaque (98/2/0,2) pour donner 5,0 g d'un solide jaune. Ce solide est repris avec un mélange d'acide chlorhydrique aqueux et d'acétate d'éthyle. La phase aqueuse est séparée, neutralisée avec de l'hydrogénocarbonate de sodium et le produit est extrait au moyen de chloroforme. La phase organique est séchée sur sulfate de sodium et le solvant évaporé sous pression réduite pour conduire à 4,6 g d'un solide blanc.
RMN ¹H (CDCl₃) δ : 8,80 (d, 1H), 7,90 (d, 1H), 7,55 (s, 1H), 7,50 (d, 1H), 7,15 (d, 1H), 2,80 (s, 3H), 2,75 (s, 3H) ppm.

### Etape 3.3. 6-(3,3-diméthyl-pipérazin-1-yl)-2-méthyl-3-(2-méthyl-pyridin-4-yl)-imidazo[1,2-b]pyridazine

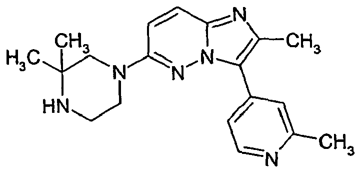

Une solution de 0,30 g (1,2 mmoles) de 6-chloro-2-méthyl-3-(2-méthyl-pyridin-4-yl)-imidazo[1,2-*b*]pyridazine obtenu à l'étape 3.2. et 0,40 g (3,5 mmoles) de 2,2-diméthyl-pipérazine dans 5 ml de pentanol est porté au reflux pendant 48 heures. Le pentanol est alors partiellement évaporé sous pression réduite et le milieu est repris avec une solution aqueuse d'acide chlorhydrique. Cette phase aqueuse est lavée avec de l'acétate d'éthyle puis basifiée au moyen d'une solution de soude aqueuse et le produit est extrait avec du dichlorométhane. La phase aqueuse est séchée sur sulfate de sodium et le solvant est chassé sous pression réduite. Le résidu est purifié sur 15 g de gel de silice en éluant avec un mélange de dichlorométhane, de méthanol et d'ammoniaque (94/4/0,4) pour donner 0,12 g d'un solide blanc.
PF : 110-112°C
RMN ¹H (CDCl₃) δ : 8,60 (d, 1H), 7,70 (d, 1H), 7,65 (s, 1H), 7,55 (d, 1H), 6,80 (d, 1H), 3,45 (m, 2H), 3,25 (s, 2H), 3,10 (m, 2H), 2,65 (s, 3H), 2,60 (s, 3H), 1,2 (s, 6H) ppm.

### Exemple 4 (composé n° 30) : (-)-3-(2-Méthoxy-pyridin-4-yl)-2-méthyl-6-((R)-3-méthyl-pipérazin-1-yl)-imidazo[1,2-b]pyridazine

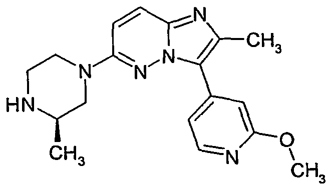

### Etape 4.1. : 6-Chloro-2-méthyl-3-(2-méthoxy-pyridin-4-yl)-imidazo[1,2-b]pyridazine

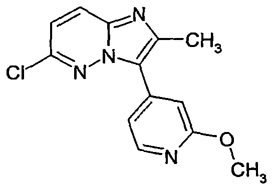

Un mélange de 1,15 g (3,92 mmoles) de 6-chloro-3-iodo-2-méthyl-imidazo[1,2-b]pyridazine, 0,72 g (4,7 mmoles) d'acide 2-méthoxy-pyridin-4-ylboronique et 3,8 g (12 mmoles) de carbonate de césium dans 75 ml d'un mélange de tétrahydrofurane et d'eau (90/10) est purgé avec de l'argon puis 0,29 g ( 0,35 mmoles) de complexe de 1,1'-bis(diphénylphosphino)ferrocènedichloro palladium (II) et de dichlorométhane (PdCl2(dppf).CH₂Cl₂) sont additionnés. Après 16 heures de chauffage à reflux, le mélange est versé dans une solution aqueuse d'acide chlorhydrique 1 N glacée, la phase aqueuse est lavée avec de l'acétate d'éthyle puis basifiée par addition de bicarbonate de sodium. Le produit est ensuite extrait avec du dichlorométhane. La phase organique est séchée sur sulfate de sodium et le solvant évaporé sous pression réduite. Le résidu solide est purifié sur 35 g de gel de silice en éluant avec un mélange de dichlorométhane, de méthanol et d'ammoniaque (98/2/0,2) pour donner 0,77 g d'un solide blanc.
PF: 132-134°C
RMN ¹H (CDCl₃) δ : 8,35 (d, 1H), 7,90 (d, 1H), 7,3 (d, 1H), 7,25 (s, 1H), 7,15 (d, 1H), 4,05 (s, 3H), 2,65 (s, 3H) ppm.

### (-)-3-(2-Méthoxy-pyridin-4-yl)-2-méthyl-6-((R)-3-méthyl-pipérazin-1-yl)-imidazo[1,2-b]pyridazine

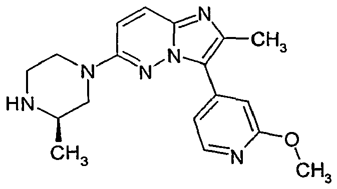

Une solution de 0,35 g (1,3 mmole) de 6-chloro-2-méthyl-3-(2-méthoxy-pyridin-4-yl)-imidazo[1,2-*b*]pyridazine obtenu à l'étape 4.1. et de 0,38 g (3,8 mmoles) de (*R*)-2-méthylpipérazine dans 5 ml de pentanol est chauffé à 150°C pendant 24 heures. On additionne alors 0,1 g (1,0 mmoles) de (R)-2-méthyl-pipérazine supplémentaire et le chauffage est poursuivi pendant 24 heures. Le milieu est versé dans une solution aqueuse d'acide chlorhydrique 1 N. La phase aqueuse est lavée avec de l'acétate d'éthyle puis basifiée au moyen d'une solution de bicarbonate de sodium et le produit est extrait avec du dichlorométhane. La phase organique est séchée sur sulfate de sodium et le solvant est chassé sous pression réduite. Le résidu huileux est purifié sur 25 g de gel de silice en éluant avec un mélange de dichlorométhane, de méthanol et d'ammoniaque (95/5/0,5) pour donner 0,15 g d'un solide blanc.
PF: 104-106°C
[α]_{D} (c=1 ; CH₂Cl₂)= -17,1°
RMN ¹H (CDCl₃) δ : 8,25 (d, 1H), 7,70 (d, 1H), 7,40 (d, 1H), 7,25 (s, 1H), 6,85 (d, 1H), 3,9-4,1 (m, 2H), 4,00 (s, 3H), 2,85-3,2 (m, 4H), 2,60 (s, 3H), 2,6 (m, 1H), 2,0 (m large, 1H), 1,15 (d, 3H) ppm.

### Exemple 5 (composé n°68) : {4-[6-(Hexahydro-pyrrolo[3,4-c]pyrrol-2(1H)-yl)-2-méthyl-imidazo[1,2-b]pyridazin-3-yl]-pyridin-2-yl}-amine

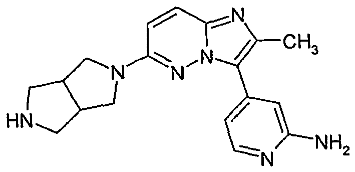

### Etape 5.1.: [4-(4,4,5,5-tétraméthyl-1,3,2-dioxaborolan-2-yl)pyridin-2-yl]carbamate de tert-butyle

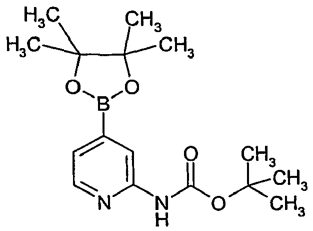

A une solution de 6,76 (24,8 mmoles) de (4-bromo-pyridin-2-yl)carbamate de *tert*-butyle (Deady, Leslie W.; Korytsky, Olga L.; Rowe, Jeffrey E.; Aust. J. Chem.; 35; 10; 1982; 2025-2034) dans 150 ml de diméthylformamide on additionne 8,0 g (81 mmoles) d'acétate de potassium préalablement séché à 130°C et 6,9 g (27 mmoles) de bis(pinacolato)diboron. On fait ensuite barboter un courant d'argon pendant quelques instants et on ajoute 1,2 g (1,5 mmoles) de 1,1'-bis(diphénylphosphino))ferrocènedichloropalladium (II). Le mélange est agité à 80°C sous argon, pendant 2 heures puis versé sur une solution saturée aqueuse de chlorure d'ammonium. Le produit est extrait avec de l'acétate d'éthyle, la phase organique est séchée sur sulfate de sodium et le solvant est chassé sous pression réduite. Le résidu est trituré dans 300 ml d'éther di-isopropylique à reflux et l'insoluble est séparé par filtration. Le filtrat est refroidi, partiellement concentré sous pression réduite. Après addition de 70 ml d'hexane, le précipité formé est isolé par filtration pour conduire à 4,2 g de solide orangé après séchage.
PF : 188-193°C
RMN ¹H (CDCl₃) δ : 8,15 (m, 2H), 7,85 (s large, 1H), 7,15 (d, 1H), 1,40 (s, 9H), 1,20 (s, 12H) ppm.

### Etape 5.2.: [4-(6-Chloro-2-méthyl-imidazo[1,2-b]pyridazin-3-yl)-pyridin-2-yl]-carbamate de tert-butyle

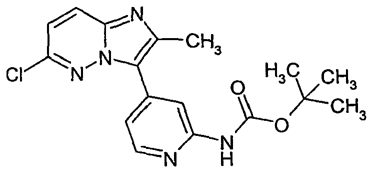

Un mélange de 3,19 g (10,9 mmoles) de 6-chloro-3-iodo-2-méthyl-imidazo[1,2-*b*]pyridazine, de 4,18 g (13,0 mmoles) de [4-(4,4,5,5-tétraméthyl-1,3,2-dioxaborolan-2-yl)pyridin-2-yl]carbamate de *tert*-butyle et de 10,6 g (32,6 mmoles) de carbonate de césium dans 250 ml d'un mélange de tétrahydrofurane et d'eau (90/10) est purgé avec de l'argon. 0,80 g ( 0,98 mmoles) de 1,1'-bis(diphénylphosphino)ferrocènedichloro palladium (II) (PdCl₂(dppf)) sont alors additionnés. La réaction est chauffée pendant 3 heures à reflux puis le solvant est évaporé sous pression réduite. Le résidu est repris avec du chloroforme et la phase organique est lavée avec une solution aqueuse saturée de chlorure d'ammonium. La phase organique est séchée sur sulfate de sodium et le solvant évaporé sous pression réduite. Le résidu solide est purifié sur 110 g de gel de silice en éluant avec un mélange de dichlorométhane, de méthanol et d'ammoniaque (97/3/0,3) pour donner 3,6 g d'un solide beige foncé. Le solide est trituré dans de l'éther diéthylique pour fournir 3,0 g de solide blanc après filtration et séchage.
PF : 260°C
RMN ¹H (CDCl₃) δ : 8,50 (d, 1H), 8,45 (s, 1H), 7,90 (d, 1H), 7,85 (s large, 1H), 7,45 (d, 1H), 7,10 (d, 1H), 2,70 (s, 3H), 1,55 (s, 9H) ppm.

### Etape 5.3. : 4-(6-Chloro-2-méthyl-imidazo[1,2-b]pyridazin-3-yl)-pyridin-2-ylamine

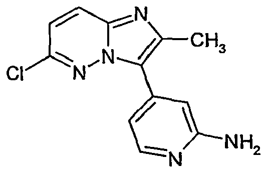

A une solution de 7,70 g (21,5 mmoles) de [4-(6-chloro-2-méthyl-imidazo[1,2-*b*]pyridazin-3-yl)-pyridin-2-yl]-carbamate de *tert*-butyle obtenu à l'étape 5.2. dans 200 ml de chloroforme on additionne 80 ml (1,1 moles) d'acide trifluoroacétique à 10°C. Le mélange est agité à température ambiante pendant 4 heures puis le solvant est évaporé sous pression réduite. L'huile brune obtenue est reprise avec une solution aqueuse d'acide chlorhydrique 3N et la phase aqueuse est lavée avec de l'éther diéthylique.
La phase aqueuse est ensuite alcalinisée par addition d'ammoniaque aqueux dilué et le précipité qui se forme est séparé par filtration. Le solide est dissout dans du chloroforme, la solution est lavée avec de l'eau, séchée sur sulfate de sodium et concentrée sous pression réduite pour conduire à un solide blanc. Celui-ci est trituré dans un mélange d'éther diéthylique et d'hexane pour donner 3,9 g de poudre blanche après filtration et séchage.
Rendement : 70%
PF : 188°C
RMN ¹H (CDCl₃) δ : 8,25 (d, 1H), 7,9 (d, 1H), 7,10 (d, 1H), 7,05 (d, 1H), 6,95 (s, 1H), 4,7 (sl, 2H), 2,65 (s, 3H) ppm.

### Etape 5.4.: {4-[6-(5-Benzyl-hexahydro-pyrrolo[3,4-c]pyrrol-2(1H)-yl)-2-méthyl-imidazo[1,2-b]pyridazin-3-yl]-pyridin-2-yl}-amine

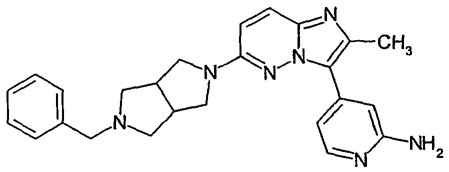

Dans un tube scellé, une solution de 0,62 g (2,4 mmoles) de 4-(6-chloro-2-méthyl-imidazo[1,2-*b*]pyridazin-3-yl)-pyridin-2-ylamine obtenu à l'étape 5.3. et de 1,5 g (7,3 moles) de 2-benzyle-octahydro-pyrrolo[3,4-c]pyrrole dans 5 ml de pentanol est chauffée à 150°C pendant 40 heures. Après refroidissement, le milieu est traité avec une solution aqueuse d'acide chlorhydrique 1 N. La phase aqueuse obtenue est lavée avec de l'éther diéthylique, puis basifiée au moyen d'ammoniaque aqueux dilué.

Le produit est extrait avec du dichlorométhane, la phase organique est séchée sur sulfate de sodium et concentrée sous pression réduite.

L'huile brune isolée est chromatographiée sur colonne de gel de silice en éluant avec un mélange de dichlorométhane, de méthanol et d'ammoniaque (95/5/0,5) pour conduire 0,89 g de solide amorphe.
Rendement : 87%
RMN ¹H (CDCl₃) δ : 8,20 (d, 1H), 7,65 (d, 1H), 7,3 (m, 5H), 7,20 (d, 1H), 7,05 (s, 1H), 6,65 (d, 1H), 4,5 (sl, 2H), 3,8-3,55 (m, 2H et s, 2H), 3,35 (dd, 2H), 3,00 (m, 2H), 2,75 (m, 2H), 2,60 (s, 3H), 2,55 (dd, 2H) ppm.

### Etape 5.5. : {4-[6-(Hexahydro-pyrrolo[3,4-c]pyrrol-2(1H)-yl)-2-méthyl-imidazo[1,2-b]pyridazin-3-yl]-pyridin-2-yl}-amine

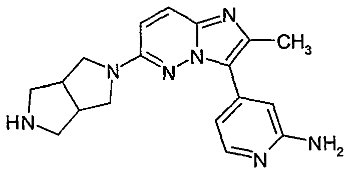

Un mélange de 0,88 g (2,1 mmoles) de {4-[6-(5-benzyl-hexahydro-pyrrolo[3,4-*c*]pyrrol-2(1*H*)-yl)-2-méthyl-imidazo[1,2-*b*]pyridazin-3-yl]-pyridin-2-yl}-amine obtenu à l'étape 5.4., de 2,0 g (31 mmoles) de formiate d'ammonium et de 1,0 g de palladium à 10% sur charbon (50% d'humidité) dans 60 ml de méthanol est agité à reflux pendant une heure. Le milieu est refroidit et filtré. Le filtrat est concentré sous pression réduite et le résidu est repris dans du dichlorométhane. La phase organique est lavée avec un minimum de solution de soude 1 N, séchée sur sulfate de sodium et concentrée sous pression réduite. Le résidu est purifié par chromatographie sur gel de silice en éluant avec un mélange de dichlorométhane de méthanol et d'ammoniaque (90/10/1) pour conduire à 0,17 g de solide beige après trituration dans un mélange de 20 ml d'éther diéthylique et de 5 ml d'acétonitrile, filtration et séchage.
PF > 155°C (décomposition) ; M+H : 336
RMN ¹H (CDCl₃) δ : 8,20 (d, 1H), 7,65 (d, 1H), 7,20 (d, 1H), 7,05 (s, 1H), 6,65 (d, 1H), 4,5 (sl, 1H), 3,7 (m, 2H), 3,5-3,1 (m, 4H), 3,1-2,8 (m, 4H), 2,60 (s, 3H), 2 (sl, 2H) ppm.

### Exemple 6 (composé n°69): {4-[6-(Hexahydro-pyrrolo[3,4-c]pyrrol-2(1H)-yl)-2-méthyl-imidazo[1,2-b]pyridazin-3-yl]-pyridin-2-yl}-méthyl-amine

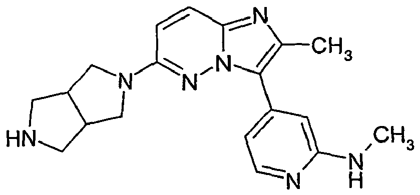

### Etape 6.1. : [4-(6-Chloro-2-méthyl-imidazo[1,2-b]pyridazin-3-yl)-pyridin-2-yl]-méthylcarbamate de tertiobutyle

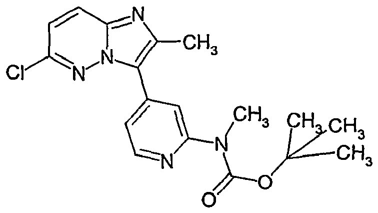

A une suspension de 0,22 g (5,6 mmoles) d'hydrure de sodium (à 60% dans l'huile) dans 35 ml de diméthylformamide sous courant d'argon et refroidie à 0°C, on additionne par portion 1,6 g (4,5 mmoles) de [4-(6-chloro-2-méthyl-imidazo[1,2-*b*]pyridazin-3-yl)-pyridin-2-yl]-carbamate de *tert*-butyle. Le mélange est agité pendant 40 minutes entre 0 et 10°C puis on additionne goutte à goutte 0,73 g (5,2 mmoles) d'iodure de méthyle dilué dans du diméthylformamide. Après 18 heures à température ambiante, le mélange est versé sur une solution aqueuse saturée de chlorure d'ammonium et le produit est extrait avec de l'acétate d'éthyle. La phase organique est séchée sur sulfate de sodium concentrée sous pression réduite pour donner une huile brune qui est chromatographiée sur colonne de gel de silice en éluant avec un mélange de dichlorométhane de méthanol d'ammoniaque (98/2/0,2). On isole ainsi 1,1 g de poudre beige après cristallisation dans l'hexane, filtration et séchage.
PF : 117-120°C
RMN ¹H (CDCl₃) δ : 8,55 (d, 1H), 8,1 (s, 1H), 7,90 (d, 1H), 7,45 (d, 1H), 7,1 (d, 1H), 3,50 (s, 3H), 2,70 (s, 3H), 1,55 (s, 9H) ppm.

### Etape 6.2. : [4-(6-Chloro-2-méthyl-imidazo[1,2-b]pyridazin-3-yl)-pyridin-2-yl]-méthyl-amine

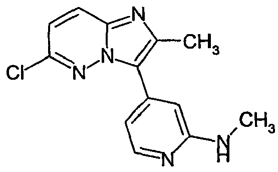

A une solution de 1,03 g (2,76 mmoles) de [4-(6-chloro-2-méthyl-imidazo[1,2-*b*]pyridazin-3-yl)-pyridin-2-yl]-méthyl-carbamate de *tert*-butyle obtenu à l'étape 6.1. dans 20 ml de chloroforme on additionne 20 ml (270 mmoles) d'acide trifluoroacétique à 10°C. Le mélange est agité à température ambiante pendant 18 heures puis le solvant est évaporé sous pression réduite. L'huile brune obtenue est triturée avec 30 ml d'éther diéthylique. Le solide formé est séparé par filtration. Il est ensuite dissout dans du chloroforme, la solution est lavée avec de l'ammoniaque dilué, séchée sur sulfate de sodium et concentrée sous pression réduite pour conduire à 0,70 g de poudre beige après séchage.
PF : 268-274°C
RMN ¹H (CDCl₃) δ : 8,25 (d, 1H), 7,85 (d, 1H), 7,10 (d, 1H), 6,95 (d, 1H), 6,85 (d, 1H), 4,85 (si, 1H), 3,00 (d, 3H), 2,65 (s, 3H) ppm.

### Etape 6.3.: {4-[6-(5-Benzyl-hexahydro-pyrrolo[3,4-c]pyrrol-2-yl)-2-méthyl-imidazo[1,2-b]pyridazin-3-yl]-pyridin-2-yl}-méthyl-amine

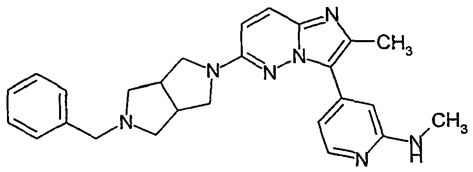

Dans un tube scellé, une solution de 0,67 g (2,5 mmoles) [4-(6-chloro-2-méthyl-imidazo[1,2-*b*]pyridazin-3-yl)-pyridin-2-yl]-méthyl-amine obtenue à l'étape 6.2. et de 1,5 g (7,3 mmoles) de 2-benzyl-octahydro-pyrrolo[3,4-*c*]pyrrole dans 5 ml de pentanol est chauffée à 150°C pendant 40 heures. Après refroidissement, le milieu est traité avec une solution aqueuse d'acide chlorhydrique 1N. La phase aqueuse obtenue est lavée avec de l'éther diéthylique puis basifiée au moyen d'ammoniaque aqueux dilué.

Le produit est extrait avec du dichlorométhane, la phase organique est séchée sur sulfate de sodium et concentrée sous pression réduite.

L'huile brune isolée est chromatographiée sur colonne de gel de silice en éluant avec un mélange de dichlorométhane, de méthanol et d'ammoniaque (95/5/0,5) pour conduire 0,98 g de solide amorphe.
RMN ¹H (CDCl₃) δ : 8,20 (d, 1H), 7,65 (d, 1H), 7,3 (m, 5H), 7,10 (d, 1H), 7,05 (s, 1H), 6,65 (d, 1H), 4,7 (dl, 1H), 3,8-3,55 (m, 2H et s, 2H), 3,4 (dd, 2H), 2,95 (m, 5H), 2,75 (m, 2H), 2,65 (s, 3H), 2,55 (dd, 2H) ppm.

### Etape 6.4. {4-[6-(Hexahydro-pyrrolo[3,4-c]pyrrol-2(1H)-yl)-2-méthyl-imidazo[1,2-b]pyridazin-3-yl]-pyridin-2-yl}-méthyl-amine

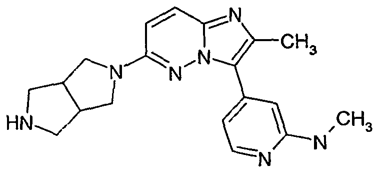

Un mélange de 0,97 g (2,2 mmoles) de {4-[6-(5-benzyl-hexahydro-pyrrolo[3,4-c]pyrrol-2(1*H*)-yl)-2-méthyl-imidazo[1,2-*b*]pyridazin-3-yl]-pyridin-2-yl}-méthyl-amine obtenu à l'étape 6.4., de 2,1 g (33 mmoles) de formiate d'ammonium et de 1,0 g de palladium à 10% sur charbon (50% d'humidité) dans 60 ml de méthanol est agité à reflux pendant une heure. Le milieu est refroidit et filtré. Le filtrat est concentré sous pression réduite et le résidu est repris dans du dichlorométhane. La phase organique est lavée avec un minimum de solution de soude 1 N, séchée sur sulfate de sodium et concentrée sous pression réduite. Le résidu est purifié par chromatographie sur gel de silice en éluant avec un mélange de dichlorométhane de méthanol et d'ammoniaque (90/10/1) pour conduire à 0,40 g de solide beige (contenant 20% d'éther en mole) après trituration dans un mélange de 20 ml d'éther diéthylique et de 5 ml d'acétonitrile, filtration et séchage.
PF : 169°C (dégradation)
RMN ¹H (CDCl₃) δ : 8,20 (d, 1H), 7,65 (d, 1H), 7,10 (d, 1H), 7,0 (s, 1H), 6,65 (d, 1H), 4,65 (sl, 1H), 3,7 (m, 2H), 3,4 (m, 2H), 3,2 (m, 2H), 3,05-2,8 (d, 3H et m, 4H), 2,60 (s, 3H) ppm.

### Exemple 7 (composé n° 79) : Chlorhydrate (3:1) de 9-(2-méthyl-3-pyridin-4-yl-imidazo[1,2-b]pyridazin-6-yl)-1-oxa-4,9-diaza-spiro[5.5]undécane

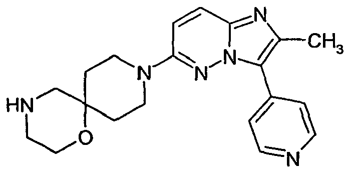

### Etape 7.1. : 6-Chloro-3-(pyridin-4-yl)-imidazo[1,2-b]pyridazine

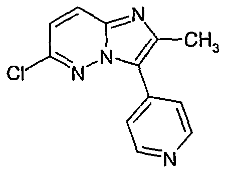

A une solution de 8,90 g (53,1 mmoles) de 6-chloro-2-méthyl-imidazo[1,2-*b*]pyridazine dans 270 ml de diméthylformamide, on additionne 13,1 g (63,7 mmoles) 4-iodopyridine, 7,34 g (53,1 mmoles) de carbonate de potassium. On fait ensuite barboter de l'argon et l'on additionne 0,60 g (2,7 mmoles) d'acétate de Palladium (II). Le mélange est chauffé à 135°C pendant 3 heures puis il est versé dans de l'eau et le produit est extrait avec de l'acétate d'éthyle. La phase organique est séchée sur sulfate de sodium et le solvant évaporé sous pression réduite. Le solide jaune obtenu est organisé sous agitation pendant 30 minutes dans de l'acétonitrile et l'on isole 6 g d'un solide beige foncé. Les eaux-mères sont concentrées sous pression réduite et purifiées sur 110 g de gel de silice en éluant avec un mélange de dichlorométhane, de méthanol et d'ammoniaque (98/2/0,2) pour donner 1,5 g de produit supplémentaire de pureté équivalente au premier jet.
Rendement global : 58%
PF : 180°C
RMN ¹H (CDCl₃) δ : 8,60 (d, 2H), 7,80 (d, 1H), 8,55 (d, 2H), 7,95 (d, 1H), 2,50 (s, 3H)

### Etape 7.2.: 9-(2-Méthyl-3-pyridin-4-yl-imidazo[1,2-b]pyridazin-6-yl)-1-oxa-4,9-diaza-spiro[5.5]undécane-4-carboxylate de tert-butyle

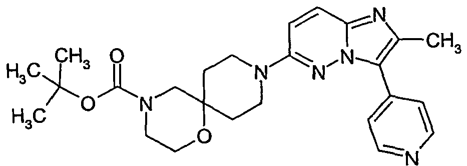

Dans un réacteur, une solution de 0,50 g (2,0 mmoles) de 6-chloro-3-(pyridin-4-yl)-imidazo[1,2-*b*]pyridazine obtenu à l'étape 7.1., de 0,90 g (3,1 mmoles) de 1-oxa-4,9-diaza-spiro[5.5]undécane-4-carboxylate de *tert*-butyle et de 0,84 ml (5,1 mmoles) de diisopropyl-éthyl-amine dans 5 ml de pentanol est chauffée à 150°C pendant 24 heures. Après refroidissement, le pentanol est éliminé par évaporation sous pression réduite, et le résidu est repris avec une solution aqueuse de bicarbonate de sodium. Le produit est extrait avec du dichlorométhane. La phase organique est séchée sur sulfate de sodium, concentrée sous pression réduite et le résidu est chromatographié en éluant avec un mélange de dichlorométhane de méthanol et d'ammoniaque (97/3/0,3) pour conduire à 0,17 g d'huile orangée utilisée telle que dans la suite de la synthèse.

### Etape 7.3. Chlorhydrate (3:1) de 9-(2-méthyl-3-pyridin-4-yl-imidazo[1,2-b]pyridazin-6-yl)-1-oxa-4,9-diaza-spiro[5.5]undécane

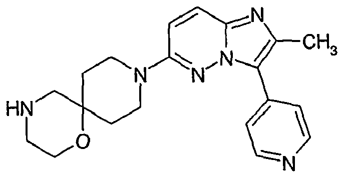

A une solution de 0,17 g (0,37 mmoles) de 9-(2-méthyl-3-pyridin-4-yl-imidazo[1,2-*b*]pyridazin-6-yl)-1-oxa-4,9-diaza-spiro[5.5]undécane-4-carboxylate de *tert*-butyle obtenue à l'étape 7.2. dans 5 ml de dichlorométhane, on additionne 0,55 ml d'acide trifluoroacétique et la solution est agitée une heure à température ambiante.

L'acide est ensuite neutralisé par addition d'une solution aqueuse de bicarbonate de sodium et la phase organique est séparée et séchée sur sulfate de sodium. Après concentration sous pression réduite, le résidu est chromatographié en éluant avec un mélange de dichlorométhane de méthanol et d'ammoniaque (90/10/1) pour conduire à une huile jaune. L'huile est reprise dans de l'acétone et le produit est transformé en son trichlorhydrate par addition d'une solution aqueuse 5-6 N d'acide chlorhydrique dans l'isopropanol. Les solvants sont évaporés sous pression réduite et le résidu solide est trituré dans de l'éthanol pour conduire à 180 mg de poudre beige.
PF >255°C ; M+H : 365
RMN ¹H (DMSO d₆) δ : 9,7 (sl, 2H), 8,90 (d, 2H), 8,35 (d, 2H), 8,10 (d, 1H), 7,65 (d, 1H), 5,3 (sl), 3,9 (m, 4H), 3,3 (m, 2H), 3,0 (m, 4H), 2,65 (m, 3H), 2,1 (m, 2H), 1,7 (m, 2H)

### Exemple 8 (composé n° 70) : 2-Méthyl-6-(5-méthyl-hexahydro-pyrrolo[3,4-c]pyrrol-2(1H)-yl)-3-pyridin-4-yl-imidazo[1,2-b]pyridazine

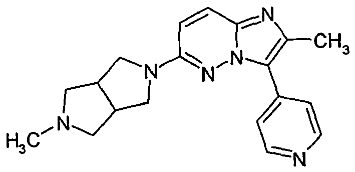

Dans un réacteur, une solution de 0,40 g (1,6 mmoles) de 6-chloro-3-(pyridin-4-yl)-imidazo[1,2-*b*]pyridazine obtenu à l'étape 7.1., de 0,41 g (3,3 mmoles) de 2-méthyl-hexahydro-pyrrolo[3,4-c]pyrrole et de 0,27 ml (1,6 mmoles) de diisopropyl-éthyl-amine dans 5 ml de pentanol est chauffée à 150°C pendant 4 jours. Après refroidissement, le milieu est traité avec une solution aqueuse d'acide chlorhydrique 1N. La phase aqueuse obtenue est lavée avec de l'éther diéthylique puis basifiée au moyen d'ammoniaque aqueux dilué.

Le produit est extrait avec du dichlorométhane, la phase organique est séchée sur sulfate de sodium et concentrée sous pression réduite.

L'huile brune isolée est chromatographiée sur colonne de gel de silice en éluant avec un mélange de dichlorométhane, de méthanol et d'ammoniaque (95/5/0,5) pour conduire à 0,148 g de poudre blanche après cristallisation dans l'éther di-éthylique et séchage.
PF : 155-159°C
RMN ¹H (DMSO d₆) δ : 8,70 (d, 2H), 7,85 (d, 2H), 7,70 (d, 1H), 6,70 (d, 1H), 3,80 (m, 2H), 3,40 (m, 2H), 3,05 (m, 2H), 2,70 (m, 2H), 2,65 (s, 3H), 2,60 (m, 2H), 2,40 (s, 3H).

### Exemple 9 (composé n° 71) : 2,7-Diméthyl-6-(5-méthyl-hexahydro-pyrrolo[3,4-c]pyrrol-2(1H)-yl)-3-pyridin-4-yl-imidazo[1,2-b]pyridazine

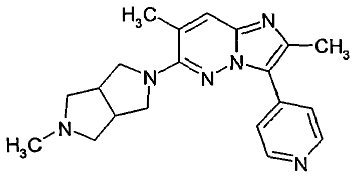

### Etape 9.1 6-chloro-4-méthyl-pyridazin-3-ylamine et 6-chloro-5-méthyl-pyridazin-3-ylamine

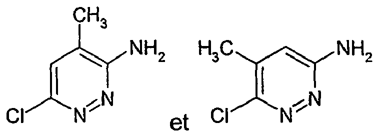

Un mélange de 50,0 g (307 mmoles) de 3,6-dichloro-4-méthylpyridazine dans 170 ml d'ammoniaque (30%) est chauffé à 120°C pendant 16 h dans un réacteur en acier à la pression interne de 10 bars.

Le réacteur est refroidit et le mélange réactionnel est versé dans 200 ml d'eau. Le solide formé est isolé par filtration et séché sous vide, pour donner 38,5 g d'un mélange contenant environ 45% de 6-chloro-4-méthyl-pyridazin-3-ylamine (CAS 64068-00-4) et 55% de 6-chloro-5-méthyl-pyridazin-3-ylamine (CAS 66346-87-0).

RMN ¹H (CDCl₃) δ : 7,20 et 6,75 (2s, 1H); (d, 0,55H) ; 4,9 (si, 2H) ; 2,40 et 2,25 (2s, 3H) ppm.

### Etape 9.2 6-chloro-2,8-diméthyl-imidazo[1,2-b]pyridazine et 6-chloro-2,7-diméthyl-imidazo[1,2-b]pyridazine

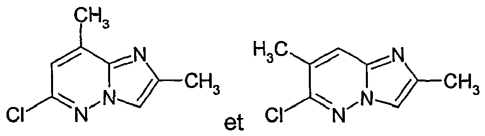

Le mélange de 16,2 g (174 mmoles) de 2-bromoacétone (CAS 78-95-5) avec 19,3 g (134 mmoles) du mélange de 6-chloro-4-méthyl-pyridazin-3-ylamine et de 6-chloro-5-méthyl-pyridazin-3-ylamine obtenu à l'étape 9.1 dans 200 ml de n-butanol est chauffé à 120°C pendant 18 heures. Après refroidissement, le solvant est éliminé par évaporation sous pression réduite et le solide est trituré dans 170 ml d'acétone. Après glaçage, le solide est séparé par filtration. La poudre beige foncée est reprise dans du chloroforme et basifiée par addition d'une solution d'ammoniaque. Le produit est extrait avec du chloroforme, la phase organique est séchée sur sulfate de sodium et le solvant est évaporé sous pression réduite pour donner 14 g d'un solide brun. La séparation des deux isomères est réalisée par chromatographie sur colonne d'alumine (800 g). Le produit est déposé sur la colonne en solution dans un mélange de toluène et de dichlorométhane, puis les isomères sont séparés en éluant par un gradient de cyclohexane dans le dichlorométhane (50% à 0%). On obtient ainsi successivement 5,2 g de 6-chloro-2,8-diméthyl-imidazo[1,2-*b*]pyridazine et 6,0 g de 6-chloro-2,7-diméthyl-imidazo[1,2-*b*]pyridazine sous forme de poudres blanches après trituration dans 50 ml d'éther di-*iso*propylique, filtration et séchage.
6-chloro-2,8-diméthyl-imidazo[1,2-*b*]pyridazine :
PF : 117-119°C
RMN ¹H (CDCl₃) δ : 8,05 (s, 1H), 2,55 (s, 3H), 2,40 (s, 3H) ppm.
6-chloro-2,7-diméthyl-imidazo[1,2-*b*]pyridazine :
PF : 185-188°C
RMN ¹H (CDCl₃) δ : 8,00 (s et s, 2H), 2,40 (s et s, 6H) ppm.

### Etape 9.3. 6-Chloro-2,7-diméthyl-3-iodo-imidazo[1,2-b]pyridazine

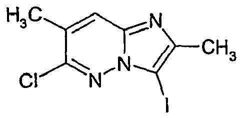

A une solution de 6,00 g (33,0 mmoles) de 6-chloro-2,7-diméthyl-imidazo[1,2-*b*]pyridazine dans 100 ml de chloroforme à température ambiante, on ajoute rapidement 82,6 ml (82,6 mmoles) d'une solution 1 M de monochlorure d'iode dans le dichlorométhane. La réaction est ensuite laissée à température ambiante pendant une heure puis on additionne une solution aqueuse de bicarbonate de sodium et une solution aqueuse de thiosulfate de sodium 5% jusqu'à décoloration. Le produit est extrait avec du dichlorométhane, la phase organique est séchée sur sulfate de sodium et le solvant évaporé sous pression réduite.

Le résidu solide jaunâtre est trituré dans 50 ml d'éther di-isopropylique puis isolé par filtration pour donner 9,7 g d'une poudre jaune après séchage.
PF : 219-220°C
RMN ¹H (CDCl₃) δ : 7,70 (s, 1H), 2,60 (s, 3H), 2,55 (s, 3H) ppm.

### Etape 9.4. 6-Chloro-2,7-diméthyl-3-(pyridin-4-yl)-imidazo[1,2-b]pyridazine

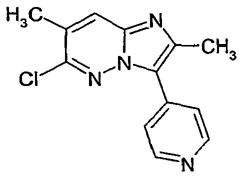

A une solution de 4,82 g (15,7 mmoles) de 6-chloro-2,7-diméthyl-3-iodo-imidazo[1,2-*b*]pyridazine, de 2,72 g (18,8 mmoles) d'acide pyridin-4-yl-boronique et de 15,3 g (47 mmoles) de carbonate de césium dans 220 ml d'un mélange de tétrahydrofurane et d'eau (9:1) sous argon, on additionne 1,15 g (1,41 mmoles) de complexe de 1,1'-bis(diphénylphosphino)ferrocènedichloropalladium (II) et de dichlorométhane (PdCl2(dppf).CH₂Cl₂). Après 18 heures de chauffage à reflux, le mélange est versé dans une solution aqueuse d'acide chlorhydrique 1 N glacée, la phase aqueuse est lavée avec de l'acétate d'éthyle puis basifiée par addition de bicarbonate de sodium. Le produit est ensuite extrait avec du dichlorométhane. La phase organique est séchée sur sulfate de sodium et le solvant évaporé sous pression réduite. Le résidu solide est purifié sur 120 g de gel de silice en éluant avec un mélange de dichlorométhane, de méthanol et d'ammoniaque (96/4/0,4) pour donner 3,05 g d'un solide blanc après trituration dans l'éther di-isopropylique filtration et séchage.
PF : 178-181 °C
RMN ¹H (DMSO d₆)) δ : 8,75 (d, 2H), 8,17 (s, 1H), 7,80 (d, 2H), 2,60 (s, 3H), 2,45 (s, 3H) ppm.

### Etape 9.5. 2,7-Diméthyl-6-(5-méthyl-hexahydro-pyrrolo[3,4-c]pyrrol-2(1H)-yl)-3-pyridin-4-yl-imidazo[1,2-b]pyridazine

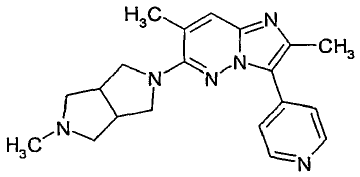

Dans un réacteur, une solution de 0,357 g (1,45 mmole) de 6-chloro-2,7-diméthyl-3-(pyridin-4-yl)-imidazo[1,2-*b*]pyridazine, de 0,256 g (2,03 mmoles) de 2-méthyl-hexahydro-pyrrolo[3,4-c]pyrrole et de 0,20 ml (1,5 mmoles) de triéthylamine dans 4 ml de pentanol est chauffée à 150°C pendant 3 jours. Après refroidissement, le milieu est traité avec une solution aqueuse d'acide chlorhydrique 1N. La phase aqueuse obtenue est lavée avec de l'éther diéthylique puis basifiée au moyen d'ammoniaque aqueux dilué.

Le produit est extrait avec du dichlorométhane, la phase organique est séchée sur sulfate de sodium et concentrée sous pression réduite.

L'huile brune isolée est chromatographiée sur colonne de gel de silice en éluant avec un mélange de dichlorométhane, de méthanol et d'ammoniaque (93/7/0,7) pour conduire à 0,230 g de poudre blanche après cristallisation dans l'acétonurie et séchage.
PF : 139-142°C
RMN ¹H (DMSO d₆) δ : 8,70 (d, 2H), 7,85 (d, 2H), 7,75 (s, 1H), 3,80 (m, 2H), 3,45 (dd, 2H), 3,25 (dd, 2H), 2,85 (m, 2H), 2,65 (dd, 2H), 2,55 (s, 3H), 2,45 (s, 3H), 2,35 (dd, 3H), 2,25 (s, 3H).

Le tableau 1 qui suit illustre les structures chimiques et les propriétés physiques de quelques composés selon l'invention.

Dans ce tableau :
- la colonne "PF°C" renseigne les points de fusion des produits en degrés Celsius. "N.D" signifie que le point de fusion est non déterminé,
- dans la colonne "sel", « HCl » représente un composé sous forme de chlorhydrate et le rapport entre parenthèses est le rapport (acide: base), « HBr » représente un composé sous forme bromhydrate et le rapport entre parenthèse est le rapport (acide : base), le signe « - » signifie que le composé se présente sous forme base,
- la colonne [α]_{D} renseigne le résultat d'analyse du pouvoir rotatoire des composés du tableau à la longueur d'onde de 589 nM ; le solvant indiqué entre parenthèses correspond au solvant employé pour réaliser la mesure du pouvoir rotatoire en degrés et la lettre « C » indique la concentration du solvant en g/100 ml. « N.A. » signifie que la mesure du pouvoir rotatoire n'est pas applicable,
- la colonne "LC-MS ou (MS)" renseigne le résultat d'analyse des produits par LC-MS (liquid chromatography coupled to Mass Spectroscopy) réalisée sur un appareil Agilent LC-MSD Trap en mode ESI positif ou par MS (Mass Spectroscopy) sur un appareil Autospec M (EBE) en utilisant la technique DCI-NH3,
- décomp. signifie décomposition,
- dégrad. signifie dégradation,
- « F » signifie fluoro,
- « Cl » signifie chloro,
- « CH₃- » signifie méthyle,
- « C₂H₅- » signifie éthyle,
- « CH₃O- » signifie Méthoxy,
- « CH₃OH » signifie méthanol ;
- « CH₃-O-CH₂- » signifie méthoxy-méthyle,
- « c-Propyl- » signifie cyclopropyle,
- « c-Butyl- » signifie cyclobutyle,
- « c-Propyl-CH₂- » signifie cyclopropyl-méthyle,
- « NH₂- » signifie amino,
- « CH₃NH- » signifie méthyl-amino,
- « CH₂Cl₂ » signifie dichlorométhane,

**TABLEAU1**

| **No** | **NALB** | **R₇** | **R₈** | **R₂** | **R₃** | **Sel** | **[α]_{D} (°) (Solvant ; C en g/100ml)** | **PF °C** | **M+H** |
|---|---|---|---|---|---|---|---|---|---|
| 1 | Pipérazin-1-yl | H | H | CH₃- | H | - | N.A. | 166-168 | 295 |
| 2 | Pipérazin-1-yl | H | H | CH₃- | H | HCl (3:1) | N.A. | 319-321 | 295 |
| 3 | Pipérazin-1-yl | H | H | CH₃- | F | - | N.A. | 139-141 | 313 |
| 4 | Pipérazin-1-yl | CH₃- | CH₃- | CH₃- | H | HCl (3:1) | N.A. | N.D. | 323 |
| 5 | Pipérazin-1-yl | H | H | CH₃-O-CH₂- | H | | N.A. | N.D. | 325 |
| 6 | Pipérazin-1-yl | H | H | C₂H₅- | H | - | N.A. | N.D. | 309 |
| 7 | Pipérazin-1-yl | H | H | C₂H₅- | H | HCl (3:1) | N.A. | N.D. | 309 |
| 8 | Pipérazin-1-yl | H | H | C₂H₅- | CH₃- | - | N.A. | N.D. | 323 |
| 9 | Pipérazin-1-yl | H | H | Isopropyl- | H | - | N.A. | 199-201 | 323 |
| 10 | Pipérazin-1-yl | H | H | Isopropyl- | CH₃- | - | N.A. | N.D. | 337 |
| 11 | Pipérazin-1-yl | H | H | c-Propyl- | H | - | N.A. | 169-171 | 321 |
| 12 | Pipérazin-1-yl | H | H | c-Propyl- | CH₃- | - | N.A. | N.D. | 335 |
| 13 | Pipérazin-1-yl | H | H | c-Propyl- | NH₂- | - | N.A. | N.D. | 336 |
| 14 | Pipérazin-1-yl | H | H | c-Propyl- | CH₃O- | - | N.A. | N.D. | 351 |
| 15 | Pipérazin-1-yl | H | H | c-Propyl- | Cl | - | N.A. | N.D. | 355 |
| 16 | Pipérazin-1-yl | H | H | Isobutyl- | H | - | N.A. | 92-119 | 337 |
| 17 | Pipérazin-1-yl | H | H | Isobutyl- | H | HCl (3:1) | N.A. | N.D. | 337 |
| 18 | Pipérazin-1-yl | H | H | c-Propyl-CH₂- | H | - | N.A. | N.D. | 335 |
| 19 | Pipérazin-1-yl | H | H | c-Butyl- | H | - | N.A. | 189-191 | 335 |
| 20 | (*R,S*)-3-Méthylpipérazin-1-yl | H | H | CH₃- | H | - | N.A. | 133-135 | 309 |
| 21 | (*R,S*)-3-Méthylpipérazin-1-yl | H | H | CH₃- | CH₃- | - | N.A. | 145-147 | 323 |
| 22 | (*R,S*)-3-Méthylpipérazin-1-yl | H | H | c-Propyl- | CH₃- | - | N.A. | N.D. | 349 |
| 23 | (*R,S*)-3-Méthylpipérazin-1-yl | H | H | c-Propyl- | NH₂- | - | N.A. | N.D. | 350 |
| 24 | (*R,S*)-3-Méthylpipérazin-1-yl | H | H | c-Propyl- | CH₃O- | - | N.A. | N.D. | 365 |
| 25 | (*R,S*)-3-Méthylpipérazin-1-yl | H | H | c-Propyl- | Cl | - | N.A. | N.D. | 369 |
| 26 | (*R*)-3-Méthylpipérazin-1-yl | H | H | CH₃- | H | - | -18,3 (CH₃OH ; C = 1,012) | 146-148 150-152 | 309 |
| 27 | (*R*)-3-Méthylpipérazin-1-yl | H | H | CH₃- | CH₃- | - | +3,3 (CH₂Cl₂ ; C = 1,032;) | 157-159 | 323 |
| 28 | (*R*)-3-Méthylpipérazin-1-yl | H | H | CH₃- | NH₂- | - | -11,6 (CH₃OH ; C = 1,000 ;) | 172-177 174-177 | 324 |
| 29 | (*R*)-3-Méthylpipérazin-1-yl | H | H | CH₃- | CH₃NH- | - | -6,6 (CH₃OH C = 0.966 ;) | 65 | 338 |
| 30 | (*R*)-3-Méthylpipérazin-1-yl | H | H | CH₃- | CH₃O- | - | -17,1 (CH₂Cl₂ ; C = 1,000;) | 104-106 | 339 |
| 31 | (*R*)-3-Méthylpipérazin-1-yl | H | H | C₂H₅- | H | - | -4,3 (CHCl₃ ; C = 0,235 ;) | N.D. | 323 |
| 32 | (*R*)-3-Méthylpipérazin-1-yl | H | H | C₂H₅- | F | - | -13,9 (CHCl₃ ; C = 0,245 ;) | N.D. | 341 |
| 33 | (*R*)-3-Méthylpipérazin-1-yl | H | H | c-Propyl-CH₂- | H | - | -20,2 (CHCl₃ ; C = 0,104;) | N.D. | 349 |
| 34 | (*R*)-3-Méthylpipérazin-1-yl | H | H | c-Propyl-CH₂- | CH₃- | - | -9,6 (CHCl₃ ; C=0,114;) | N.D. | 363 |
| 35 | (*S*)-3-Méthylpipérazin-1-yl | H | H | CH₃- | H | - | +17,3 (CH₃OH ; C = 0,788 ;) | 146-148 | 309 |
| 36 | (*S*)-3-Méthylpipérazin-1-yl | H | H | CH₃- | CH₃O- | - | + 16,3 (CH₂Cl₂ ; C = 1,000 ;) | 106-108 | 339 |
| 37 | (*S*)-3-Méthylpipérazin-1-yl | H | H | c-Propyl- | H | - | -12,3 (CHCl₃ ; C = 0,77;) | N.D. | 335 |
| 38 | (*S*)-3-Méthylpipérazin-1-yl | H | H | CH₃- | NH₂- | - | +11,7 (CH₂Cl₂ ; C = 1,000;) | 172-177 177-178 | 324 |
| 39 | 4-Méthylpipérazin-1-yl | H | H | CH₃- | H | - | N.A. | 150-152 154-156 | 309 |
| 40 | 4-Méthylpipérazin-1-yl | H | H | CF₃- | H | - | N.A. | 182-184 | 363 |
| 41 | (*R,S*)-3-Hydroxyméthylpipérazin-1-yl | H | H | CH₃- | H | - | N.A. | 237-240 | 325 |
| 42 | 4-Éthylpipérazin-1-yl | H | H | CH₃- | H | HCl (3:1) | N.A. | 305-307 | 323 |
| 43 | 4-Éthylpipérazin-1-yl | H | H | CH₃- | CH₃- | - | N.A. | 144-146 | 337 |
| 44 | 4-(iso-Propyl)pipérazin-1-yl | H | H | CH₃- | H | HCl (3:1) | N.A. | 304-306 | 338 |
| 45 | 4-(iso-Propyl)pipérazin-1-yl | H | H | CH₃- | CH₃- | - | N.A. | 97-99 | 351 |
| 46 | 4-(iso-Propyl)pipérazin-1-yl | H | H | CH₃- | NH₂- | HCl (3:1) | N.A. | >300 | 352 |
| 47 | 4-(cyclo-Butyl)pipérazin-1-yl | H | H | CH₃- | H | - | N.A. | 122-132 | 349 |
| 48 | 4-(cyclo-Butyl)pipérazin-1-yl | H | H | CH₃- | NH₂- | - | N.A. | 132-144 | 364 |
| 49 | 3,3-Diméthylpipérazin-1-yl | H | H | CH₃- | CH₃- | - | N.A. | 110-112 | 337 |
| 50 | 3,3-Diméthylpipérazin-1-yl | H | H | CH₃- | CH₃NH- | - | N.A. | 151-156 | 352 |
| 51 | 3,3-Diméthylpipérazin-1-yl | H | H | c-Propyl- | CH₃- | - | N.A. | N.D. | 363 |
| 52 | 3,3-Diméthylpipérazin-1-yl | H | H | c-Propyl- | NH₂- | - | N.A. | N.D. | 364 |
| 53 | 3,3-Diméthylpipérazin-1-yl | H | H | c-Propyl- | F | - | N.A. | N.D. | 367 |
| 54 | cis-3,5-Diméthylpipérazin-1-yl | H | H | CH₃- | H | HCl (3:1) | N.A. | 343-345 | 323 |
| 55 | *cis*-3,5-Diméthyl pi pérazin-1-yl | H | H | CH₃- | CH₃- | - | N.A. | 127-129 | 337 |
| 56 | *cis*-3,5-Diméthylpipérazin-1-yl | H | H | CH₃- | NH₂- | - | N.A. | 213-215 | 338 |
| 57 | *cis*-3,5-Diméthylpipérazin-1-yl | H | H | CH₃- | CH₃NH- | - | N.A. | 111-126 | 352 |
| 58 | *cis*-3,5-Diméthylpipérazin-1-yl | H | H | c-Propyl- | H | - | N.A. | N.D. | 349 |
| 59 | *cis*-3,5-Diméthylpipérazin-1-yl | H | H | c-Propyl- | NH₂- | - | N.A. | N.D. | 364 |
| 60 | (*S*)-Hexahydro-pyrrolo[1,2-a]pyrazin-2-yl | H | H | CH₃- | H | - | +6 (CH₂Cl₂ ; C = 1,000 ;) | 154-156 | 335 |
| | 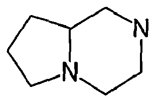 | | | | | | | | |
| 61 | (*S*)-Hexahydro-pyrrolo[1,2-*a*]pyrazin-2-yl | H | H | CH₃- | CH₃- | - | -1,4 (CH₂Cl₂ ; C = 1,014) | 153-155 | 349 |
| 62 | (1*S*,4*S*)-2,5-diaza-bicyclo[2.2.1]hept-2-yl | H | H | CH₃- | H | - | N.D. | 120°C décomp. | 307 |
| | 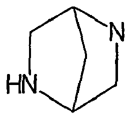 | | | | | | | | |
| 63 | (*R,S*)-2,5-diaza-bicyclo[2.2.1]hept-2-yl | H | H | CH₃- | CH₃- | HBr (1:1) | N.A. | >300 | 321 |
| 64 | (*R,S*)-1,4-Diaza-bicyclo[3.2.2]non-4-yl | H | H | c-Propyl- | CH₃- | - | N.A. | N.D. | 375 |
| | 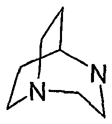 | | | | | | | | |
| 65 | (*R,S*)-Hexahydro-pyrrolo[3,4-*b*]pyrrol-5(1*H*)-yl | H | H | CH₃- | CH₃- | - | N.A. | 131-133 | 335 |
| 66 | Hexahydro-pyrrolo[3,4-*c*]pyrrol-2(1*H*)-yl | H | H | CH₃- | H | - | N.A. | 153-155 | 321 |
| | 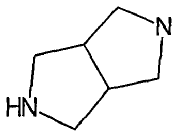 | | | | | | | | |
| 67 | Hexahydro-pyrrolo[3,4-*c*]pyrrol-2(1*H*)-yl | H | H | CH₃- | CH₃- | HCl (3:1) | N.A. | 214-221 | 335 |
| 68 | Hexahydro-pyrrolo[3,4-*c*]pyrrol-2(1*H*)-yl | H | H | CH₃- | NH₂- | - | N.A. | 155 dégrad. | 336 |
| 69 | Hexahydro-pyrrolo[3,4-*c*]pyrrol-2(1*H*)-yl | H | H | CH₃- | CH₃NH- | - | N.A. | 169 dégrad. | 350 |
| 70 | 5-Méthyl-hexahydro-pyrrolo[3,4-*c*]pyrrol-2(1*H*)-yl | H | H | CH₃- | H | - | N.A. | 155-159 | 335 |
| 71 | 5-Méthyl-hexahydro-pyrrolo[3,4-*c*]pyrrol-2(1*H*)-yl | CH₃- | H | CH₃- | H | - | N.A. | 139-142 | 349 |
| 72 | 5-Isopropyl-hexahydro-pyrrolo[3,4-*c*]pyrrol-2(1*H*)-yl | H | H | CH₃- | H | - | CH₃- | 172-175 | 363 |
| 73 | 5-Isopropyl-hexahydro-pyrrolo[3,4-*c*]pyrrol-2(1*H*)-yl | H | H | CH₃- | NH₂- | - | N.A. | 215-218 | 378 |
| 74 | Hexahydro-pyrrolo[3,4-*c*]pyrrol-2(1*H*)-yl | H | H | CH₃- | CH₃O- | - | N.A. | 95 | 351 |
| 75 | (*R,S*)-Octahydro-pyrrolo[3,4-*b*]pyridin-6-yl | H | H | CH₃- | H | - | N.A. | 130-135 | 335 |
| | 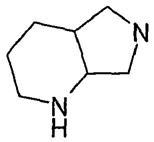 | | | | | | | | |
| 76 | 2,7-Diaza-spiro[3.5]non-7-yl | H | H | CH₃- | CH₃- | - | N.A. | 117-120 | 349 |
| | 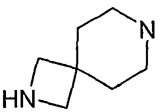 | | | | | | | | |
| 77 | (*R,S*)-2,7-Diaza-spiro[4.5]déc-2-yl | H | H | CH₃- | H | - | N.A. | 134-136 | 349 |
| | 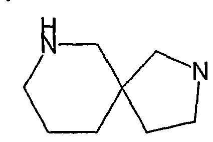 | | | | | | | | |
| 78 | 2,9-Diaza-spiro[5.5]undéc-9-yl | H | H | CH₃- | H | HCl (3:1) | N.A. | >260 Dégrad. | 363 |
| | 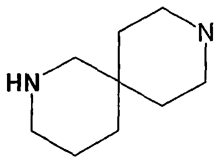 | | | | | | | | |
| 79 | 2,9-Diaza-spiro[5.5]undéc-9-yl | H | H | CH₃- | CH₃- | HCl (3:1) | N.A. | >295 | 377 |
| | 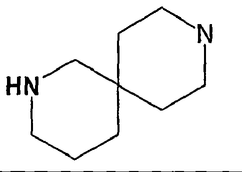 | | | | | | | | |
| 80 | 1-Oxa-4,9-diaza-spiro[5.5]undéc-9-yl | H | H | CH₃- | H | HCl (3:1) | N.A. | >255 | 365 |
| | 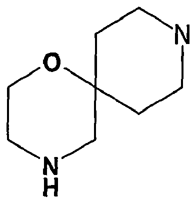 | | | | | | | | |
| 81 | 1-Oxa-4,9-diaza-spiro[5.5]undéc-9-yl | H | H | CH₃- | NH₂- | HCl (3:1) | N.A. | >300 | 380 |
| | 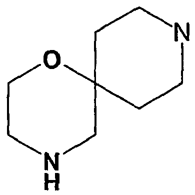 | | | | | | | | |
| 82 | 4-(Pyrrolidin-1-yl)-pipéridin-1-yl | H | H | CH₃- | H | - | N.A. | 150-153 143-145 | 363 |
| 83 | 4-(Pyrrolidin-1-yl)-pipéridin-1-yl | H | H | CH₃- | CH₃- | - | N.A. | 124-126 | 377 |
| 84 | 4-(Pyrrolidin-1-yl)-pipéridin-1-yl | H | H | CH₃- | NH₂- | - | N.A. | 129-138 | 378 |
| 85 | (*R,S*)-[1,3']Bipyrrolidinyl-1'-yl- | H | H | CH₃- | H | - | N.A. | 155-157 | 349 |

### Exemples biologiques

La capacité des composés de l'invention à inhiber la phosphorylation de la caséine par les caséine kinase 1 epsilon et delta peut être évaluée selon la procédure décrite dans le document US20050131012.

### Dosage sur Plaque-Filtre-d'ATP-³³P pour le criblage des inhibiteurs de CK1epsilon :

On mesure l'effet des composés pour inhiber la phosphorylation de la caséine par l'enzyme caséine kinase 1 epsilon (CK1 epsilon) en utilisant un dosage de la caséine par filtration d'ATP-³³P in vitro.

La Caséine Kinase 1 epsilon (0,58 mg/ml) est obtenue par des procédés de fermentation et de purification effectués selon des méthodes bien connues de l'homme du métier ou peut également être obtenue auprès d'Invitrogen Corporation^{™} (human CK1 epsilon).

Les composés sont testés à cinq concentrations différentes de manière à générer des CI₅₀, c'est à dire la concentration à laquelle un composé est capable d'inhiber l'activité enzymatique de 50%, ou bien l'inhibition en % à une concentration de 10 micromolaires.

On prépare des plaques Falcon à fond en « U » en plaçant 5 µL de solutions des composés selon l'invention aux concentrations de 10, 1, 0,1, 0,01 ou 0,001 µM dans différents puits. Les solutions des composés selon l'invention à ces différentes concentrations sont préparées par dilution dans un tampon d'essai (Tris 50 mM pH 7,5, MgCl2 10 M, DTT 2 mM et EGTA 1 mM) d'une solution mère dans le DMSO à la concentration de 10 mM. Ensuite, on additionne 5 µL de caséine déphosphorylée à la concentration finale de 0,2 µg/µL, 20 µL de CK1 epsilon à la concentration finale de 3 ng/µL, et 20 µL d'ATP-³³P à la concentration finale de 0,02 µCi/µL mélangée avec de l'ATP froide (10 µM final - environ 2×106 CPM par puits). Le volume total final d'essai par puits est égal à 50 µL.

La plaque d'essai Falcon^{®} à fond en « U » citée ci-dessus est agitée au vortex, puis incubée à la température ambiante pendant 2 heures. Après 2 heures, la réaction est arrêtée par addition d'une solution glacée de 65 µL d'ATP froid (2 mM) préparée dans du tampon d'essai.

On transfère ensuite 100 µL du mélange réactionnel de la plaque Falcon^{®} à fond en U dans des plaques de filtration MAPH Millipore^{®}, préalablement imprégnées avec 25 µL de TCA glacé à 100 %.

Les plaques de filtration MAPH Millipore sont agitées doucement et on les laisse au repos à la température ambiante pendant au moins 30 minutes pour précipiter les protéines.

Après 30 minutes, les plaques de filtration sont séquentiellement lavées et filtrées avec 2x150 µL de TCA à 20%, 2×150 µL de TCA à 10% et 2×150 µL de TCA à 5% (6 lavages au total par plaque/900 µL par puits).

On laisse les plaques sécher pendant une nuit à la température ambiante. Ensuite, on ajoute 40 µL de liquide de scintillation Microscint-20 Packard^{®} par puits et les plaques sont fermées de manière étanche. On mesure alors le rayonnement émis par chaque puits pendant 2 minutes dans un compteur à scintillation Topcount NXT Packard^{®} où les valeurs de CPM /puits sont mesurées.

On détermine l'inhibition en % de la capacité de l'enzyme à phosphoryler le substrat (caséine) pour chaque concentration de composé testé. Ces données d'inhibition exprimées en % sont utilisées pour calculer la valeur de CI50 pour chaque composé comparativement aux contrôles.

Les études cinétiques ont déterminé la valeur de K_{M} pour ATP comme étant de 21 µM dans ce système d'essai.

Dans ces conditions, les composés les plus actifs de l'invention présentent des CI₅₀ (concentration inhibant de 50 % l'activité enzymatique de la Caséine Kinase 1 Epsilon ou Caséine Kinase 1 Delta) comprises entre 1 nM et 500 nM.

Le tableau 2 ci-dessous présente les CI₅₀ d'inhibition de la phosphorylation de la Caséine Kinase 1 Epsilon pour quelques composés selon l'invention.

**Tableau 2**

| Composé N° | CK1 epsilon CI₅₀ (nM) |
|---|---|
| 1 | 57 |
| 53 | 292 |
| 66 | 13 |
| 78 | 78 |

La capacité des composés de l'invention à inhiber la phosphorylation de la caséine par les caséines kinases 1 epsilon et delta peut être évaluée en utilisant un test de fluorescence FRET (« transfert d'énergie entre molécules fluorescentes », de l'anglais « Fluorescence Resonance Energy Transfert ») à partir du kit « Z'Lyte^{™} kinase assay Kit » (référence PV3670 ; Invitrogen Corporation^{™}) selon les instructions du fournisseur.

Les Caséines Kinases 1 utilisées sont obtenues chez Invitrogen Corporation (human CK1 epsilon PV3500 et human CK1 delta PV3665).

Un peptide substrat, marqué à ses deux extrémités par un groupe fluorophore donneur (la coumarine) et un groupe fluorophore accepteur (la fluorescéine) constituant un système FRET est phosphorylé en présence d'ATP par la caséine kinase 1 epsilon ou delta en présence de concentrations croissantes de composés de l'invention.

Le mélange est traité au moyen d'une protéase site spécifique coupant spécifiquement le peptide substrat pour former deux fragments fluorescents présentant un grand ratio d'émission par fluorescence.

La fluorescence observée est donc reliée à la capacité des produits de l'invention à inhiber la phosphorylation du peptide substrat par la caséine kinase 1 epsilon ou de la caséine kinase 1 delta.

Les composés de l'invention sont mis en solution à des concentrations différentes à partir d'une solution mère à 10 mM dans le DMSO diluée dans un tampon contenant 50 mM HEPS, pH 7,5, 1 mMEGTA, 0,01% Brij-35, 10 mM MgCl pour la caséine kinase 1 epsilon et supplémenté avec Trizma Base (50 mM), pH 8,0 et NaN3 (0,01% finaux) pour la caséine kinase 1 delta.

La phosphorylation du peptide substrat SER/THR 11 obtenu chez Invitrogen Corporation^{™} est réalisée à la concentration finale de 2 µM. La concentration en ATP est de 4 fois le K_{M}, celui-ci étant de 2 µM pour la caséine kinase 1 epsilon et de 4 µM pour la caséine kinase 1 delta.

La mesure de la fluorescence émise est mesurée aux longueurs d'onde de 445 et 520 nm (excitation à 400 nm).

Dans ces conditions, les composés les plus actifs de l'invention présentent des CI₅₀ (concentration inhibant de 50 % l'activité enzymatique de la Caséine Kinase 1 Epsilon ou Caséine Kinase 1 Delta) comprises entre 1 nM et 500 nM.

Le tableau 3 ci-dessous présente les CI₅₀ d'inhibition de la phosphorylation de la Caséine Kinase 1 Delta pour quelques composés selon l'invention.

**Tableau 3**

| Composé N° | CK1 delta CI₅₀ (nM) |
|---|---|
| 1 | < 1 |
| 54 | < 1 |

II apparaît donc que les composés selon l'invention ont une activité inhibitrice sur l'enzyme Caséine Kinase 1 epsilon ou Caséine Kinase 1 delta.

### Protocoles expérimentaux de dosage circadien cellulaire

Des cultures de fibroblastes Mper1-luc Rat-1 (P2C4) ont été réalisées en divisant les cultures tous les 3-4 jours (environ 10-20 % de confluence) sur des flacons de culture de tissus en polystyrène dégazés de 150 cm² (Falcon® # 35-5001) et maintenues en milieu de croissance [EMEM (Cellgro #10-010-CV) ; sérum bovin foetal à 10 % (FBS; Gibco #16000-044) ; et 50 I.U./ml de pénicilline-streptomycine (Cellgro #30-001-Cl)] à 37°C et sous CO₂ 5 %.

Des cellules issues de cultures de fibroblastes Rat-1 à 30-50 % de confluence telle que décrite ci-dessus ont été co-transfectées avec des vecteurs contenant le marqueur de sélection pour la résistance à la Zéocine pour une transfection stable et un gène rapporteur de la luciférase dirigé par le promoteur mPer-1. Après 24 à 48 heures, les cultures ont été divisées sur des plaques de 96 puits et maintenues en milieu de croissance additionné de 50-100 µg/ml de Zéocine (Invitrogen^{®} #45-0430) pendant 10-14 jours. Les transfectants stables résistant à la Zéocine ont été évalués pour l'expression du rapporteur en ajoutant au milieu de croissance de la luciférine 100 µM (Promega^{®} #E1603^{®}) et en dosant l'activité de la luciférase sur un compteur à scintillation TopCount^{®} (Packard Modèle #C384V00). Les clones de cellule Rat-1 exprimant aussi bien la résistance à la Zéocine que l'activité de la luciférase dirigée par mPer1 ont été synchronisés par choc au sérum avec du sérum de cheval à 50 % [HS (Gibco^{®} #16050-122)] et l'activité du rapporteur circadien a été évaluée. Le clone P2C4 de fibroblastes Mper1-luc Rat-1 a été sélectionné pour l'essai du composé. Des fibroblastes Mper1-luc Rat-1 (P2C4) à 40-50 % de confluence obtenus selon le protocole décrit précédemment ont été étalés sur des plaques de culture de tissu opaques de 96 puits (Perkin Elmer^{®} #6005680). Les cultures sont maintenues en milieu de croissance additionné de 100 µg/ml de Zéocine (Invitrogen #45-0430) jusqu'à ce qu'elles aient atteint 100 % de confluence (48-72 h). Les cultures ont ensuite été synchronisées avec 100 µL de milieu de synchronisation [EMEM (Cellgro #10-010-CV) ; 100 I.U. /ml de pénicilline-streptomycine (Cellgro #30-001-C1) ; HS à 50% (Gibco #16050-122)] pendant 2 heures à 37°C et sous CO₂ 5%. Après synchronisation, les cultures ont été rincées avec 100 µL d'EMEM (Cellgro #10-010-CV) pendant 10 minutes à température ambiante. Après rinçage, le milieu a été remplacé par 300 µL de milieu indépendant de CO₂ [CO₂I (Gibco #18045-088); L-glutamine 2 mM (Cellgro #25-005-C1) ; 100 U.I./ml de pénicilline-streptomycine (Cellgro #30-001-C1) ; luciférine 100 µM (Promega #E 1603)]. Les composés de l'invention testés pour les effets circadiens ont été ajoutés à du milieu indépendant de CO₂ dans du DMSO à 0,3 % (concentration finale). Les cultures ont été fermées immédiatement de manière étanche avec du film TopSeal-A^{®} (Packard #6005185) et transférées pour la mesure de l'activité de luciférase.

Après synchronisation, les plaques d'essai ont été maintenues à 37°C dans une étuve de culture de tissu (Forma Scientific Modèle #3914). L'activité de luciférase *in vivo* a été estimée en mesurant l'émission relative de lumière sur un compteur à scintillation TopCount (Packard Modèle #C384V00).

L'analyse de périodes a été effectuée soit en déterminant l'intervalle entre les minimums d'émission relative de lumière sur plusieurs jours ou par transformation de Fourier.

Les deux méthodes ont produit une estimation de période pratiquement identique sur une gamme de périodes circadiennes. La puissance est rapportée en CE Delta (t+1h), qui est présentée comme la concentration micromolaire efficace qui a induit un prolongement de la période de 1 heure. Les données ont été analysées par ajustement d'une courbe hyperbolique aux données exprimées en changement de période (ordonnée) en fonction de la concentration du composé à tester (abscisse) dans le logiciel XLfit™ et la CE Delta (t+1h) a été interpolée à partir de cette courbe.

Le tableau 4 ci-dessous présente les CE Delta (t+1h) pour quelques composés selon l'invention.

**Tableau 4**

| Composé N° | CE Delta (t+1 h) (nM) |
|---|---|
| 1 | 20 |
| 53 | 319 |
| 66 | 633 |
| 78 | 515 |

En inhibant les enzymes CK1epsilon et/ou de CK1delta, les composés objets de l'invention modulent la rythmicité circadienne, et peuvent être utiles pour le traitement des désordres liés au rythme circadien.

Les composés selon l'invention peuvent notamment être utilisés pour la préparation d'un médicament destiné à prévenir ou à traiter les désordres du sommeil ; les troubles du rythme circadien, tels que notamment ceux dus au décalage horaire, au travail posté.

Parmi les troubles du sommeil, on distingue notamment les troubles primaires du sommeil tels que la dyssomnie (par exemple l'insomnie primaire), la parasomnie, l'hypersomnie (par exemple la somnolence excessive), la narcolepsie, les troubles du sommeil liés à l'apnée du sommeil, les troubles du sommeil liés au rythme circadien et les dyssomnies non spécifiées par ailleurs, les troubles du sommeil associés à des troubles médicaux/psychiatriques.

Les composés objets de l'invention provoquent également un déplacement de la phase circadienne et une telle propriété peut être utile dans le cadre d'une monothérapie ou une thérapie combinée potentielle cliniquement efficace pour les troubles de l'humeur.

Parmi les troubles de l'humeur, on distingue notamment les troubles dépressifs (dépression unipolaire), les troubles bipolaires, les troubles de l'humeur dus à une affection médicale générale ainsi que les troubles de l'humeur induits par des substances pharmacologiques. Parmi les troubles bipolaires, on distingue notamment les troubles bipolaires I et troubles bipolaires II, dont notamment les troubles affectifs saisonniers.

Les composés objets de l'invention modulant la rythmicité circadienne, peuvent être utiles dans le traitement des troubles anxieux et dépressifs dus en particulier à une altération sur la sécrétion de CRF.

Parmi les troubles dépressifs, on distingue notamment les troubles dépressifs majeurs, troubles dysthymiques, les troubles dépressifs non spécifiés par ailleurs.

Les composés objets de l'invention modulant la rythmicité circadienne, peuvent être utiles pour la préparation d'un médicament destiné à traiter les maladies liées à la dépendance à des substances d'abus telles que la cocaïne, la morphine, la nicotine, l'éthanol, le cannabis.

En inhibant la caséine kinase 1 epsilon et/ou la caséine kinase 1 delta, les composés selon l'invention peuvent être utilisés pour la préparation de médicaments, notamment pour la préparation d'un médicament destiné à prévenir ou à traiter des maladies reliées à l'hyperphosphorylation de la protéine tau, notamment la maladie d'Alzheimer.

Ces médicaments trouvent également leur emploi en thérapeutique, notamment dans le traitement ou la prévention des maladies causées ou exacerbées par la prolifération des cellules et en particulier des cellules tumorales.

Comme inhibiteur de la prolifération des cellules tumorales, ces composés sont utiles dans la prévention et le traitement des tumeurs liquides telles que les leucémies, des tumeurs solides à la fois primaires et métastasiques, des carcinomes et cancers, en particulier : cancer du sein ; cancer du poumon ; cancer de l'intestin grêle, cancer du colon et du rectum ; cancer des voies respiratoires, de l'oropharynx et de l'hypopharynx ; cancer de l'oesophage ; cancer du foie, cancer de l'estomac, cancer des canaux biliaires, cancer de la vésicule biliaire, cancer du pancréas ; cancers des voies urinaires y compris rein, urothélium et vessie; cancers du tractus génital féminin y compris cancer de l'utérus, du col de l'utérus, des ovaires, chloriocarcinome et trophoblastome; cancers du tractus génital masculin y compris cancer de la prostate, des vésicules séminales, des testicules, tumeurs des cellules germinales; cancers des glandes endocrines y compris cancer de la thyroïde, de l'hypophyse, des glandes surrénales ; cancers de la peau y compris hémangiomes, mélanomes, sarcomes, incluant le sarcome de Kaposi ; tumeurs du cerveau, des nerfs, des yeux, des méninges, incluant astrocytomes, gliomes, glioblastomes, rétinoblastomes, neurinomes, neuroblastomes, schwannomes, méningiomes ; tumeurs malignes hématopoïétiques ; leucémies, (Acute Lymphocytic Leukemia (ALL), Acute Myeloid Leukemia (AML), Chronic Myeloid Leukemia (CML), Chronic lymphocytic leukemia (CLL)) chloromes, plasmocytomes, leucémies des cellules T ou B, lymphomes non hodgkiniens ou hodgkiniens, myélomes, hémopathies malignes diverses.

Les composés selon l'invention peuvent donc être utilisés pour la préparation de médicaments, en particulier de médicaments inhibiteurs de la caséine kinase 1 epsilon et/ou de la caséine kinase 1 delta.

Ainsi, selon un autre de ses aspects, l'invention a pour objet des médicaments qui comprennent un composé de formule (I), ou un sel d'addition de ce dernier à un acide pharmaceutiquement acceptable ou encore un hydrate ou un solvate du composé de formule (I).

Selon un autre de ses aspects, la présente invention concerne des compositions pharmaceutiques comprenant, en tant que principe actif, un composé selon l'invention. Ces compositions pharmaceutiques contiennent une dose efficace d'au moins un composé selon l'invention ou un sel pharmaceutiquement acceptable, un hydrate ou solvate dudit composé, ainsi qu'au moins un excipient pharmaceutiquement acceptable.

Lesdits excipients sont choisis selon la forme pharmaceutique et le mode d'administration souhaité, parmi les excipients habituels qui sont connus de l'Homme du métier.

Dans les compositions pharmaceutiques de la présente invention pour l'administration orale, sublinguale, sous-cutanée, intramusculaire, intraveineuse, topique, locale, intra trachéale, intra nasale, transdermique ou rectale, le principe actif de formule (I) ci-dessus, ou son sel, solvate ou hydrate éventuel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intra trachéale, intraoculaire, intra nasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscaramellose sodique | 6,0 mg |
| Amidon de maïs | 15,0 mg |
| Hydroxypropyl-méthylcellulose | 2,25 mg |
| Stéarate de magnésium | 3,0 mg |

Par voie orale, la dose de principe actif administrée par jour peut atteindre 0,1 à 20 mg/kg, en une ou plusieurs prises.

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

La présente invention a donc un lien avec une méthode de traitement des pathologies ci-dessus indiquées qui comprend l'administration, à un patient, d'une dose efficace d'un composé selon l'invention, ou un de ses sels pharmaceutiquement acceptables ou hydrates ou solvates.

## Revendications

1. Composé répondant à la formule générale (I) 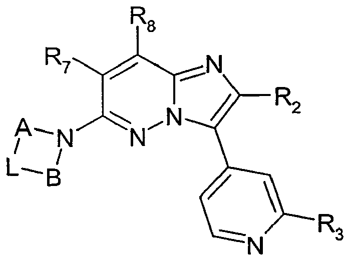 dans laquelle
- R₂ représente un groupe C₁₋₆-alkyle, C₃₋₇-cycloalkyle, C₃₋₇cycloalkyl-C₁₋₄-alkyle, C₁₋₄-alkyloxy-C₁₋₄-alkyle, C₃₋₇-cycloalkyloxy-C₁₋₄-alkyle, C₃₋₇cycloalkyl-C₁₋₄-alkyloxy-C₁₋₄-alkyle, hydroxy-C₁₋₆-alkyle, C₁₋₄-fluoroalkyle ;
- R₃ représente un atome d'hydrogène ou un substituant choisi parmi les atomes d'halogène et les groupes C₁₋₃ alkyle, -NR₄R₅, hydroxyle ou C₁₋₄ alkyloxy ;
- A représente un groupe C₁₋₇-alkylène éventuellement substitué par un ou deux groupes Rₐ ;
- B représente un groupe C₁₋₇-alkylène éventuellement substitué par un groupe R_{b} ;
- L représente soit un atome d'azote éventuellement substitué par un groupe R_{c} ou R_{d}, soit un atome de carbone substitué par un groupe Rₑ₁ et un groupe R_{d} ou deux groupes Rₑ₂ ;
les atomes de carbone de A et de B étant éventuellement substitués par un ou plusieurs groupes R_{f} identiques ou différents l'un de l'autre ;
Rₐ, R_{b} et R_{c} sont définis tels que :
deux groupes Rₐ peuvent former ensemble un groupe C₁₋₆-alkylène ;
Rₐ et R_{b} peuvent former ensemble une liaison ou un groupe C₁₋₆-alkylène;
Rₐ et R_{c} peuvent former ensemble une liaison ou un groupe C₁₋₆-alkylène ;
R_{b} et R_{c} peuvent former ensemble une liaison ou un groupe C₁₋₆-alkylène ;
R_{d} représente un groupe choisi parmi l'atome d'hydrogène et les groupes C₁₋₆-alkyle, C₃₋₇-cycloalkyle, C₃₋₇-cycloalkyl-C₁₋₆-alkyle, C₁₋₆-alkylthio-C-₁₋₆-alkyle, C₁₋₆-alkyloxy-C₁₋₆-alkyle, C₁₋₆-fluoroalkyle, hydroxy-C₁₋₆-alkyle ;
Rₑ₁ représente un groupe -NR₄R₅ ou une monoamine cyclique comportant éventuellement un atome d'oxygène, la monoamine cyclique étant éventuellement substituée par un ou plusieurs substituants choisis parmi l'atome de fluor et les groupes C₁₋₆-alkyle, C₁₋₆-alkyloxy, hydroxyle ;
Deux Rₑ₂ forment avec l'atome de carbone qui les porte une monoamine cyclique comportant éventuellement un atome d'oxygène, cette monoamine cyclique étant éventuellement substituée par un ou plusieurs groupes R_{f} identiques ou différents l'un de l'autre ;
R_{f} représente un groupe C₁₋₆-alkyle, C₃₋₇-cycloalkyle, C₃₋₇-cycloalkyl-C₁₋₆-alkyle, C₁₋₆-alkyloxy-C₁₋₆-alkyle, C₃₋₇-cycloalkyloxy-C₁₋₄-alkyle, C₃₋₇-cycloalkyl-C₁₋₄-alkyloxy-C₁-₄-alkyle, hydroxy-C₁₋₆-alkyle ou C₁₋₆-fluoroalkyle ;
R₄ et R₅ représentent, indépendamment l'un de l'autre, un atome d'hydrogène ou un groupe C₁₋₄ alkyle, C₃₋₇-cycloalkyle, C₃₋₇-cycloalkyle-C₁₋₆-alkyle ;
- R₇ et R₈ représentent, indépendamment l'un de l'autre, un atome d'hydrogène ou un groupe un groupe C₁₋₆-alkyle ;
à l'état de base ou de sel d'addition à un acide.

2. Composé de formule générale (I) selon la revendication 1, **caractérisé en ce que** :
- R₂ représente un groupe C₁₋₄-alkyle, C₃₋₄-cycloalkyle-C₁₋₄-alkyle, C₁₋₄-alkyloxy-C₁₋₄-alkyle, C₁₋₄-fluoroalkyle.

3. Composé de formule générale (I) selon la revendication 1 ou 2, **caractérisé en ce que** :
- R₃ représente atome d'hydrogène, de fluor ou de chlore ou un groupe méthyle, méthylamino, -NH₂, méthoxy.

4. Composé de formule générale (I) selon l'une quelconque des revendications 1 à 3, **caractérisé en ce que** :
- R₇ et R₈ représentent, indépendamment l'un de l'autre, un atome d'hydrogène ou un groupe méthyle.

5. Composé de formule générale (I) selon l'une quelconque des revendications 1 à 4, **caractérisé en ce que** :
- A représente un groupe C₁₋₇-alkylène éventuellement substitué par un ou deux groupes Rₐ ;
- B représente un groupe C₁₋₇-alkylène éventuellement substitué par un groupe R_{b} ;
- L représente un atome d'azote éventuellement substitué par un groupe R_{c} ou R_{d},
les atomes de carbone de A et de B étant éventuellement substitués par un ou plusieurs groupes R_{f} identiques ou différents l'un de l'autre ;
- deux groupes Rₐ peuvent former ensemble un groupe C₁₋₆-alkylène ;
- Rₐ et R_{b} peuvent former ensemble une liaison ou un groupe C₁₋₆-alkylène;
- Rₐ et R_{c} peuvent former ensemble une liaison ou un groupe C₁₋₆-alkylène ;
- R_{b} et R_{c} peuvent former ensemble une liaison ou un groupe C₁₋₆-alkylène ;
- R_{d} représente un groupe choisi parmi l'atome d'hydrogène et les groupes C₁₋₆-alkyle, C₃₋₇-cycloalkyle, C₃₋₇-cycloalkyl-C₁₋₆-alkyle, C₁₋₆-alkylthio-C-₁₋₆-alkyle, C₁₋₆-alkyloxy-C₁₋₆-alkyle, C₁₋₆-fluoroalkyle, hydroxy-C₁₋₆-alkyle ;
- R_{f} représente un groupe C₁₋₆-alkyle, C₃₋₇-cycloalkyle, C₁₋₆-alkyloxy-C₁₋₆-alkyle, C₃₋₇-cycloalkyloxy-C₁₋₄-alkyle, C₃₋₇-cycloalkyl-C₁₋₄-alkyloxy-C₁₋₄-alkyle, hydroxy-C₁₋₆-alkyle.

6. Composé de formule générale (I) selon l'une quelconque des revendications 1 à 4, **caractérisé en ce que** :
- A représente un groupe C₁₋₇-alkylène éventuellement substitué par un ou deux groupes Rₐ ;
- B représente un groupe C₁₋₇-alkylène éventuellement substitué par un groupe R_{b} ;
- L représente un atome de carbone substitué par deux groupes Rₑ₂ ;
les atomes de carbone de A et de B étant éventuellement substitués par un ou plusieurs groupes R_{f} identiques ou différents l'un de l'autre ;
- Deux Rₑ₂ forment avec l'atome de carbone qui les porte une monoamine cyclique comportant éventuellement un atome d'oxygène, cette monoamine cyclique étant éventuellement substituée par un ou plusieurs groupes R_{f} identiques ou différents l'un de l'autre ;
- R_{f} représente un groupe C₁₋₆-alkyle.

7. Composé de formule générale (I) selon l'une quelconque des revendications 1 à 4, **caractérisé en ce que** :
- A représente un groupe C₁₋₇-alkylène;
- B représente un groupe C₁₋₇-alkylène ;
- L représente un atome de carbone substitué par un groupe Rₑ₁ et un groupe R_{d} ;
- R_{d} représente un atome d'hydrogène ;
- Rₑ₁ représente un groupe -NR₄R₅ où R₄ et R₅ représentent, indépendamment l'un de l'autre, un atome d'hydrogène ou un groupe C₁₋₄ alkyle, ou bien Rₑ₁ représente une monoamine cyclique comportant éventuellement un atome d'oxygène, la monoamine cyclique étant éventuellement substituée par un ou plusieurs substituants choisis parmi les groupes C₁₋₆-alkyle, hydroxyle.

8. Composé de formule générale (I) selon l'une quelconque des revendications 1 à 4, **caractérisé en ce que** :
- R₂ représente un groupe méthyle, éthyle, isopropyle, isobutyle, cyclopropyle, cyclobutyle, cyclopropyl-méthyle, méthoxy-méthyle, trifluorométhyle ;
- R₃ représente atome d'hydrogène, de fluor ou de chlore ou un groupe méthyle, méthylamino, -NH₂, méthoxy ;
- L'amine cyclique formée par -N-A-L-B- représente un groupe pipérazin-1-yle, (*R,S*)-3-méthylpipérazin-1-yle, (*R*)-3-méthylpipérazin-1-yle, (*S*)-3-méthylpipérazin-1-yle, 4-méthylpipérazin-1-yle, 4-éthyl-pipérazin-1-yle, 4-(isopropyl)pipérazin-1-yle, 4-(cyclobutyl)pipérazin-1-yle, (*R,S*)-3-(hydroxyméthyl)-pipérazin-1-yle, 3,3-diméthylpipérazin-1-yle, *cis*-3,5-diméthylpipérazin-1-yle, (*S*)-hexahydropyrrolo[1,2-a]pyrazin-2-yle, (1*S*,4*S*)-2,5-diazabicyclo[2.2.1]hept-2-yle, (*R*,*S*)-2,5-diazabicyclo[2.2.1]hept-2-yle, (*R*,*S*)-1,4-diazabicyclonon-4-yle, (*R*,*S*)-hexahydropyrrolo[3,4-b]pyrrol-5(1*H*)-yle, hexahydropyrrolo[3,4-c]pyrrol-2(1*H*)-yle, 5-méthyl-hexahydropyrrolo[3,4-*c*]pyrrol-2(1*H*)-yle, 5-isopropyl-hexahydropyrrolo[3,4-*c*]pyrrol-2(1*H*)-yle, (*R,S*)-octahydropyrrolo[3,4-*b*]pyridin-6-yle ;
- R₇ et R₈ représentent, indépendamment l'un de l'autre, un atome d'hydrogène ou un groupe méthyle.

9. Composé de formule générale (I) selon l'une quelconque des revendications 1 à 4, **caractérisé en ce que** :
- R₂ représente un groupe méthyle ;
- R₃ représente atome d'hydrogène ;
- L'amine cyclique formée par -N-A-L-B- représente une 2,7-diaza-spiro[3.5]non-7-yle, (*R,S*)-diaza-spiro[4.5]déc-2-yle, 2,9-diaza-spiro[5.5]undéc-9-yle ou 1-oxa-4,9-diaza-spiro-undéc-9-yle ;
- R₇ et R₈ représentent, indépendamment l'un de l'autre, un atome d'hydrogène.

10. Composé de formule générale (I) selon l'une quelconque des revendications 1 à 4, **caractérisé en ce que** :
- R₂ représente un groupe méthyle ;
- R₃ représente atome d'hydrogène ou un groupe méthyle ;
- l'amine cyclique formée par -N-A-L-B- représente une 4-(pyrrolidin-1-yl)-pipéridin-1-yle ou une (*R,S*)-[1,3']bipyrrolidinyl-1'-yle ;
- R₇ et R₈ représentent, indépendamment l'un de l'autre, un atome d'hydrogène.

11. Composé de formule générale (I) selon la revendication 1, choisi parmi :
- 2-Méthyl-6-pipérazin-1-yl-3-pyridin-4-yl-imidazo[1,2-*b*]pyridazine et son chlorhydrate (3:1) ;
- 3-(2-Fluoro-pyridin-4-yl)-2-méthyl-6-pipérazin-1-yl-imidazo[1,2-*b*]pyridazine ;
- 2,7,8-Triméthyl-6-pipérazin-1-yl-3-pyridin-4-yl-imidazo[1,2-*b*]pyridazine et son chlorhydrate (3:1) ;
- 2-Méthoxyméthyl-6-pipérazin-1-yl-3-pyridin-4-yl-imidazo[1,2-*b*]pyridazine ;
- 2-Ethyl-6-pipérazin-1-yl-3-pyridin-4-yl-imidazo[1,2-*b*]pyridazine et son chlorhydrate (3:1) ;
- 2-Ethyl-6-pipérazin-1-yl-3-(2-méthyl-pyridin-4-yl)-imidazo[1,2-*b*]pyridazine ;
- 2-Isopropyl-6-pipérazin-1-yl-3-pyridin-4-yl-imidazo[1,2-*b*]pyridazine ;
- 2-Isopropyl-3-(2-méthyl-pyridin-4-yl)-6-pipérazin-1-yl-imidazo[1,2-*b*]pyridazine ;
- 2-Cyclopropyl-6-pipérazin-1-yl-3-pyridin-4-yl-imidazo[1,2-*b*]pyridazine ;
- 2-Cyclopropyl-3-(2-méthyl-pyridin-4-yl)-6-pipérazin-1-yl-imidazo[1,2-*b*]pyridazine ;
- 4-(2-Cyclopropyl-6-pipérazin-1-yl-imidazo[1,2-*b*]pyridazin-3-yl)-pyridin-2-ylamine ;
- 2-Cyclopropyl-3-(2-méthoxy-pyridin-4-yl)-6-pipérazin-1-yl-imidazo[1,2-*b*]pyridazine ;
- 3-(2-Chloro-pyridin-4-yl)-2-cyclopropyl-6-pipérazin-1-yl-imidazo[1,2-*b*]pyridazine ;
- 2-Isobutyl-6-pipérazin-1-yl-3-pyridin-4-yl-imidazo[1,2-*b*]pyridazine et son chlorhydrate (3:1) ;
- 2-Cyclopropylméthyl-6-pipérazin-1-yl-3-pyridin-4-yl-imidazo[1,2-*b*]pyridazine ;
- 2-Cyclobutyl-6-pipérazin-1-yl-3-pyridin-4-yl-imidazo[1,2-*b*]pyridazine ;
- (*R,S*)-2-Méthyl-6-(3-méthyl-pipérazin-1-yl)-3-pyridin-4-yl-imidazo[1,2-*b*]pyridazine ;
- (*R,S*)-2-Méthyl-6-(3-méthyl-pipérazin-1-yl)-3-(2-méthyl-pyridin-4-yl)-imidazo[1,2-*b*]pyridazine ;
- (*R,S*)-2-Cyclopropyl-6-(3-méthyl-pipérazin-1-yl)-3-(2-méthyl-pyridin-4-yl)-imidazo[1,2-*b*]pyridazine ;
- (*R,S*)-4-[2-Cyclopropyl-6-(3-méthyl-pipérazin-1-yl)-imidazo[1,2-*b*]pyridazin-3-yl]-pyridin-2-ylamine ;
- (*R,S*)-2-Cyclopropyl-3-(2-méthoxy-pyridin-4-yl)-6-(3-méthyl-pipérazin-1-yl)-imidazo[1,2-*b*]pyridazine ;
- (*R,S*)-3-(2-Chloro-pyridin-4-yl)-2-cyclopropyl-6-(3-méthyl-pipérazin-1-yl)-imidazo[1,2-*b*]pyridazine ;
- 2-Méthyl-6-((*R*)-3-méthyl-pipérazin-1-yl)-3-pyridin-4-yl-imidazo[1,2-*b*]pyridazine ;
- 2-Méthyl-6-((*R*)-3-méthyl-pipérazin-1-yl)-3-(2-méthyl-pyridin-4-yl)-imidazo[1,2-*b*]pyridazine ;
- 4-[2-Méthyl-6-((*R*)-3-méthyl-pipérazin-1-yl)-imidazo[1,2-*b*]pyridazin-3-yl]-pyridin-2-ylamine ;
- Méthyl-{4-[2-méthyl-6-((*R*)-3-méthyl-pipérazin-1-yl)-imidazo[1,2-*b*]pyridazin-3-yl]-pyridin-2-yl}-amine ;
- 3-(2-Méthoxy-pyridin-4-yl)-2-méthyl-6-((*R*)-3-méthyl-pipérazin-1-yl)-imidazo[1,2-*b*]pyridazine ;
- 2-Ethyl-6-((*R*)-3-méthyl-pipérazin-1-yl)-3-pyridin-4-yl-imidazo[1,2-*b*]pyridazine ;
- 2-Ethyl-3-(2-fluoro-pyridin-4-yl)-6-((*R*)-3-méthyl-pipérazin-1-yl)-imidazo[1,2-*b*]pyridazine ;
- 2-Cyclopropylméthyl-6-((*R*)-3-méthyl-pipérazin-1-yl)-3-pyridin-4-yl-imidazo[1,2-*b*]pyridazine ;
- 2-Cyclopropylméthyl-6-((*R*)-3-méthyl-pipérazin-1-yl)-3-(2-méthyl-pyridin-4-yl)-imidazo[1,2-*b*]pyridazine ;
- 2-Méthyl-6-((*S*)-3-méthyl-pipérazin-1-yl)-3-pyridin-4-yl-imidazo[1,2-*b*]pyridazine ;
- 3-(2-Methoxy-pyridin-4-yl)-2-méthyl-6-((*S*)-3-méthyl-pipérazin-1-yl)-imidazo[1,2-*b*]pyridazine ;
- 2-Cyclopropyl-6-((*S*)-3-méthyl-pipérazin-1-yl)-3-pyridin-4-yl-imidazo[1,2-*b*]pyridazine ;
- 4-[2-Méthyl-6-((*S*)-3-méthyl-pipérazin-1-yl)-imidazo[1,2-*b*]pyridazin-3-yl]-pyridin-2-ylamine ;
- 2-Méthyl-6-(4-méthyl-pipérazin-1-yl)-3-pyridin-4-yl-imidazo[1,2-*b*]pyridazine ;
- 6-(4-Méthyl-pipérazin-1-yl)-3-pyridin-4-yl-2-trifluorométhyl-imidazo[1,2-*b*]pyridazine ;
- [4-(2-Méthyl-3-pyridin-4-yl-imidazo[1,2-*b*]pyridazin-6-yl)-pipérazin-2-yl]-méthanol ;
- 6-(4-Ethyl-pipérazin-1-yl)-2-méthyl-3-pyridin-4-yl-imidazo[1,2-*b*]pyridazine et son chlorhydrate (3:1) ;
- 6-(4-Ethyl-pipérazin-1-yl)-2-méthyl-3-(2-méthyl-pyridin-4-yl)-imidazo[1,2-*b*]pyridazine ;
- 6-(4-Isopropyl-pipérazin-1-yl)-2-méthyl-3-pyridin-4-yl-imidazo[1,2-*b*]pyridazine et son chlorhydrate (3:1) ;
- 6-(4-Isopropyl-pipérazin-1-yl)-2-méthyl-3-(2-méthyl-pyridin-4-yl)-imidazo[1,2-*b*]pyridazine ;
- 4-[6-(4-Isopropyl-pipérazin-1-yl)-2-méthyl-imidazo[1,2-*b*]pyridazin-3-yl]-pyridin-2-yl}-amine et son chlorhydrate (3:1) ;
- 6-(4-Cyclobutyl-pipérazin-1-yl)-2-méthyl-3-pyridin-4-yl-imidazo[1,2-*b*]pyridazine ;
- 4-[6-(4-Cyclobutyl-pipérazin-1-yl)-2-méthyl-imidazo[1,2-*b*]pyridazin-3-yl]-pyridin-2-ylamine ;
- 6-(3,3-Diméthyl-pipérazin-1-yl)-2-méthyl-3-(2-méthyl-pyridin-4-yl)-imidazo[1,2-b]pyridazine ;
- {4-[6-(3,3-Diméthyl-pipérazin-1-yl)-2-méthyl-imidazo[1,2-b]pyridazin-3-yl]-pyridin-2-yl}-méthyl-amine ;
- 2-Cyclopropyl-6-(3,3-diméthyl-pipérazin-1-yl)-3-(2-méthyl-pyridin-4-yl)-imidazo[1,2-b]pyridazine ;
- 4-[2-Cyclopropyl-6-(3,3-diméthyl-pipérazin-1-yl)-imidazo[1,2-b]pyridazin-3-yl]-pyridin-2-ylamine ;
- 2-Cyclopropyl-6-(3,3-diméthyl-pipérazin-1-yl)-3-(2-fluoro-pyridin-4-yl)-imidazo[1,2-b]pyridazine ;
- 6-(*cis*-3,5-Diméthyl-pipérazin-1-yl)-2-méthyl-3-pyridin-4-yl-imidazo[1,2-b]pyridazine et son chlorhydrate (3:1) ;
- 6-(*cis*-3,5-Diméthyl-pipérazin-1-yl)-2-méthyl-3-(2-méthyl-pyridin-4-yl)-imidazo[1,2-*b*]pyridazine ;
- 4-[6-(*cis*-3,5-Diméthyl-pipérazin-1-yl)-2-méthyl-imidazo[1,2-*b*]pyridazin-3-yl]-pyridin-2-ylamine ;
- {4-[6-(*cis*-3,5-Diméthyl-pipérazin-1-yl)-2-méthyl-imidazo[1,2-*b*]pyridazin-3-yl]-pyridin-2-yl}-méthyl-amine ;
- 2-Cyclopropyl-6-(*cis*-3,5-diméthyl-pipérazin-1-yl)-3-pyridin-4-yl-imidazo[1,2-*b*]pyridazine ;
- 4-[2-Cyclopropyl-6-(*cis*-3,5-diméthyl-pipérazin-1-yl)-imidazo[1,2-*b*]pyridazin-3-yl]-pyridin-2-ylamine ;
- 6-(*S*)-Hexahydro-pyrrolo[1,2-*a*]pyrazin-2-yl-2-méthyl-3-pyridin-4-yl-imidazo[1,2-*b*]pyridazine ;
- 6-(*S*)-Hexahydro-pyrrolo[1,2-*a*]pyrazin-2-yl-2-méthyl-3-(2-méthyl-pyridin-4-yl)-imidazo[1,2-*b*]pyridazine ;
- 6-(1S,4S)-2,5-Diaza-bicyclo[2.2.1]hept-2-yl-2-méthyl-3-pyridin-4-yl-imidazo[1,2-*b*]pyridazine ;
- (*R*,*S*)-6-(2,5-Diaza-bicyclo[2.2.1]hept-2-yl)-2-méthyl-3-(2-méthyl-pyridin-4-yl)-imidazo[1,2-*b*]pyridazine et son bromhydrate (1:1) ;
- 4-[2-Cyclopropyl-6-(1,4-diaza-bicyclo[3.2.2]non-4-yl)-3-(2-méthyl-pyridin-4-yl)-imidazo[1,2-b]pyridazine ;
- (*R*,*S*)-6-(Hexahydro-pyrrolo[3,4-*b*]pyrrol-5(1*H*)-yl)-2-méthyl-3-(2-méthyl-pyridin-4-yl)-imidazo[1,2-*b*]pyridazine ;
- 6-Hexahydro-pyrrolo[3,4-*c*]pyrrol-2(1*H*)-yl-2-méthyl-3-pyridin-4-yl-imidazo[1,2-*b*]pyridazine ;
- 6-Hexahydro-pyrrolo[3,4-*c*]pyrrolo-2(1*H*)-yl-2-méthyl-3-(2-méthyl-pyridin-4-yl)-imidazo[1,2-*b*]pyridazine et son chlorhydrate (3: 1) ;
- 4-(6-Hexahydro-pyrrolo[3,4-*c*]pyrrol-2(1*H*)-yl-2-méthyl-imidazo[1,2-*b*]pyridazin-3-yl)-pyridin-2-ylamine ;
- [4-(6-Hexahydro-pyrrolo[3,4-*c*]pyrrol-2(1*H*)-yl-2-méthyl-imidazo[1,2-b]pyridazin-3-yl)-pyridin-2-yl]-méthyl-amine ;
- 2-Méthyl-6-(5-méthyl-hexahydro-pyrrolo[3,4-*c*]pyrrol-2(1*H*)-yl)-3-pyridin-4-yl-imidazo[1,2-*b*]pyridazine ;
- 2,7-Diméthyl-6-(5-méthyl-hexahydro-pyrrolo[3,4-*c*]pyrrol-2(1*H*)-yl)-3-pyridin-4-yl-imidazo[1,2-*b*]pyridazine ;
- 6-(-5-Isopropyl-hexahydro-pyrrolo[3,4-c]pyrrol-2(1*H*)-yl)-2-méthyl-3-pyridin-4-yl-imidazo[1,2-*b*]pyridazine ;
- 4-[6-(-5-Isopropyl-hexahydro-pyrrolo[3,4-*c*]pyrrol-2(1*H*)-yl)-2-méthyl-imidazo[1,2-*b*]pyridazin-3-yl]-pyridin-2-ylamine ;
- 6-Hexahydro-pyrrolo[3,4-*c*]pyrrol-2(1*H*)-yl-3-(2-méthoxy-pyridin-4-yl)-2-méthyl-imidazo[1,2-*b*]pyridazine ;
- (*R*,*S*)-2-Méthyl-6-(octahydro-pyrrolo[3,4-*b*]pyridin-6-yl)-3-pyridin-4-yl-imidazo[1,2-*b*]pyridazine ;
- 6-(2,7-Diaza-spiro[3.5]non-7-yl)-2-méthyl-3-(2-méthyl-pyridin-4-yl)-imidazo[1,2-*b*]pyridazine ;
- (*R*,*S*)-6-(2,7-Diaza-spiro[4.5]déc-2-yl)-2-méthyl-3-pyridin-4-yl-imidazo[1,2-*b*]pyridazine ;
- 9-(2-Méthyl-3-pyridin-4-yl-imidazo[1,2-*b*]pyridazin-6-yl)-2,9-diaza-spiro[5.5]undécane et son chlorhydrate (3:1) ;
- 9-[2-Méthyl-3-(2-méthyl-pyridin-4-yl)-imidazo[1,2-*b*]pyridazin-6-yl]-2,9-diaza-spiro[5.5]undécane et son chlorhydrate (3:1) ;
- 9-(2-Méthyl-3-pyridin-4-yl-imidazo[1,2-*b*]pyridazin-6-yl)-1-oxa-4,9-diaza-spiro[5.5]undécane et son chlorhydrate (3:1) ;
- 4-[2-Méthyl-6-(1-oxa-4,9-diaza-spiro[5.5]undéc-9-yl)-imidazo[1,2-*b*]pyridazin-3-yl]-pyridin-2-ylamine et son chlorhydrate (3:1) ;
- 2-Méthyl-3-pyridin-4-yl-6-(4-pyrrolidin-1-yl-pipéridin-1-y)-imidazo[1,2-*b*]pyridazine ;
- 2-Méthyl-3-(2-méthyl-pyridin-4-yl)-6-(4-pyrrolidin-1-yl-pipéridin-1-yl)-imidazo[1,2-*b*]pyridazine ;
- 4-[2-Méthyl-6-(4-pyrrolidin-1-yl-pipéridin-1-yl)-imidazo[1,2-b]pyridazin-3-yl]-pyridin-2-ylamine ;
- (*R*,*S*)-6-[1,3']Bipyrrolidinyl-1'-yl-2-méthyl-3-pyridin-4-yl-imidazo[1,2-b]pyridazine.

12. Procédé de préparation d'un composé de formule (I) selon la revendication 1, **caractérisé en ce que** l'on fait réagir un composé de formule générale (IIa) 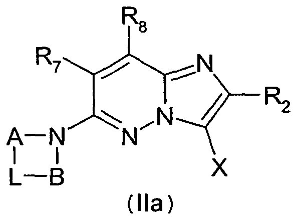 dans laquelle R₂, A, L, B, R₇ et R₈ sont tels que définis selon la revendication 1 et X représente un atome de brome ou d'iode, avec un composé de formule (IVa) 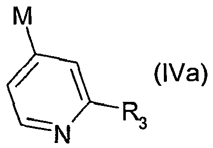 dans laquelle R₃ est tel que défini dans la revendication 1 et M représente un groupe trialkylstannyle, dihydroxyboryle ou dialkoxyboryle.

13. Procédé de préparation d'un composé de formule (I) selon la revendication 1, **caractérisé en ce que** :
a) on fait réagir un composé de formule générale (II) 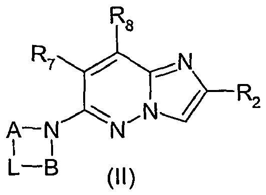 dans laquelle R₂, A, L, B, R₇ et R₈ sont tels que définis selon la revendication 1 avec un mélange d'un dérivé de pyridine de formule générale (IVb) 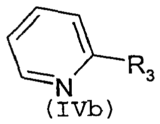 dans laquelle R₃ représente un atome d'hydrogène ou un groupe C₁₋₃-alkyle en présence de chloroformiate d'alkyle, pour obtenir un composé de formule (IIb) 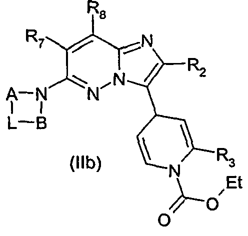 dans laquelle R₂, A, L, B, R₇ et R₈ sont tels que définis selon la revendication 1 et R₃ représente un atome d'hydrogène ou un groupe C₁₋₃-alkyle ;
b) on fait réagir le composé de formule générale (IIb) obtenu à l'étape a) avec de l'ortho-chloranile dans un solvant.

14. Procédé de préparation d'un composé de formule (I) selon la revendication 1, **caractérisé en ce que** l'on fait réagir un composé de formule générale (II) 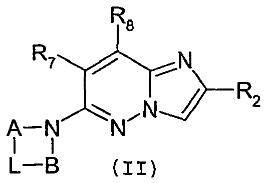 dans laquelle R₂, R₇, R₈, A, L, et B sont tels que définis selon la revendication 1, avec un composé de formule générale (IVc) 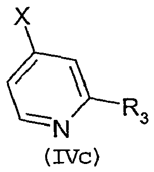 dans la quelle R₃ est tel que défini selon la revendication 1 et X représente un atome d'halogène, en présence d'un catalyseur, d'une base minérale et dans un solvant polaire aprotique.

15. Médicament, **caractérisé en ce qu'**il comprend un composé de formule (I) selon l'une quelconque des revendications 1 à 11, à l'état de base ou de sel d'addition à un acide pharmaceutiquement acceptable.

16. Composition pharmaceutique, **caractérisée en ce qu'**elle comprend un composé de formule (I) selon l'une quelconque des revendications 1 à 11, à l'état de base ou de sel d'addition à un acide pharmaceutiquement acceptable, ainsi qu'au moins un excipient pharmaceutiquement acceptable.

17. Utilisation d'un composé de formule générale (I) selon l'une quelconque des revendications 1 à 11, pour la préparation d'un médicament destiné au traitement ou à la prévention des désordres du sommeil, des troubles du rythme circadien.

18. Utilisation d'un composé de formule générale (I) selon l'une quelconque des revendications 1 à 11, pour la préparation d'un médicament destiné au traitement ou à la prévention des troubles bipolaires.

19. Utilisation d'un composé de formule générale (I) selon l'une quelconque des revendications 1 à 11, pour la préparation d'un médicament destiné au traitement ou à la prévention des maladies liées à la dépendance à des substances d'abus.

20. Utilisation d'un composé de formule générale (I) selon l'une quelconque des revendications 1 à 11, pour la préparation d'un médicament destiné au traitement ou à la prévention des maladies reliées à l'hyperphosphorylation de la protéine tau.

21. Utilisation d'un composé de formule générale (I) selon l'une quelconque des revendications 1 à 11, pour la préparation d'un médicament destiné au traitement ou à la prévention des maladies causées ou exacerbées par la prolifération des cellules.

22. Utilisation d'un composé de formule générale (I) selon la revendication 21, **caractérisé en ce que** les cellules sont des cellules tumorales.

## Claims

1. Compound confirming to the general formula (I) 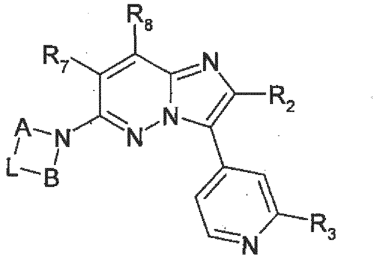 in which
- R₂ represents a C₁₋₆-alkyl, C₃₋₇-cycloalkyl, C₃₋₇-cycloalkyl-C₁₋₄-alkyl, C₁₋₄-alkyloxy-C₁₋₄-alkyl, C₃₋₇-cycloalkyloxy-C₁₋₄-alkyl, C₃₋₇-cycloalkyl-C₁₋₄-alkyloxy-C₁₋₄-alkyl, hydroxy-C₁₋₆-alkyl or C₁₋₄-fluoroalkyl group;
- R₃ represents a hydrogen atom or a substituent selected from halogen atoms and C₁₋₃-alkyl, -NR₄R₅, hydroxyl or C₁₋₄-alkyloxy groups;
- A represents a C₁₋₇-alkylene group optionally substituted by one or two groups Rₐ;
- B represents a C₁₋₇-alkylene group optionally substituted by a group R_{b};
- L represents either a nitrogen atom optionally substituted by a group R_{c} or R_{d}, or a carbon atom substituted by a group Rₑ₁ and a group R_{d} or two groups Rₑ₂;
the carbon atoms of A and of B being optionally substituted by one or more groups R_{f} that are identical or different from one another;
Rₐ, R_{b} and R_{c} are defined such that:
two groups Rₐ may together form a C₁₋₆-alkylene group;
Rₐ and R_{b} may together form a bond or a C₁₋₆-alkylene group;
Rₐ and R_{c} may together form a bond or a C₁-₆-alkylene group;
R_{b} and R_{c} may form a bond or a C₁₋₆-alkylene group;
R_{d} represents a group selected from the hydrogen and C₁₋₆-alkyl, C₃₋₇-cycloalkyl, C₃₋₇-cycloalkyl-C₁₋₆-alkyl, C₁₋₆-alkylthio-C₁₋₆-alkyl, C₁₋₆-alkyloxy-C₁₋₆-alkyl, C₁₋₆-fluoroalkyl and hydroxy-C₁₋₆-alkyl groups;
Rₑᵢ represents a group -NR₄R₅ or a cyclic monoamine optionally containing an oxygen atom, the cyclic monoamine being optionally substituted by one or more selected from the fluorine atom and C₁₋₆-alkyl, C₁₋₆-alkyloxy and hydroxyl groups;
two radicals Rₑ₂ form, with the carbon atom which carries them, a cyclic monoamine optionally containing an oxygen atom, this cyclic monoamine being optionally substituted by one or more groups R_{f} that are identical or different from one another;
R_{f} represents a C₁₋₆-alkyl, C₃₋₇-cycloalkyl, C₃₋₇-cycloalkyl-C₁₋₆-alkyl, C₁₋₆-alkyloxy-C₁₋₆-alkyl, C₃₋₇-cycloalkyloxy-C₁₋₄-alkyl, C₃₋₇-cycloalkyl-C₁₋₄-alkyloxy-C₁₋₄-alkyl, hydroxy-C₁₋₆-alkyl or C₁₋₆-fluoroalkyl group;
R₄ and R₅ represent, independently of one another, a hydrogen atom or a C₁₋₄ alkyl, C₃₋₇-cycloalkyl or C₃₋₇-cycloalkyl-C₁₋₆-alkyl group;
R₇ and R₈ represent, independently of one another, a hydrogen atom or a C₁₋₆-alkyl group;
in the form of a base or acid addition salt.

2. Compound of general formula (I) according to Claim 1, **characterized in that**:
- R₂ represents a C₁₋₄-alkyl, C₃₋₄-cycloalkyl-C₁₋₄-alkyl, C₁₋₄-alkyloxy-C₁₋₄-alkyl or C₁₋₄-fluoroalkyl group.

3. Compound of general formula (I) according to Claim 1 or 2, **characterized in that**:
- R₃ represents hydrogen, fluorine or chlorine atom or a methyl, methylamino, -NH₂ or methoxy group.

4. Compound of general formula (I) according to any one of Claims 1 to 3, **characterized in that**:
- R₇ and Rₐ represent, independently of one another, a hydrogen atom or a methyl group.

5. Compound of general formula (I) according to any of Claims 1 to 4, **characterized in that**:
- A represents a C₁₋₇-alkylene group optionally substituted by one or two groups Rₐ;
- B represents a C₁₋₇-alkylene group optionally substituted by a group R_{b};
- L represents a nitrogen atom optionally substituted by a group R_{c} or R_{d},
the carbon atoms of A and of B being optionally substituted by one or more groups R_{f} that are identical or different from one another;
- two groups Rₐ may together form a C₁₋₆-alkylene group;
- Rₐ and R_{b} may together form a bond or or a C₁₋₆-alkylene group;
- Rₐ and R_{c} may together form a bond or a C₁₋₆-alkylene group;
- R_{b} and R_{c} may together form a bond or a C₁₋₆-alkylene group;
- R_{d} represents a group selected from the hydrogen atom and C₁₋₆-alkyl, C₃₋₇-cycloalkyl, C₃₋₇-cycloalkyl-C₁₋₆-alkyl, C₁₋₆-alkylthio-C-₁₋₆-alkyl, C₁₋₆-alkyloxy-C₁₋₆-alkyl, C₁₋₆-fluoroalkyl and hydroxy-C₁₋₆-alkyl groups;
- R_{f} represents a C₁₋₆-alkyl, C₃₋₇-cycloalkyl, C₁₋₆-alkyloxy-C₁₋₆-alkyl, C₃₋₇-cycloalkyloxy-C₁₋₄-alkyl, C₃₋₇-cycloalkyl-C₁₋₄-alkyloxy-C₁₋₄-alkyl or hydroxy-C₁₋₆-alkyl group.

6. Compound of general formula (I) according to any one of Claims 1 to 4, **characterized in that**:
- A represents a C₁₋₇-alkylene group optionally substituted by one or two groups Rₐ;
- B represents a C₁₋₇-alkylene group optionally substituted by a group R_{b};
- L represents a carbon atom substituted by two groups Rₑ₂;
the carbon atoms of A and of B being optionally substituted by one or more groups R_{f} that are identical or different from one another;
- two radicals Rₑ₂ form, with the carbon atom which carries them, a cyclic monoamine optionally containing an oxygen this cyclic monoamine being optionally substituted by or more groups R_{f} that are identical or different from one another;
- R_{f} represents a C₁₋₆-alkyl group.

7. Compound of general formula (I) according to any one of Claims 1 to 4, **characterized in that**:
- A represents a C₁₋₇-alkylene group;
- B represents a C₁₋₇-alkylene group;
- _{L} represents a carbon atom substituted by a group Rₑ₁ and a group R_{d};
- R_{d} represents a hydrogen atom;
- Rₑ₁ represents a group -NR₄R₅ in which R₄ and R₅ represent, independently of one another, a hydrogen atom or a C₁₋₄-alkyl group, or Rₑ₁ represents a cyclic monoamine optionally containing an oxygen atom, the cyclic monoamine being optionally substituted by one or more substituents selected from C₁₋₆-alkyl and hydroxyl groups.

8. Compound of general formula (I) according to any one of Claims 1 to 4, **characterized in that**:
- R₂ represents a methyl, ethyl, isopropyl, isobutyl, cyclopropyl, cyclobutyl, cyclopropylmethyl, methoxymethyl or trifluoromethyl group;
- R₃ represents hydrogen, fluorine or chlorine atom or a methyl, methylamino, -NH₂ or methoxy group;
- the cyclic amine formed by -N-A-L-B- represents a piperazin-1-yl, (*R*,*S*)-3-methylpiperazin-1-yl, (*R*)-3-methylpiperazin-1-yl, (*S*)-3-methylpiperazin-1-yl, 4-methylpiperazin-1-yl, 4-ethyl-piperazin-1-yl, 4-(isopropyl)piperazin-1-yl, 4-(cyclobutyl)piperazin-1-yl, (*R*,*S*)-3-(hydroxymethyl)piperazin-1-yl, 3,3-dimethylpiperazin-1-yl, *cis*-3,5-dimethylpiperazin-1-yl, (*S*)-hexahydrop-yrrolo[1,2-a]pyrazin-2-yl, (1*S*,4*S*)-2,5-diazabicyclo[2.2.1]hept-2-yl, (*R*,*S*)-2,5-diazabicyclo[2.2.1]hept-2-yl, (*R*,*S*)-1,4-diazabicyclonon-4-yl, (*R*,*S*)-hexahydropyrrolo[3,4-*b*]pyrrol-5(1*H*)-yl, hexahydropyrrolo[3,4-*c*]pyrrol-2(1*H*)-yl, 5-methylhexahydropyrrolo[3,4-*c*]pyrrol-2(1*H*)-yl, 5-isopropylhexahydropyrrolo[3,4-*c*]pyrrol-2(1*H*)-yl or (*R*,*S*)-octahydropyrrolo-[3,4-*b*]pyridin-6-yl group;
- R₇ and R₈ represent, independently of one another, a hydrogen atom or a methyl group.

9. Compound of general formula (I) according to any one of Claims 1 to 4, **characterized in that**:
- R₂ represents a methyl group;
- R₃ represents hydrogen atom;
- the cyclic amine formed by -N-A-L-B- represents a 2,7-diazaspiro[3.5]non-7-yl, (*R*,*S*)-diazaspiro[4,5]dec-2-yl, 2,9-diazaspiro[5.5]undec-9-yl or 1-oxa-4,9-diazaspiroundec-9-yl;
- R₇ and R₈ represent, independently of one another, a hydrogen atom.

10. Compound of general formula (I) according to any one of Claims 1 to 4, **characterized in that**:
- R₂ represents a methyl group;
- R₃ represents hydrogen atom or a methyl group;
- the cyclic amine formed by -N-A-L-B- represents a 4-(pyrrolidin-1-yl)piperidin-1-yl or an (*R*,*S*)-[1,3']bipyrrolidinyl-1'-yl;
- R₇ and R₈ represent, independently of one another, a hydrogen atom.

11. Compound of general formula (I) according to Claim 1, selected from:
- 2-Methyl-6-piperazin-1-yl-3-pyridin-4-yl-imidazo[1,2-*b*]pyridazine and its hydrochloride (3:1);
- 3-(2-Fluoropyridin-4-yl)-2-methyl-6-piperazin-1-ylimidazo[1,2-*b*]pyridazine;
- 2,7,8-Trimethyl-6-piperazin-1-yl-3-pyridin-4-ylimidazo[1,2-*b*]pyridazine and its hydrochloride (3:1);
- 2-Methoxymethyl-6-piperazin-1-yl-3-pyridin-4-ylimidazo[1,2-*b*]pyridazine;
- 2-Ethyl-6-piperazin-1-yl-3-pyridin-4-yl-imidazo[1,2-*b*]pyridazine and its hydrochloride (3:1);
- 2-Ethyl-6-piperazin-1-yl-3-(2-methylpyridin-4-yl)imidazo[1,2-*b*]pyridazine;
- 2-Isopropyl-6-piperazin-1-yl-3-pyridin-4-ylimidazo[1,2-*b*]pyridazine;
- 2-Isopropyl-3-(2-methylpyridin-4-yl)-6-piperazin-1-ylimidazo[1,2-*b*]pyridazine; 2-Cyclopropyl-6-piperazin-1-yl-3-pyridin-4-ylimidazo[1,2-*b*]pyridazine;
- 2-Cyclopropyl-3-(2-methylpyridin-4-yl)-6-piperazin-1-ylimidazo[1,2-*b*]pyridazine;
- 4-(2-Cyclopropyl-6-piperazin-1-ylimidazo[1,2-*b*]pyridazin-3-yl)pyridin-2-ylamine;
- 2-Cyclopropyl-3-(2-methoxypyridin-4-yl)-6-piperazin-1--ylimidazo[1,2-*b*]pyridazine;
- 3-(2-Chloropyridin-4-yl)-2-cyclopropyl-6-piperazin-1-ylimidazo[1,2-*b*]pyridazine;
- 2-isobutyl-6-piperazin-1-yl-3-pyridin-4-ylimidazo[1,2-*b*]pyridazine and its hydrochloride (3:1);
- 2-Cyclopropylmethyl-6-piperazin-1-yl-3-pyridin-4-ylimidazo[1,2-*b*]pyridazine; 2-Cyclobutyl-6-piperazin-1-yl-3-pyridin-4-ylimidazo[1,2-*b*]pyridazine;
- (*R*,*S*)-2-Methyl-6-(3-methylpiperazin-1-yl)-3-pyridin-4-ylimidazo[1,2-*b*]pyridazine;
- (*R*,*S*)-2-Methyl-6-(3-methylpiperazin-1-yl)-3-(2-methylpyridin-4-yl)imidazo[1,2-*b*]pyridazine;
- (*R*,*S*)-2-Cyclopropyl-6-(3-methylpiperazin-1-yl)-3-(2-methylpyridin-4-yl)imidazo[1,2-*b*]pyridazine;
- (*R*,*S*)-4-[2-Cyclopropyl-6-(3-methylpiperazin-1-yl)imidazo[1,2-*b*]pyridazin-3-yl]pyridin-2-ylamine;
- (*R*,*S*)-2-Cyclopropyl-3-(2-methoxypyridin-4-yl)-6-(3-methylpiperazin-1-yl)imidazo[1,2-*b*]pyridazine;
- (*R*,*S*)-3-(2-Chloropyridin-4-yl)-2-cyclopropyl-6-(3-methylpiperazin-1-yl)imidazo[1,2-*b*]pyridazine;
- 2-Methyl-6-((*R*)-3-methylpiperazin-1-yl)-3-pyridin-4-ylimidazo[1,2-*b*]pyridazine;
- 2-Methyl-6-((*R*)-3-methylpiperazin-1-yl)-3-(2-methylpyridin-4-yl)imidazo[1,2-*b*]pyridazine;
- 4-[2-Methyl-6-((*R*)-3-methylpiperazin-1-yl)imidazo[1,2-*b*]pyridazin-3-yl]pyridin-2-ylamine;
- Methyl-{4-[2-methyl-6-((*R*)-3-methylpiperazin-1-yl)imidazo[1,2-b]pyridazin-3-yl]pyridin-2-yl}-amine;
- 3-(2-Methoxypyridin-4-yl)-2-methyl-6-((*R*)-3-methylpiperazin-1-yl)imidazo[1,2-*b*]pyridazine;
- 2-Ethyl-6-((*R*)-3-methylpiperazin-1-yl)-3-pyridin-4-ylimidazo[1,2-*b*]pyridazine;
- 2-Ethyl-3-(2-fluoropyridin-4-yl)-6-((*R*)-3-methylpiperazin-1-yl)imidazo[1,2-b]pyridazine;
- 2-Cyclopropylmethyl-6-((*R*)-3-methylpiperazin-1-yl)-3-pyridin-4-ylimidazo[1,2-*b*]pyridazine;
- 2-Cyclopropylmethyl-6-((*R*)-3-methylpiperazin-1-yl)-3-(2-methylpyridin-4-yl)imidazo[1,2-*b*]pyridazine;
- 2-Methyl-6-((*S*)-3-methylpiperazin-1-yl)-3-pyridin-4-ylimidazo[1,2-*b*]pyridazine;
- 3-(2-Methoxypyridin-4-yl)-2-methyl-6-((*S*)-3-methylpiperazin-1-yl)imidazo[1,2-*b*]pyridazine;
- 2-Cyclopropyl-6-((*S*)-3-methylpiperazin-1-yl)-3-pyridin-4-ylimidazo[1,2-*b*]pyridazine;
- 4-[2-Methyl-6-((*S*)-3-methylpiperazin--yl)imidazo[1,2-*b*]pyridazin-3-yl]pyridin-2-ylamine; 2-Methyl-6-(4-methylpiperazin-1-yl)-3-pyridin-4-ylimidazo[1,2-*b*]pyridazine;
- 6-(4-Methylpiperazin-1-yl)-3-pyridin-4-yl-2-trifluoromethylimidazo[1,2-*b*]pyridazine;
- [4-(2-Methyl-3-pyridin-4-ylimidazo[1,2-*b*]pyridazin-6-yl)piperazin-2-yl]methanol;
- 6-(4-Ethylpiperazin-1-yl)-2-methyl-3-pyridin-4-ylimidazo[1,2-*b*]pyridazine and its hydrochloride (3:1);
- 6-(4-Ethylpiperazin-1-yl)-2-methyl-3-(2-methylpyridin-4-yl)imidazo[1,2-*b*]pyridazine;
- 6-(4-Isopropylpiperazin-1-yl)-2-methyl-3-pyridin-4-ylimidazo[1,2-*b*]pyridazine and its hydrochloride (3:1);
- 6-(4-Isopropylpiperazin-1-yl)-2-methyl-3-(2-methylpyridin-4-yl)imidazo[1,2-*b*]pyridazine;
- 4-[6-(4-lsopropylpiperazin-1-yl)-2-methylimidazo[1,2-*b*]pyridazin-3-yl]pyridin-2-yl}-amine and its hydrochloride (3:1);
- 6-(4-Cyclobutylpiperazin-1-yl)-2-methyl-3-pyridin-4-ylimidazo[1,2-b]pyridazine;
- 4-[6-(4-Cyclobutylpiperazin-1-yl)-2-methylimidazo[1,2-*b*]pyridazin-3-yl]pyridin-2-ylamine;
- 6-(3,3-Dimethylpiperazin-1-yl)-2-methyl-3-(2-methylpyridin-4-yl)imidazo[1,2-b]pyridazine;
- (4-[6-(3,3-Dimethylpiperazin-1-yl)-2-methylimidazo[1,2-b]pyridazin-3-yl]pyridin-2-yl)methyl-amine;
- 2-Cyclopropyl-6-(3,3-dimethylpiperazin-1-yl)-3-(2-methylpyridin-4-yl)imidazo[1,2-b]pyridazine;
- 4-[2-Cyclopropyl-6-(3,3-dimethylpiperazin-1-yl)imidazo[1,2-b]pyridazin-3-yl]pyridin-2-ylamine;
- 2-Cyclopropyl-6-(3,3-dimethylpiperazin-1-yl)-3-(2-fluoropyridin-4-yl)imidazo[1,2-b]pyridazine;
- 6-(*cis*-3,5-Dimethylpiperazin-1-yl)-2-methyl-3-pyridin-4-ylimidazo[1,2-*b*]pyridazine and its hydrochloride (3:1);
- 6-(*cis-*3,5-Dimethylpiperazin-1-yl)-2-methyl-3-(2-methylpyridin-4-yl)imidazo[1,2-*b*]pyridazine;
- 4-[6-(*cis*-3,5-Dimethylpiperazin-1-yl)-2-methylimidazo[1,2-*b*]pyridazin-3-yl]pyridin-2-ylamine;
- {4-[6-(*cis*-3,5-Dimethylpiperazin-1-yl)-2-methylimidazo[1,2-*b*]pyridazin-3-yl]pyridin-2-yl}-methylamine;
- 2-Cyclopropyl-6-(*cis*-3,5-dimethylpiperazin-1-yl)-3-pyridin-4-ylimidazo[1,2-*b*]pyridazine;
- 4-[2-Cyclopropyl-6-(*cis*-3,5-dimethylpiperazin-1-yl)imidazo[1,2-*b*]pyridazin-3-yl]pyridin-2-ylamine;
- 6-(*S*)-Hexahydropyrrolo[1,2-*a*]pyrazin-2-yl-2-methyl-3-pyridin-4-ylimidazo[1,2-*b*]pyridazine;
- 6-(*S*)-Hexahydropyrrolo[1,2-a]pyrazin-2-yl-2-methyl-3-(2-methylpyridin-4-yl)imidazo[1,2-*b*]pyridazine;
- 6-(1*S*,4*S*)-2,5-Diazabicyclo[2.2.1]hept-2-yl-2-methyl-3-pyridin-4-ylimidazo[1,2-*b*]pyridazine;
- (*R,S*)-6-(2,5-Diazabicyclo[2.2.1]hept-2-yl)-2-methyl-3-(2-methylpyridin-4-yl)imidazo[1,2-*b*]pyridazine and its hydrobromide (1:1);
- 4-[2-Cyclopropyl-6-(1,4-diazabicyclo[3.2.2]non-4-yl)-3-(2-methylpyridin-4-yl)imidazo[1,2-b]pyridazine; ;
- (*R,S*)-6-(Hexahydropyrrolo[3,4-*b*]pyrrol-5(1*H*-yl)-2-methyl-3-(2-methylpyridin-4-yl)-imidazo[1,2-*b*]pyridazine;
- 6-Hexahydropyrrolo[3,4-*c*]pyrrol-2(1*H*)-yl-2-methyl-3-pyridin-4-ylimidazo[1,2-*b*]pyridazine-,
- 6-Hexahydropyrrolo[3,4-*c*]pyrrol-2(1*H*)-yl-2-methyl-3-(2-methylpyridin-4-yl)-imidazo[1,2-*b*]pyridazine and its hydrochloride (3:1);
- 4-(6-Hexahydropyrrolo[3,4-*c*]pyrrol-2(1*H*)-yl-2-methylimidazo[1,2-*b*]pyridazin-3-yl)pyridin-2-ylamine;
- [4-(6-Hexahydropyrrolo[3,4-*c*]pyrrol-2(1*H*)-yl-2-methylimidazo[1,2-*b*]pyridazin-3-yl)pyridin-2-yl]-methylamine;
- 2-Methyl-6-(5-methylhexahydropyrrolo[3,4-*c*]pyrrol[3,4-*c*]pyrrol-2(1*H*)-yl)-3-pyridin-4-ylimidazol[1,2-*b*]pyridazine;
- 2,7-Dimethyl-6-(5-methylhexahydropyrrolo[3,4-*c*]pyrrol-2(1*H*)-yl)-3-pyridin-4-yl-imidazo[1,2-*b*]pyridazine;
- 6-(-5-Isopropylhexahydropyrrolo[3,4-*c*]pyrrol-2(1*H*)-yl)-2-methyl-3-pyridin-4-ylimidazo[1,2-*b*]pyridazine;
- 4-[6-(-5-Isopropylhexahydropyrrolo[3,4-*c*]pyrrol-2(1*H*)-yl)-2-methylimidazo[1,2-*b*]pyridazin-3-yl]pyridin-2-ylamine;
- 6-Hexahydropyrrolo[3,4-*c*]pyrrol-2(1*H*)-yl-3-(2-methoxypyridin-4-yl)-2-methylimidazo[1,2-*b*]pyridazine;
- (*R,S*)-2-Methyl-6-(octahydropyrrolo[3,4-*b*]pyridin-6-yl)-3-pyridin-4-ylimidazo[1,2-*b*]pyridazine;
- 6-(2,7-Diazaspiro[3.5]non-7-yl)-2-methyl-3-(2-methylpyridin-4-yl)imidazo[1,2-*b*]pyridazine;
- (*R,S*)-6-(2,7-Diazaspiro[4.5]dec-2-yl)-2-methyl-3-pyridin-4-ylimidazo[1,2-*b*]pyridazine;
- 9-(2-Methyl-3-pyridin-4-yl-imidazo[1,2-*b*]pyridazin-6-yl)-2,9-diazaspiro[5.5]undecane and its hydrochloride (3:1);
- 9-[2-Methyl-3-(2-methylpyridin-4-yl)imidazo[1,2-*b*]pyridazin-6-yl]-2,9-diazaspiro[5.5]undecane and it hydrochloride (3:1);
- 9-(2-Methyl-3-pyridin-4-ylimidazo[1,2-*b*]pyridazin-6-yl)-1-oxa-4,9-and it hydrochloride (3:1);
- 4-[2-Methyl-6-(1-oxa-4,9-diazaspiro[5.5]undec-9-yl)imidazo[1,2-b]pyridazin-3-yl]pyridin-2-ylamine and its hydrochloride (3:1);
- 2-Methyl-3-pyridin-4-yl-6-(4-pyrrolidin-1-ylpiperidin-1-yl)imidazo[1,2-*b*]pyridazine;
- 2-Methyl-3-(2-methylpyridin-4-yl)-6-(4-pyrrolidin-1-ylpiperidin-1-yl)imidazo[1,2-*b*]pyridazine;
- 4-[2-Methyl-6-(4-pyrrolidin-1-yipiperidin-1-yl)imidazo[1,2-*b*]pyridazin-3-yl]pyridin-2-ylamine;
- (*R,S*)-6-[1,3']Bipyrrolidinyl-1'-yl-2-methyl-3-pyridin-4-ylimidazo[1,2-*b*]pyridazine.

12. Process for preparing a compound of formula (I) according to Claims 1, **characterized in that** a compound of general formula (IIa) 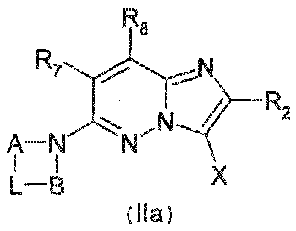 in which R₂, A, L, B, R₇ and R₈ are as defined according to Claim 1 and X represents a bromine or iodine atom is reacted it compound of formula (IVa) 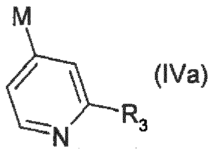 in which R₃ is as defined in Claim 1 and M represents a trialkylstannyl, dihydroxyboryl or dialkoxyboryl group.

13. for preparing a compound of formula (I) according to Claim 1, **characterized in that**:
a) a compound of general formula (II) 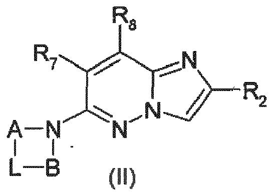 in which R₂, A, L, B, R₇ and Rₐ are as defined according to Claim 1 is reacted with a mixture of a pyridine derivative of general formula (IVb) 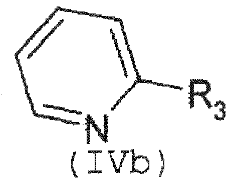 in which 3 represents a hydrogen atom or a C₁₋₃-alkyl group in the presence of alkyl chloroformate, to give a compound of formula (IIb) 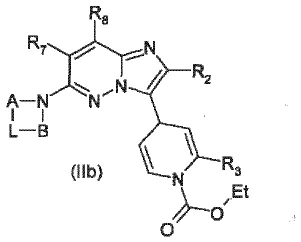 in which R₂, A, L, B, R₇ and R₈ are as defined according to Claim 1 and R₃ represents a hydrogen atom or a C₁₋₃-alkyl group; and
b) the compound of general formula (IIb) obtained in step a) is reacted with ortho-chloranil in a solvent.

14. Process for preparing a compound of formula (I) according to Claim 1, **characterized in that** a compound of general formula (II) 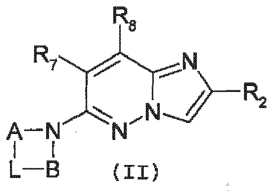 in which R₂, R₇, R₈, A, L and B are as defined according to Claim 1, is reacted with a compound of general formula (IVc) 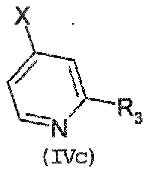 in which R₃ is as defined according to Claim 1 and X represents a halogen atom, in the presence of a catalyst and of an inorganic base and in an aprotic polar solvent.

15. Medicament, **characterized in that** it comprises a compound of formula (I) to any one of Claims 1 to 11, in the form a base or an addition salt with pharmaceutically acceptable acid.

16. Pharmaceutical composition, **characterized in that** it comprises a compound of formula (I) according to any of Claims 1 to 11, in the form of a base or of an addition salt with a pharmaceutically acceptable acid, and also art one pharmaceutically acceptable excipient.

17. Use of a compound of general formula (I) according to any one of Claims 1 to 11, for the preparation of a medicament for treating or preventing sleep disorders or circadian rhythm disorders.

18. Use of a compound general formula (I) according to any one of Claims 1 to 11, for the preparation of a medicament for treating or preventing bipolar disorders.

19. Use of a compound of general formula (I) according to any one of Claims 1 to 11, for the preparation of a medicament for treating or preventing diseases associated with a dependence on abuse substances.

20. Use of a compound of general formula (I) according to any one of Claims 1 to 11, for the preparation of a medicament for treating or preventing diseases related to hyperphosphorylation of the tau protein.

21. Use of a compound of general formula (I) according to any one of Claims 1 to 11, for the preparation of a medicament for treating or preventing diseases caused or exacerbated by cell proliferation.

22. Use of a compound of general formula (I) according to Claim 21, **characterized in that** the cells are tumour cells.

## Patentansprüche

1. Verbindungen der allgemeinen Formel (I) 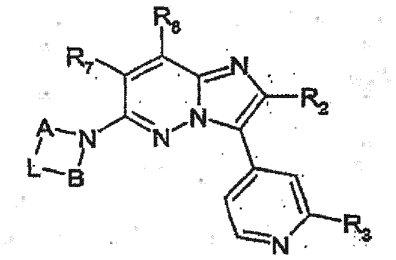 worin
- R₂ für eine C₁₋₆₋Alkyl- C₃₋₇-Cycloalkyl-, C₃₋₇-Cycloalkyl-C₁₋₄-alkyl-, C₁₋₄-Alkyloxy-C₁₋₄-alkyl-, C₃₋₇-Cycloalkyloxy-C₁-₄-alkyl-, C₃₋₇-Cycloalkyl-C₁₋₄-alkyloxy-C₁₋₄-alkyl-, Hydroxy-C₁₋₆-alkyl- oder C₁₋₄-Fluoralkylgruppe steht;
- R₃ für ein Wasserstoffatom oder einen unter Halogenatomen und C₁₋₃-Alkyl-, -NR₄R₅-, Hydroxyl-oder C₁₋₄-Alkoxygruppen ausgewählten Substituenten steht;
- A für eine C₁₋₇-Alkylengrüppe, die gegebenenfalls durch eine oder zwei Gruppen Rₐ substituiert ist, steht;
- B für eine C₁₋₇-Alkylengruppe, die gegebenenfalls durch eine Gruppe R_{b} substituiert ist, steht;
- L für ein Stickstoffatom, das gegebenenfalls durch eine Gruppe R_{c} oder R_{d} substituiert ist, oder ein Kohlenstoffatom, das durch eine Gruppe Rₑ₁ und eine Gruppe R_{d} oder zwei Gruppen Rₑ₂ substituiert ist, steht;
wobei die Kohlenstoffatome von A und B gegebenenfalls durch eine oder mehrere Gruppen R_{f}, die gleich oder voneinander verschieden sind, substituiert sind;
Rₐ, R_{b} und R_{c} so definiert sind, dass:
zwei Gruppen Rₐ zusammen eine C₁₋₆-Alkylengruppe bilden können;
Rₐ und R_{b} zusammen eine Bindung oder eine C₁₋₆-Alkylengruppe bilden können;
Rₐ und Rₑ zusammen eine Bindung oder eine C₁₋₆-Alkylengruppe bilden können;
R_{b} und Rₑ zusammen eine Bindung oder eine C₁₋₆-Alkylengruppe bilden können;
R_{d} für eine unter einem Wasserstoffatom und C₁₋₆-Alkyl-, C₃₋₇-Cycloalkyl-, C₃₋₇-Cycloalkyl-C₁₋₆-alkyl-, C₁₋₆-Alkylthio-C₁₋₆-alkyl-, C₁₋₆-Alkyloxy-C₁₋₆-alkyl-, C₁₋₆-Fluoralkyl- und Hydroxy-C₁₋₆-alkylgruppen ausgewählte Gruppe steht;
Rₑ₁ für eine -NR₄R₅-Gruppe oder ein cyclisches Monoamin, das gegebenenfalls ein Sauerstoffatom enthält, steht, wobei dieses cyclische Monoamin gegebenenfalls durch einen oder mehrere unter einem Fluoratom und C₁₋₆-Alkyl-, C₁₋₆-Alkyloxy- und Hydroxylgruppen ausgewählte Substituenten substituiert ist;
zwei Reste Rₑ₂ mit dem Kohlenstoffatom, das sie trägt, ein cyclisches Monoamin, das gegebenenfalls ein Sauerstoffatom enthält, bilden, wobei das cyclische Monoamin gegebenenfalls durch eine oder mehrere Gruppen R_{f}, die gleich oder voneinander verschieden sind, substituiert ist;
R_{f} für eine C₁₋₆₋Alkyl-, C₃₋₇-Cycloalkyl-, C₃₋₇-Cycloalkyl-C₁₋₆-alkyl-, C₁₋₆-Alkyloxy-C₁₋₆-alkyl-, C₃-₇-Cycloalkyloxy-C₁₋₄-alkyl-, C₃-₇-Cycloalkyl-C₁₋₄-alkyloxy-C₁₋₄-alkyl-, Hydroxy-C₁₋₆-alkyl- oder C₁₋₆-Fluoralkylgruppe steht;
R₄ und R₅ unabhängig voneinander für ein Wasserstoffatom oder eine C₁₋₄-Alkyl-, C₃₋₇-Cycloalkyl-oder C₃₋₇-Cycloalkyl-C₁₋₆-alkylgruppe stehen;
- R₇ und R₈ unabhängig voneinander für ein Wasserstoffatom oder eine C₁₋₆-Alkylgruppe stehen;
in Basenform oder in Form eines Additionssalzes mit einer Säure.

2. Verbindung der allgemeinen Formel (I) nach Anspruch 1, **dadurch gekennzeichnet, dass**:
- R₂ für eine C₁₋₄-Alkyl-, C₃₋₄-Cycloalkyl-C₁₋₄-alkyl-, C₁₋₄-Alkyloxy-C₁₋₄-alkyl- oder C₁₋₄-Fluoralkylgruppe steht.

3. Verbindung der allgemeinen Formel (I) nach Anspruch 1 oder 2, **dadurch gekennzeichnet, dass**:
- R₃ für ein Wasserstoff-, Fluor- oder Chloratom oder eine Methyl-, Methylamino-, -NH₂- oder Methoxygruppe steht.

4. Verbindung der allgemeinen Formel (I) nach einem der Ansprüche 1 bis 3, **dadurch gekennzeichnet, dass**:
- R₇ und R₈ jeweils unabhängig voneinander für ein Wasserstoffatom oder eine Methylgruppe stehen.

5. Verbindung der allgemeinen Formel (I) nach einem der Ansprüche 1 bis 4, **dadurch gekennzeichnet, dass**:
- A für eine C₁₋₇-Alkylengruppe, die gegebenenfalls durch eine oder zwei Gruppen Rₐ substituiert ist, steht;
- B für eines C₁₋₇-Alkylengruppe, die gegebenenfalls durch eine Gruppe R_{b} substituiert ist, steht;
- L für ein Stickstoffatom, das gegebenenfalls durch eine Gruppe R_{c} oder R_{d} substituiert ist, steht;
wobei die Kohlenstoffatome von A und B gegebenenfalls durch eine oder mehrere Gruppen R_{f}, die gleich oder voneinander verschieden sind, substituiert sind;
- zwei Gruppen Rₐ zusammen eine C₁₋₆-Alkylengruppe bilden können;
- Rₐ und R_{b} zusammen eine Bindung oder eine C₁₋₆-Alkylengruppe bilden können;
- Rₐ und R_{c} zusammen eine Bindung oder eine C₁₋₆-Alkylengruppe bilden können;
- R_{b} und R_{c} zusammen eine Bindung oder eine C₁₋₆-Alkylengruppe bilden können;
- R_{d} für eine unter einem- Wasserstoffatom und C₁₋₆-Alkyl-, C₃-₇-Cycloalkyl-, C₃₋₇-Cycloalkyl-C₁₋₆-alkyl-, C₁₋₆-Alkylthio-C-₁₋₆-alkyl-, C₁₋₆-Alkyloxy-C₁₋₆-alkyl-, C₁₋₆-Fluoralkyl- und Hydroxy-C₁₋₆-alkylgruppen ausgewählte Gruppe steht;
- R_{f} für eine C₁₋₆-Alkyl-, C₃₋₇-Cycloalkyl-, C₁₋₆-Alkyloxy-C₁₋₆-alkyl-, C₃₋₇-Cycloalkyloxy-C₁₋₄-alkyl-, C₃₋₇-Cycloalkyl-C₁₋₄-alkyloxy-C₁₋₄-alkyl- oder Hydroxy-C₁₋₆-alkylgruppe steht.

6. Verbindung der allgemeinen Formel (I) nach einem der Ansprüche 1 bis 4, **dadurch gekennzeichnet, dass**:
- A für eine C₁₋₇-Alkylengruppe, die gegebenenfalls durch eine oder zwei Gruppen Rₐ substituiert ist, steht;
- B für eine C₁₋₇-Alkylengruppe, die gegebenenfalls durch eine Gruppe R_{b} substituiert ist, steht;
- L für ein Kohlenstoffatom, das durch zwei Gruppen Rₑ₂ substituiert ist, steht;
wobei die Kohlenstoffatome von A und B gegebenenfalls durch eine oder mehrere Gruppen R_{f}, die gleich oder voneinander verschieden sind, substituiert sind;
- zwei Reste Rₑ₂ mit dem Kohlenstoffatom, das sie trägt, ein cyclisches Monoamin, das gegebenenfalls ein Sauerstoffatom enthält, bilden, wobei dieses cyclische Monoamin gegebenenfalls durch eine oder mehrere Gruppen R_{f}, die gleich oder voneinander verschieden sind, substituiert ist;
- R_{f} für eine C₁₋₆-Alkylgruppe steht.

7. Verbindung der allgemeinen Formel (I) nach einem der Ansprüche 1 bis 4, **dadurch gekennzeichnet, dass**:
- A für eine C₁₋₇-Alkylengruppe steht;
- B für eine C₁₋₇-Alkylengruppe steht;
- L für ein Kohlenstoffatom, das durch eine Gruppe Rₑ₁ und eine Gruppe R_{d} substituiert ist, steht;
- R_{d} für ein Wasserstoffatom steht;
- Rₑ₁ für eine -NR₄R₅-Gruppe steht, wobei R₄ und R₅ unabhängig voneinander für ein Wasserstoffatom oder eine C₁₋₄-Alkylgruppe stehen, oder Rₑ₁ auch für ein cyclisches Monoamin, das gegebenenfalls ein Sauerstoffatom enthält, steht, wobei das cyclische Monoamin gegebenenfalls durch einen oder mehrere unter einem Fluoratom und C₁₋₆-Alkyl- und Hydroxylgruppen ausgewählte Substituenten substituiert ist.

8. Verbindung der allgemeinen Formel (I) nach einem der Ansprüche 1 bis 4, **dadurch gekennzeichnet, dass**:
- R₂ für eine Methyl-, Ethyl-, Isopropyl-, Isobutyl-, Cyclopropyl-, Cyclobutyl-, Cyclopropylmethyl-, Methoxymethyl- oder Trifluormethylgruppe steht;
- R₃ für ein Wasserstoff-, Fluor- oder Chloratom oder eine Methyl-, Methylamino-, -NH₂- oder Methoxygruppe steht;
- das durch -N-A-L-B- gebildete cyclische Amin für eine Piperazin-1-yl-, (*R,S*)-3-Methylpiperazin-1-yl-, (*R*)-3-Methylpiperazin-1-yl-, (*S*)-3-Methylpiperazin-1-yl-, 4-Methylpiperazin-1-yl-, 4-methylpiperazin-1-yl-, 4-(Isopropyl)piperazin-1-yl-, 4-(Cyclobutyl)piperazin-1-yl-, (*R,S*)-3-(Hydroxymethyl)piperazin-2-yl-, 3,3-Dimethylpiperazin-1-yl-, *cis*-3,5-Dimethylpiperazin-1-yl-, (*S*)-Hexa-hydropyrrolo[1,2-*a*]pyrazin-2-yl-, (1*S*,4*S*)-2,5-Diazabicyclo[2.2.1]hept-2-yl-, (*R,S*)-2,5-Diaza-bicyclo[2.2.1]hept-2-yl-, (*R,S*)-1,4-Diazabicyclo-non-4-yl-, (*R,S*)-Hexahydropyrrolo[3,4-*b*]pyrrol-5(1*H*)-yl-, Hexahydropyrrolo[3,4-*c*]pyrrol-2(1*H*)-yl-, 5-Methylhexahydropyrrolo[3,4-*c*]pyrrol-2(1*H*)-yl-, 5-Isopropylhexahydropyrrolo[3,4-*c*]pyrrol-2(1*H*)-yl- oder (*R,S*)-Octahydropyrrolo[3,4-*b*]-pyridin-6-ylgruppe steht;
- R₇ und R₈ unabhängig voneinander für ein Wasserstoffatom oder eine Methylgruppe stehen.

9. Verbindung der allgemeinen Formel (I) nach einem der Ansprüche 1 bis 4, **dadurch gekennzeichnet, dass**:
- R₂ für eine Methylgruppe steht;
- R₃ für ein Wasserstoffatom steht;
- das durch -N-A-L-B- gebildete cyclische Amin für 2,7-Diazaspiro[3.5]non-7-yl, (*R,S*)-Diazaspiro-[4.5]dec-2-yl, 2,9-Diazaspiro[5.5]undec-9-yl oder 1-Oxa-4,9-diazaspiroundec-9-yl steht;
- R₇ und R₈ unabhängig voneinander für ein Wasserstoffatom stehen.

10. Verbindung der allgemeinen Formel (I) nach einem der Ansprüche 1 bis 4, **dadurch gekennzeichnet, dass**:
- R₂ für eine Methylgruppe steht;
- R₃ für ein Wasserstoffatom oder eine Methylgruppe steht;
- das durch -N-A-L-B- gebildete cyclische Amin für 4-(Pyrrolidin-1-yl)piperidin-1-yl oder (*R,S*)-[1,3']Bipyrrolidinyl-1'-yl steht;
- R₇ und R₈ unabhängig voneinander für ein Wasserstoffatom stehen.

11. Verbindung der allgemeinen Formel (I) nach Anspruch 1, ausgewählt unter:
- 2-Methyl-6-piperazin-1-yl-3-pyridin-4-ylimidazo[1,2-b]pyridazin und seinem Hydrochlorid (3:1);
- 3-(2-Fluorpyridin-4-yl)-2-methyl-6-piperazin-1-ylimidazo[1,2-b]pyridazin;
- 2,7,8-Trimethyl-6-piperazin-1-yl-3-pyridin-4-ylimidazo[1,2-*b*]pyridazin und seinem Hydrochlorid (3:1);
- 2-Methoxymethyl-6-piperazin-1-yl-3-pyridin-4-yl-imidazo[1,2-*b*]pyridazin;
- 2-Ethyl-6-piperazin-1-yl-3-pyridin-4-ylimidazo[1,2-*b*]pyridazin und seinem Hydrochlorid (3:1) ;
- 2-Ethyl-6-piperazin-1-yl-3-(2-methylpyridin-4-yl)imidazo[1,2-*b*]pyridazin;
- 2-Isopropyl-6-piperazin-1-yl-3-pyridin-4-ylimidazo[1,2-*b*]pyridazin;
- 2-Isopropyl-3-(2-methylpyridin-4-yl)-6-piperazin-1-ylimidazo[1,2-*b*]pyridazin;
- 2-Cyclopropyl-6-piperazin-1-yl-3-pyridin-4-ylimidazo[1,2-*b*]pyridazin;
- 2-Cyclopropyl-3-(2-methylpyridin-4-yl)-6-piperazin-1-ylimidazo[1,2-*b*]pyridazin;
- 4-(2-Cyclopropyl-6-piperazin-1-ylimidazo[1,2-*b*]-pyridazin-3-yl)pyridin-2-ylamin;
- 2-Cyclopropyl-3-(2-methoxypyridin-4-yl)-6-piperazin-1-ylimidazo[1,2-*b*]pyridazun;
- 3-(2-Chlorpyridin-4-yl)-2-cyclopropyl-6-p.iperazin-1-ylimidazo[1,2-*b*]pyridazin;
- 2-Isobutyl-6-piperazin-1-yl-3-pyridin-4-ylimidazo[1,2-*b*]pyridazin und seinem Hydrochlorid (3:1);
- 2-Cyclopropylmethyl-6-piperazin-1-yl-3-pyridin-4-ylimidazo[1,2-*b*]pyridazin;
- 2-Cyclobutyl-6-piperazin-1-yl-3-pyridin-4-ylimidazo[1,2-*b*]pyridazin;
- (*R,S*)-2Methyl-6-(3-methylpiperazin-1-yl)-3-pyridin-4-ylimidazo[1,2-*b*]pyridazin;
- (*R,S*)-2-Methyl-6-(3-methylpiperazin-1-yl)-3-(2-methylpyridin-4-yl)imidazo[1,2-*b*]pyridazin;
- (*R,S*)-2-Cyclopropyl-6-(3-methylpiperazin-1-yl)-3-(2-methylpyridin-4-yl)imidazo[1,2-*b*]pyridazin;
- (*R,S*)-4-[2-Cyclopropyl-6-(3-methylpiperazin-1-yl)imidazo[1,2-*b*]pyridazin-3-yl] pyridin-2-ylamin;
- (*R,S*)-2-Cyclopropyl-3-(2-methoxypyridin-4-yl)-6-(3-methylpiperazin-1-yl)imidazo[1,2-*b*]pyridazin;
- (*R,S*)-3-(2-Chlorpyridin-4-yl)-2-cyclopropyl-6-(3-methylpiperazin-1-yl)imidazo[1,2-*b*]pyridazin;
- 2-Methyl-6-((*R*)-3-methylpiperazin-1-yl)-3-pyridin-4-ylimidazo[1,2-*b*]pyridazin;
- 2-Methyl-6-((*R*)-3-methylpiperazin-1-yl)-3-(2-methylpyridin-4-yl)imidazo[1,2-*b*]pyridazin;
- 4-[2-Methyl-6-((*R*)-3-methylpiperazin-1-yl)-imidazo[1,2-*b*]pyridazin-3-yl]pyridin-2-ylamin;
- Methyl-{4-[2-methyl-6-((*R*)-3-methylpiperazin-1-yl)imidazo[1,2-*b*]pyridazin-3-yl]pyridin-2-yl}-amin;
- 3-(2-Methoxypyridin-4-yl)-2-methyl-6-((*R*)-3-methylpiperazin-1-yl)imidazo[1,2-*b*]pyridazin;
- 2-Ethyl-6-((*R*)-3-methylpiperazin-1-yl)-3-pyridin-4-ylimidazo[1,2-*b*]pyridazin;
- 2-Ethyl-3-(2-fluorpyridin-4-yl)-6-((*R*)-3-methylpiperazin-1-yl)imidazo[1,2-*b*]pyridazin;
- 2-Cyclopropylmethyl-6-((*R*)-3-methylpiperazin-1-yl)-3-pyridin-4-ylimidazo[1,2-*b*]pyridazin;
- 2-Cyclopropylmethyl-6-((*R*)-3-methylpiperazin-1-yl)-3-(2-methylpyridin-4-yl)imidazo[1,2-*b*]-pyridazin;
- 2-Methyl-6-((*S*)-3-methylpiperazin-1-yl)-3-pyridin-4-ylimidazo[1,2-*b*]pyridazin;
- 3-(2-Methoxypyridin-4-yl)-2-methyl-6-((*S*)-3-methylpiperazin-1-yl)imidazo [1,2-*b*] pyridazin;
- 2-Cyclopropyl-6-((*S*)-3-methylpiperazin-1-yl)-3-pyridin-4-ylimidazo[1,2-*b*]pyridazin;
- 4-[2-Methyl-6-((*S*)-3-methylpiperazin-1-yl)-imidazo[1,2-*b*]pyridazin-3-yl]pyridin-2-ylamin;
- 2-Methyl-6-(4-methylpiperazin-1-yl)-3-pyridin-4-ylimidazo[1,2-*b*]pyridazin;
- 6-(4-Methylpiperazin-1-yl)-3-pyridin-4-yl-2-trifluormethylimidazo[1,2-*b*]pyridazin;
- [4-(2-Methyl-3-pyridin-4-ylimidazo[1,2-*b*]-pyridazin-6-yl)piperazin-2-yl]methanol;
- 6-(4-Ethylpiperazin-1-yl)-2-methyl-3-pyridin-4-ylimidazo[1,2-*b*]pyridazin und seinem Hydrochlorid (3:1) ;
- 6-(4-Ethylpiperazin-1-yl)-2-methyl-3-(2-methyl-pyridin-4-yl)imidazo[1,2-*b*]pyridazin;
- 6-(4-Isopropylpiperazin-1-yl)-2-methyl-3-pyridin-4-ylimidazo[1,2-*b*]pyridazin und seinem Hydrochlorid (3:1);
- 6-(4-Isopropylpiperazin-1-yl)-2-methyl-3-(2-methylpyridin-4-yl)imidazo[1,2-*b*]pyridazin;
- 4-[6-(4-Isopropylpiperazin-1-yl)-2-methyl-imidazo[1,2-*b*]pyridazin-3-yl]pyridin-2-yl}-amin und seinem Hydrochlorid (3:1);
- 6-(4-Cyclobutylpiperazin-1-yl)-2-methyl-3-pyridin-4-ylimidazo[1,2-*b*]pyridazin;
- 4-[5-(4-Cyclobutylpiperazin-1-yl)-2-methyl-imidazo[1,2-*b*]pyridazin-3-yl]pyridin-2-ylamin;
- 6-(3,3-Dimethylpiperazin-1-yl)-2-methyl-3-(2-methylpyridin-4-yl)imidazo[1,2-*b*]pyridazin;
- {4-[6-(3,3-Dimethylpiperazin-1-yl)-2-methyl-imidazo[1,2-*b*]pyridazin-3-yl]pyridin-2-yl}methylamin;
- 2-Cyclopropyl-6-(3,3-dimethylpiperazin-1-yl)-3-(2-methylpyridin-4-yl)imidazo[1,2-*b*]pyridazin;
- 4-[2-Cyclopropyl-6-(3,3-dimethylpiperazin-1-yl)-imidazo[1,2-*b*]pyridazin-3-yl]pyridin-2-ylamin;
- 2-Cyclopropyl-6-(3,3-dimethylpiperazin-1-yl)-3-(2-fluorpyridin-4-yl)imidazo[1,2-*b*]pyridazin;
- 6-(cis-3,5-Dimethylpiperazin-1-yl)-2-methyl-3-pyridin-4-ylimidazo[1,2-*b*]pyridazin und seinem Hydrochlorid (3:1);
- 6-(*cis*-3,5-Dimethylpiperazin-1-yl)-2-methyl-3-(2-methylpyridin-4-yl)imidazo[1,2-*b*]pyridazin;
- 4-[6-(*cis*-3,5-Dimethylpiperazin-1-yl)-2-methyl-imidazo[1,2-*b*]pyridazin-3-yl]pyridin-2-ylamin;
- {4-[6-(*cis*-3,5-Dimethylpiperazin-1-yl)-2-methyl-imidazo[1,2-*b*]pyridazin-3-yl]pyridin-2-yl}methylamin;
- 2-Cyclopropyl-6-(*cis*-3,5-dimethylpiperazin-1-yl)-3-pyridin-4-ylimidazo[1,2-*b*]pyridazin;
- 4-[2-Cyclopropyl-6-(*cis*-3,5-dimethylpiperazin-1-yl)imidazo[1,2-*b*]pyridazin-3-yl]pyridin-2-ylamin;
- 6-(*S*)-Hexahydropyrrolo[1,2-*a*]pyrazin-2-yl-2-methyl-3-pyridin-4-ylimidazo[1,2-*b*]pyridazin;
- 6-(5)-Hexahydropyrrolo[1,2-*a*]pyrazin-2-yl-2-methyl-3-(2-methylpyridin-4-yl)imidazo[1,2-*b*]-pyridazin;
- 6-(1*S*,4*S*)-2,5-Diazabicyclo[2.2.1]hept-2-yl-2-methyl-3-pyridin-4-ylimidazo[1,2-*b*]pyridazin;
- (*R,S*)-6-(2,5-Diazabicyclo[2.2.1]hept-2-yl)-2-methyl-3-(2-methylpyridin-4-yl)imidazo[1,2-*b*]-pyridazin und seinem Hydrobromid (1:1);
- 4-[2-Cyclopropyl-6-(1,4-diazabicyclo[3.2.2]non-4-yl)-3-(2-methylpyridin-4-yl)imidazo[1,2-*b*]-pyridazin;
- (*R,S*)-6-(Hexahydropyrrolo[3,4-*b*]pyrrol-5(1*H*)-yl)-2-methyl-3-(2-methylpyridin-4-yl)-imidazo[1,2-*b*]pyridazin;
- 6-Hexahydropyrrolo[3,4-*c*]pyrrol-2(1*H*)-yl-2-methyl-3-pyridin-4-ylimidazo[1,2-*b*]pyridazin;
- 6-Hexahydropyrrolo[3,4-*c*]pyrrol-2(1*H*)-yl-2-methyl-3-(2-methylpyridin-4-yl)-imidazo[1,2-*b*]-pyridazin und seinem Hydrochlorid (3:1);
- 4-(6-Hexahydropyrrolo[3,4-*c*]pyrrol-2(1*H*)-yl-2-methylimidazo[1,2-*b*]pyridazin-3-yl)pyridin-2-ylamin;
- [4-(6-Hexahydropyrrolo[3,4-*c*]pyrrol-2(1*H*)-yl-2-methylimidazo[1,2-*b*]pyridazin-3-yl)pyridin-2-yl]-methylamin;
- 2-Methyl-6-(5-methylhexahydropyrrolo[3,4-*c*]-pyrrol-2(1*H*)-yl)-3-pyridin-4-ylimidazo[1,2-*b*]-pyridazin;
- 2,7-Dimethyl-6-(5-methylhexahydropyrrolo[3,4-*c*]-pyrrol-2(1*H*)-yl)-3-pyridin-4-ylimidazo[1,2-*b*]-pyridazin;
- 6-(5-Isopropylhexahydropyrrolo[3,4-*c*]pyrrol-2(1*H*)-yl)-2-methyl-3-pyridin-4-ylimidazo[1,2-*b*]-pyridazin;
- 4-[6-(5-Isopropylhexahydropyrrolo[3,4-*c*]pyrrol-2(1*H*)-yl)-2-methylimidazo[1,2-*b*]pyridazin-3-yl]-pyridin-2-ylamin;
- 6-Hexahydropyrrolo[3,4-*c*]pyrrol-2(1*H*)-yl-3-(2-methoxypyridin-4-yl)-2-methylimidazo[1,2-*b*]-pyridazin;
- (*R,S*)-2-Methyl-6-(octahydropyrrolo[3,4-*b*]-pyridin-6-yl)-3-pyridin-4-ylimidazo[1,2-*b*]-pyridazin;
- 6-(2,7-Diazaspiro[3.5]non-7-yl)-2-methyl-3-(2-methylpyridin-4-yl)imidazo[1,2-*b*]pyridazin;
- (*R,S*)-6-(2,7-Diazaspiro[4.5]dec-2-yl)-2-methyl-3-pyridin-4-ylimidazo[1,2-*b*]pyridazin;
- 9-(2-Methyl-3-pyridin-4-ylimidazo[1,2-*b*]-pyridazin-6-yl)-2,9-diazaspiro[5.5]undecan und seinem Hydrochlorid (3:1);
- 9-[2-Methyl-3-(2-methylpyridin-4-yl)imidazo[1,2-*b*]pyridazin-6-yl]-2,9-diazaspiro[5.5]undecan und seinem Hydrochlorid (3:1);
- 9-(2-Methyl-3-pyridin-4-ylimidazo[1,2-*b*]-pyridazin-6-yl)-1-oxa-4,9-diazaspiro[5.5]undecan und seinem Hydrochlorid (3:1);
- 4-[2-Methyl-6-(1-oxa-4,9-diazaspiro[5.5]undec-9-yl)imidazo[1,2-*b*]pyridazin-3-yl]pyridin-2-ylamin und seinem Hydrochlorid (3:1);
- 2-Methyl-3-pyridin-4-yl-6-(4-pyrrolidin-1-yl-piperidin-1-yl)imidazo[1,2-*b*]pyridazin;
- 2-Methyl-3-(2-methylpyridin-4-yl)-6-(4-pyrrolidin-1-ylpiperidin-1-yl)imidazo[1,2-*b*]-pyridazin;
- 4-[2-Methyl-6-(4-pyrrolidin-1-ylpiperidin-1-yl)-imidazo[1,2-*b*]pyridazin-3-yl]pyridin-2-ylamin;
- (*R,S*)-6-[1,3']Bipyrrolidinyl-1'-yl-2-methyl-3-pyridin-4-ylimidazo[1,2-*b*]pyridazin.

12. Verfahren zur Herstellung einer Verbindung der Formel (I) nach Anspruch 1, **dadurch gekennzeichnet, dass** man eine Verbindung der allgemeinen Formel (IIa) 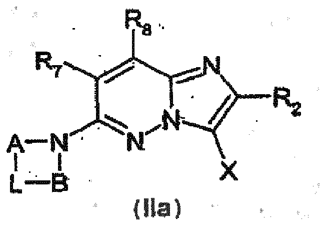 worin R₂, A, L, B, R₇ und R₈ die in Anspruch 1 angegebene Bedeutung besitzen und X für ein Brom-oder Iodatom steht, mit einer Verbindung der Formel (IVa) 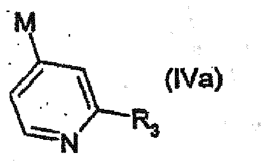 worin R₃ die in Anspruch 1 angegebene Bedeutung besitzt und M für eine Trialkylstannyl-, Dihydroxyboryl- oder Dialkoxyborylgruppe steht, umsetzt.

13. Verfahren zur Herstellung einer Verbindung der Formel (I) nach Anspruch 1, **dadurch gekennzeichnet, dass** man:
a) eine Verbindung der allgemeinen Formel (II) 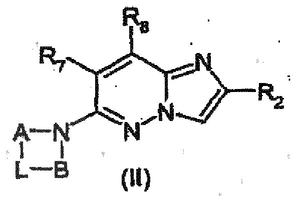 worin R₂, A, L, B, R₇ und R₈ die in Anspruch 1 angegebene Bedeutung besitzen, mit einer Mischung eines Pyridinderivats der allgemeinen Formel (IVb) 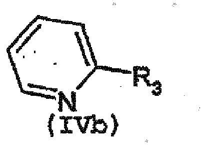 worin R₃ für ein Wasserstoffatom oder eine C₁₋₃-Alkylgruppe steht, in Gegenwart von Chlorameisensäurealkylester zu einer Verbindung der Formel (IIb) 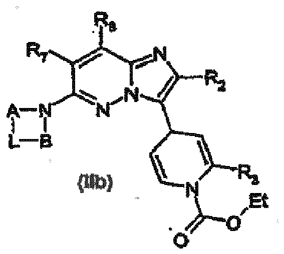 worin R₂, A, L, B, R₇ und R₈ die in Anspruch 1 angegebene Bedeutung besitzen und R₃ für ein Wasserstoffatom oder eine C₁₋₃-Alkylgruppe steht, umsetzt;
b) die in Schritt a) erhaltene Verbindung der allgemeinen Formel (IIb) in einem Lösungsmittel mit ortho-Chloranil umsetzt.

14. Verfahren zur Herstellung einer Verbindung der Formel (I) nach Anspruch 1, **dadurch gekennzeichnet, dass** man eine Verbindung der allgemeinen Formel (II) 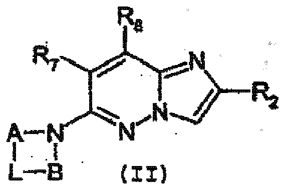 worin R₂, R₇, R₈, A, L und B die in Anspruch 1 angegebene Bedeutung besitzen, in Gegenwart eines Katalysators und einer anorganischen Base und in einem aprotischen polaren Lösungsmittel mit einer Verbindung der allgemeinen Formel (IVc) 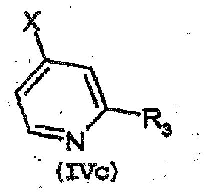 worin R₃ die in Anspruch 1 angegebene Bedeutung besitzt und X für ein Halogenatom steht, umsetzt.

15. Medikament, **dadurch gekennzeichnet, dass** es eine Verbindung der Formel (I) nach einem der Ansprüche 1 bis 11 in Basenform oder in Form eines Additionssalzes mit einer pharmazeutisch unbedenklichen Säure umfasst.

16. Pharmazeutische Zusammensetzung, **dadurch gekennzeichnet, dass** sie eine Verbindung der Formel (I) nach einem der Ansprüche 1 bis 11 in Basenform oder in Form eines Additionssalzes mit einer pharmazeutisch unbedenklichen Säure sowie mindestens einen pharmazeutisch unbedenklichen Hilfsstoff umfasst.

17. Verwendung einer Verbindung der allgemeinen Formel (I) nach einem der Ansprüche 1 bis 11 zur Herstellung eines Medikaments zur Behandlung oder Prävention von Schlafstörungen oder Störungen des zirkadianen Rhythmus.

18. Verwendung einer Verbindung der allgemeinen Formel (I) nach einem der Ansprüche 1 bis 11 zur Herstellung eines Medikaments zur Behandlung oder Prävention von bipolaren Störungen.

19. Verwendung einer Verbindung der allgemeinen Formel (I) nach einem der Ansprüche 1 bis 11 zur Herstellung eines Medikaments zur Behandlung oder Prävention von Erkrankungen, die mit Drogenabhängigkeit assoziiert sind.

20. Verwendung einer Verbindung der allgemeinen Formel (I) nach einem der Ansprüche 1 bis 11 zur Herstellung eines Medikaments zur Behandlung oder Prävention von Erkrankungen, die mit Hyperphosphorylierung des tau-Proteins in Zusammenhang stehen.

21. Verwendung einer Verbindung der allgemeinen Formel (I) nach einem der Ansprüche 1 bis 11 zur Herstellung eines Medikaments zur Behandlung oder Prävention von Erkrankungen, die durch Zellproliferation verursacht oder verschlimmert werden.

22. Verwendung einer Verbindung der allgemeinen Formel (I) nach Anspruch 21, **dadurch gekennzeichnet, dass** es sich bei den Zellen um Tumorzellen handelt.
